# EUROPÄISCHE PATENTANMELDUNG

(11) **EP 3 524 962 A1**
(43) Veröffentlichungstag der Anmeldung: **14.08.2019**
(21) Anmeldenummer: 19157662.8
(22) Anmeldetag: 07.12.2016
(51) Int. Cl.: G01N 21/17, G01N 21/552, A61B 5/145, A61B 5/1455, G01N 33/49, G01N 21/63, A61B 5/00, G01N 21/55, G01N 33/66, G01N 21/3563, G01N 21/33, G01N 33/483

(54) **VORRICHTUNG UND VERFAHREN ZUM ANALYSIEREN EINES STOFFS**

(30) Priorität: 02.08.2016 DE 102016214262; 19.02.2016 DE 102016215580; 09.12.2015 WO PCT/DE2015/200532
(62) Teilanmeldung aus: 16822616.5
(71) Anmelder: Diamontech GmbH, 10245 Berlin (DE)
(72) Erfinder: BAUER, Alexander, 61440 Oberursel (DE); HERTZBERG, Otto, 60318 Frankfurt am Main (DE); LUBINSKI, Thorsten, 10245 Berlin (DE)
(74) Vertreter: Lucke, Andreas

(57) **Zusammenfassung**

Die Erfindung bezieht sich unter anderem auf eine Vorrichtung (10) zum Analysieren eines Stoffes (101) mit einer Anregungssendeeinrichtung (100) zum Erzeugen mindestens eines elektromagnetischen Anregungsstrahls (SA), insbesondere Anregungs-Lichtstrahls, mit zumindest einer Anregungswellenlänge, einer Detektionseinrichtung (106) zur Detektion eines Reaktionssignals (SR) und einer Einrichtung (107) zum Analysieren des Stoffes anhand des detektierten Reaktionssignals (SR).

## Beschreibung

Das vorliegende Schutzrecht bezieht sich auf eine Vorrichtung und ein Verfahren zum Analysieren eines Stoffs. Die hier beschriebene Vorrichtung und das hier beschriebene Verfahren können zum Beispiel zur Analyse von tierischem oder menschlichem Gewebe, in einer Ausführungsform zur Messung von Glukose bzw. Blutzucker, genutzt werden.

Bekannte Verfahren zum Analysieren eines Stoffs, insbesondere zum Messen von Blutzucker, sind beispielsweise in den folgenden Druckschriften beschrieben:
- Guo et al.: "Noninvasive glucose detection in human skin using wavelength modulated differential laser photothermal radiometry", Biomedical Optics Express, Vol, 3, 2012, No. 11,
- Uemura et al.:"Non-invasive blood glucose measurement by Fourier transform infrared spectroscopic analysis through the mucous membrane of the lip: application of a chalcogenide optical fiber system", Front Med Biol Eng. 1999; 9(2): 137-153,
- Farahi et al.: "Pump probe photothermal spectroscopy using quantum cascade lasers", J. Phys. D. Appl. Phys. 45 (2012) und
- M. Fujinami et al.: "Highly sensitive detection of molecules at the liquid/liquid interface using total internal reflection-optical beam deflection based an photothermal spectroscopy", Rev. Sei. Instrum., Vol. 74, Number 1 (2003).
- (1) von Lilienfeld-Toal, H. Weidenmüller, M. Xhelaj, A. Mäntele, W. A Novel Approach to Non- Invasive Glucose Measurement by Mid-Infrared Spectroscopy: The Combination of Quantum Cascade Lasers (QCL) and Photoacoustic Detection Vibrational Spectroscopy, 38:209-215, 2005.
- (2) Pleitez, M. von Lilienfeld-Toal, H. Mäntele W. Infrared spectroscopic analysis of human interstitial fluid in vitro and in vivo using FT-IR spectroscopy and pulsed quantum cascade lasers (QCL): Establishing a new approach to non invasive glucose measurement Spectrochimica acta. Part A, Molecular and biomolecular spectroscopy, 85:61-65, 2012
- (3) Pleitez, M. et al. In Vivo Noninvasive Monitoring of Glucose Concentration in Human Epidermis by Mid-Infrared Pulsed Photoacoustic Spectroscopy Analytical Chemsitry, 85:1013-1020, 2013.
- (4) Pleitez, M. Lieblein, T. Bauer, A. Hertzberg, O. von Lilienfeld-Toal, H. Mäntele, W. Windowless ultrasound photoacoustic cell for in vivo mid-IR spectroscopy of human epidermis: Low interference by changes of air pressure, temperature, and humidity caused by skin contact opens the possibility for a non-invasive monitoring of glucose in the interstitial fluid Review of Scientific Instruments 84, 2013
- (5) M. A. Pleitez Rafael, O. Hertzberg, A. Bauer, M. Seeger, T. Lieblein, H. von Lilienfeld-Toal, and W. Mäntele. Photothermal deflectometry enhanced by total internal reflection enables non- invasive glucose monitoring in human epidermis. The Analyst, November 2014.

Es liegt die Aufgabe zugrunde, eine Vorrichtung anzugeben, mit der sich ein Stoff, insbesondere ein tierisches oder menschliches Gewebe oder ein Bestandteil oder Inhaltsstoff des Gewebes, besonders einfach und kostengünstig analysieren lässt.

Diese Aufgabe wird unter anderem durch eine Vorrichtung mit den Merkmalen gemäß Anspruch 1 gelöst. Ausgestaltungen der Vorrichtung sind in Unteransprüchen angegeben.

Verwiesen wird auf die deutsche Patentschrift DE 10 2014 108 424 B3, auf deren Inhalt hier konkret Bezug genommen wird und auf die diese Anmeldung inhaltlich aufbaut; der komplette Inhalt der deutschen Patentschrift DE 10 2014 108 424 B3 soll daher durch diese hier vorgenommene explizite Bezugnahme auch als Offenbarungsgehalt dieser Anmeldung angesehen werden ("incorporation by reference" für sämtliche Details der dortigen Offenbarung). Insbesondere bezieht sich diese Bezugnahme auf sämtliche in den erteilten Patentansprüchen genannten Merkmale. Darüber hinaus bezieht sich die Bezugnahme insbesondere auch auf Details des dort genannten Anregungs-Lichtstrahls, beispielsweise auf die dort genannten Zahlenwerte der Pulsfrequenzen und Wellenlängen(-bereiche), und ebenso auf die Details zur Messung von Glukosegehalt in der interstitiellen Flüssigkeit.

Die vorliegende PCT-Schutzrechtsanmeldung bezieht sich neben den unmittelbar bei Einreichung ausdrücklich genannten Anspruchsgegenständen und Ausführungsbeispielen auch auf weitere Aspekte, die am Ende der hiesigen Beschreibung aufgezählt sind. Diese Aspekte können einzeln oder in Gruppen jeweils mit Merkmalen der bei Einreichung genannten Ansprüche kombiniert werden. Diese Aspekte stellen für sich genommen, oder miteinander, oder mit Anspruchsgegenständen kombiniert, eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen, Fortsetzungsanmeldungen ("continuation applications" in den USA), Teil-Fortsetzungsanmeldungen ("continuation-in-part applications" in den USA) oder Nachanmeldungen unter Inanspruchnahme der Priorität dieser Anmeldung geschehen.

Im Folgenden wird aber zunächst auf die bei Einreichung aufgeführten Anspruchsgegenstände eingegangen.

Es ist eine Vorrichtung zum Analysieren eines Stoffs vorgesehen mit einer Anregungssendeeinrichtung zum Erzeugen mindestens eines elektromagnetischen Anregungsstrahls, insbesondere Anregungs-Lichtstrahls, mit zumindest einer Anregungswellenlänge, einer Detektionseinrichtung zur Detektion eines Reaktionssignals und einer Einrichtung zum Analysieren des Stoffes anhand des detektierten Reaktionssignals.

Ein wesentlicher Vorteil dieser Vorrichtung ist darin zu sehen, dass sich mit dieser sehr einfach und zuverlässig ein Stoff analysieren lässt.

Die Messung von Konzentrationen einer bestimmten Substanz in einer Körperflüssigkeit wird in dem vorliegenden Schutzrecht auch anhand des Beispiels von Glukose in der ISF (interstitiellen Flüssigkeit) anschaulich gemacht. Die Gegenstände des vorliegenden Schutzrechts sind allerdings nicht darauf beschränkt.

Es können auch andere (körpereigene und nicht körpereigene) Substanzen gemessen werden, beispielsweise solche, die in der DE 10 2014 108 424 B3 beschrieben sind sowie in der die Priorität dieser beanspruchenden PCT-Anmeldung.

Insbesondere können die Verfahren und Vorrichtungen auch zur Medikamentenspiegelbestimmung ("Drug Monitoring") genutzt werden. Im Rahmen des vorliegenden Schutzrechts wird hierunter die Messung der Konzentrationen von Medikamenten im Blut oder in anderen Füssigkeitsräumen des menschlichen oder tierischen Körpers verstanden, zum Beispiel im Blutserum oder Plasma, Speichel oder Lymphe und insbesondere auch nicht-invasiv und invivo in der interstitiellen Flüssigkeit.

Die Medikamentenspiegelbestimmung kann insbesondere auch dazu genutzt werden, um die Dosierung von Medikamenten mit einer geringen therapeutischen Breite zu verbessern. Besonders bei Medikamenten, die leicht über- oder unterdosiert werden können, deren Konzentrationen leicht durch andere Medikamente beeinflusst werden oder die ab einer bestimmten Konzentration toxisch wirken, ist das Drug Monitoring sinnvoll. Dabei ist eine beispielhaft zu erreichende Richtgröße das Erreichen bzw. Aufrechterhalten eines festgelegten Wirkspiegels und die Bestimmung der dafür notwendigen individuellen Arzneimitteldosis. Mithilfe der in diesem Schutzrecht gezeigten Methoden bzw. Vorrichtungen können medizinisch wichtige Spiegel schnell bzw sogar in Echtzeit ermittelt werden, zum Beispiel von

Paracetamol, Phenytoin, Valproinsäure, Lamotrigin, Phenobarbital, Flecainid,Digitoxin, Digoxin, Tacrolimus,Everolimus,Amiodaron,Aminoglykoside,Theophyllin,Vancomycin,Lithium,Carbam azepin,Sirolimus, Methotrexat und anderen Stoffen, wobei hier jeweils unterschiedliche spektroskopische "Fingerabdrücke", d.h. charakteristische Extremstellen (insbesondere absolute und relative Absorptionsmaxima und -minima), die Detektion und Identifikation der jeweiligen Substanz erleichtern.

Die in den Ansprüchen vorgestellte Vorrichtung kann jeweils auch mit einer Dosiereinrichtung zur dosierten Zuführung eines oder mehrerer der genannten Stoffe verbunden sein, um einen Regelkreis ausbilden zu können.

Bemerkenswert ist, insbesondere auch im Zusammenhang mit der Messung von Glukose in der ISF, dass die einzig wirklich klinisch verwertbaren Ergebnisse bei der Auswertung charakteristischer Extremstellen im mittleren Infrarotbereich erreicht werden, und dort insbesondere, wenn mit einer Vielzahl von relativ eng beieinander liegenden Wellenlängen die Erkennung der Absorptionsmaxima und -minima erfolgt. Hierdurch wird die Ungenauigkeit (bedingt durch Temperaturanfälligkeit des Lasers, Rauschen der Auswerteelektronik etc) so ausgeglichen, dass stets ein ausreichend genaues Messergebnis erreicht wird. Bezüglich der Anzahl auszusendender Wellenlängen wird auf die Patentansprüche verwiesen. Hier kann es zB günstig sein, wenn bei Vorliegen von drei charakteristischen nebeneinanderliegenden Extremstellen einer Substanz, d.h. (relativen oder absoluten Minima und/ oder Maxima) mindestens zehn, vorzugsweise mindestens zwanzig Wellenlängen im Intervall zwischen den beiden äußeren Extremstellen ausgesendet werden.

Unter dem Begriff Licht werden hier elektromagnetische Wellen bzw. elektromagnetische Strahlung im sichtbaren Bereich, im nahen und fernen Infrarotbereich sowie um UV-Bereich verstanden.

Bei einer beispielhaften Ausgestaltung der Vorrichtung ist vorgesehen, dass
die Anregungssendeeinrichtung eine Strahlungsquelle, in einer Ausführungsform eine monochromatische, insbesondere eine polarisierte Strahlungsquelle oder Lichtquelle, weiter insbesondere eine Laserlichtquelle ist,
- die Vorrichtung ein optisches Medium aufweist, das mit dem Stoff, insbesondere einem ersten Bereich der Oberfläche des Stoffs, in direktem Kontakt steht,
- wobei vorzugsweise die Anregungssendeeinrichtung so angeordnet ist, dass der ausgesendete Anregungsstrahl in das optische Medium eindringt und dieses an einem vorbestimmten Punkt an der Oberfläche des optischen Mediums wieder verlässt, und
- die Vorrichtung eine Einrichtung zum Aussenden eines Messstrahls, insbesondere Mess-Lichtstrahls, umfasst, die so angeordnet ist, dass der ausgesendete Messstrahl in das optische Medium eindringt und wobei vorzugsweise im Betrieb der Messstrahl und der Anregungsstrahl sich auf einer Grenzfläche des optischen Mediums und der Oberfläche des Stoffs, an der der Messstrahl reflektiert wird, überlappen, und
- die Detektionseinrichtung eine Einrichtung zum Empfangen des das Reaktionssignal bildenden reflektierten Messstrahls und/oder zum direkten oder indirekten Detektieren einer Ablenkung des reflektierten Messstrahls ist.

Vorzugsweise weist die Vorrichtung ein optisches Medium auf, das mit dem Stoff, insbesondere einem ersten Bereich der Oberfläche des Stoffs, in einer Ausführungsform der Haut eines Menschen, in direktem Kontakt steht wobei die Detektionseinrichtung zur Detektion eines Reaktionssignals eine Parameteränderung des optischen Mediums, insbesondere in einem dem ersten Bereich benachbarten Bereich, infolge des Reaktionssignals, insbesondere eine Verformung und/oder Dichteänderung des optischen Mediums, als Folge einer lokalen, zeitabhängigen Erwärmung, erfasst. Das optische Medium kann aus einem optisch oder für Infrarotstrahlung oder Ultraviolettstrahlung, allgemein für den Anregungsstrahl und den Messstrahl transparenten Material, beispielweise Glas, Kristall, Zinksulfid (ZnS), Zinkselenid (ZnSe), Germanium (Ge), Silizium (Si) und Diamant oder einem transparenten Kunststoff, in einer Ausführungsform einem Polyethylen, bestehen. Eine lokale Erwärmung als Reaktion auf einen Wärmetransport von dem zu analysierenden Stoff oder einer Substanz des Stoffes in das optische Medium führt dort zu einer Veränderung, beispielsweise einer Materialverformung oder zu thermischen Spannungen oder lokalen Veränderungen im Brechungsindex, die detektierbar sind.

Der Stoff kann dabei in einer Ausführungsform das Gewebe eines Lebewesens insbesondere eines Menschen sein, wobei die Stoffoberfläche die Haut sein kann. Es können dann Substanzen in dem Gewebe analysiert oder gemessen werden.

Es kann zudem vorgesehen sein, dass die Detektionseinrichtung einen mit dem optischen Medium verbundenes oder in dieses integriertes Piezoelement als Detektor zur Erfassung einer Spannung, Verformung und/oder Dichteänderung aufweist.

Außerdem kann vorgesehen sein, dass die Detektionseinrichtung wenigstens einen Temperatursensor als Detektor zur Erfassung des Reaktionssignals aufweist. Dieser kann direkt am optischen Medium angeordnet sein oder in seiner Umgebung, in Abhängigkeit vom Messprinzip.

Vorzugsweise weist die Vorrichtung eine Einrichtung zur Intensitätsmodulierung des Anregungslichtstrahls auf.

Die Detektionseinrichtung ist bevorzugt zur Detektion eines zeitabhängigen Reaktionssignals in Abhängigkeit von der Wellenlänge des Anregungslichts und/oder der Intensitäts-Modulation des Anregungslichts geeignet.

Auch kann es vorgesehen sein, dass die Anregungssendeeinrichtung mindestens einen elektromagnetischen Anregungsstrahl in ein Stoffvolumen einstrahlt, das unterhalb eines ersten Bereiches der Oberfläche des Stoffs liegt.

Besonders bevorzugt umfasst die Anregungssendeeinrichtung zwei oder mehr Sendeelemente, insbesondere in Form eines ein- zwei- oder mehrdimensionalen Sendeelementarrays. Dies kann somit als Flächenarray von Sendeelementen oder auch als Sendeelementleiste ausgebildet sein (in einer Ausführungsform Halbleiterlaserarrays oder QCL-Arrays, wobei QCL für Quantenkaskadenlaser steht).

Zudem kann vorgesehen sein, dass die zwei oder mehr Sendeelemente jeweils einen eigenen elektromagnetischen Anregungsstrahl erzeugen und diesen in das Volumen unterhalb des ersten Bereiches einstrahlen. Die verschiedenen Anregungsstrahlen können nacheinander oder auch wenigstens teilweise gleichzeitig ausgesendet werden. Die verschiedenen Sendeelemente können auch zeitgleich mit verschiedenen Modulationsfrequenzen betrieben werden.

Die Wellenlängen der elektromagnetischen Anregungsstrahlen der zwei oder mehr Sendeelemente unterscheiden sich vorzugsweise. Die Wellenlängen sind bevorzugt derart gewählt, dass eine in dem zu analysierenden Stoff zu detektierende Substanz Strahlung dieser Wellenlängen besonders gut absorbiert. Es können auch zusätzlich oder alternativ Wellenlängen oder Wellenlängenbereiche gewählt werden, die die zu detektierende Substanz nicht absorbiert, die aber von anderen Substanzen absorbiert werden (sogenannte tolerante Wellenlängen), um die zu analysierende Substanz von anderen Substanzen abgrenzen zu können.

Die Anregungssendeeinrichtung umfasst in einer Ausführungsform zwei oder mehr Laser, insbesondere in Form eines ein- oder zweidimensionalen Laserarrays, wobei auch mehrere Reihen von Laserelementen zur Platzersparnis gestaffelt und versetzt hintereinander angeordnet sein können, in einer Ausführungsform einer Laserleiste, und/oder zwei oder mehr Leuchtdioden, insbesondere in Form eines ein- oder zweidimensionalen Diodenarrays, auch tiefengestaffelt und gegeneinander versetzt, in einer Ausführungsform eines flächigen Arrays oder einer Leiste. Die Outputstrahlen der Arrays können sowohl, nahe beisammen oder parallel, für jedes Strahlelement einzeln, als auch durch bereits integrierte Optiken, denselben Strahlengang haben.

Bezüglich des Aufbaus der Vorrichtung kann es vorgesehen sein, dass die Anregungssendeeinrichtung unmittelbar oder mittelbar - vorzugsweise mittels einer Justagevorrichtung - mit einem optischen Medium, das mit dem Stoff, insbesondere dem ersten Bereich der Oberfläche des Stoffs, in direktem Kontakt steht, mechanisch fest verbunden ist. Somit kann die Anregungssendeeinrichtung bereits bei der Herstellung oder zumindest vor dem Einsatz im Betrieb relativ zu dem optischen Medium justiert und fixiert werden.

Das optische Medium kann zur Befestigung und/oder Ausrichtung oder Justage von einer Anregungssendeeinrichtung und/oder Elementen einer Detektionseinrichtung wenigstens eine integrierte Erhebung und/oder Ausnehmung, wie einen Steg, eine Schulter, eine aufgesetzte Halbkugel, einen aufgesetzten Quader, einen Kegel oder eine Bohrung, Nut, Mulde oder sonstige Ausnehmung aufweisen, in oder an die die genannten Elemente (die Anregungssendeeinrichtung und /oder Elemente einer Detektionseinrichtung) angelegt, angelehnt oder an denen sie justiert oder befestigt werden können. Auch das Anarbeiten von ausgerichteten Passflächen durch Bearbeiten des optischen Mediums oder in einem Gießprozess kann vorgesehen sein.

Bezüglich der Einrichtung zur Intensitätsmodulierung kann vorgesehen sein, dass diese eine elektrische oder elektromechanische Modulationseinrichtung, die mit der Anregungssendeeinrichtung elektrisch in Verbindung steht und diese insbesondere elektrisch ansteuert, umfasst oder durch eine solche gebildet ist. Die Modulationseinrichtung kann eine Intensitätsmodulierung des Anregungsstrahls, in einer Ausführungsform eine periodische Intensitätsmodulierung, weiter beispielsweise in Form von Rechteckpulsen, einer Sägezahnfunktion oder einer Sinusfunktion oder einer anderen periodischen Funktion erzeugen.

Alternativ oder zusätzlich kann die Einrichtung zur Intensitätsmodulierung wenigstens einen im Strahlengang angeordneten gesteuerten Spiegel umfassen, durch dessen Ansteuerung die Intensität des Anregungsstrahls durch Ablenkung modulierbar ist.

Alternativ oder zusätzlich kann die Einrichtung zur Intensitätsmodulierung wenigstens eine bezüglich ihrer Transparenz steuerbare, im Strahlengang angeordnete Schicht umfassen oder durch eine solche gebildet sein. Somit kann das Modulationselement in Form eines bezüglich seiner Transmission gesteuerten Transmissionselementes ausgestaltet sein. Das Modulationselement kann aus einem Lichtstrahl mehrere, räumlich getrennte Lichtstrahlen erzeugen. Es kann in einer Ausführungsform auch vorgesehen sein, dass mit dem Modulationselement die Oberfläche einer Probe abgerastert wird. Das Modulationselement kann in einer Ausführungsform gemeinsam mit dem Array von Lichtquellen/Laserquellen gesteuert werden.

Eine Einrichtung zum Aussenden eines Messstrahls, insbesondere Mess-Lichtstrahls, ist in einer Ausführungsform zum Aussenden des Messstrahls in denjenigen Bereich eines optischen Mediums vorgesehen, der mit dem ersten Bereich der Oberfläche des Stoffs in Kontakt steht.

Die Einrichtung zum Aussenden eines Messstrahls und die Detektionseinrichtung sind in einer Ausführungsform derart zueinander ausgerichtet, dass die Detektionseinrichtung als das zeitabhängige Reaktionssignal den Messstrahl detektiert, nachdem dieser zumindest einmal an derjenigen Grenzfläche des optischen Mediums reflektiert worden ist, die mit dem Stoff, insbesondere dem ersten Bereich der Oberfläche des Stoffs, in Kontakt steht.

Mit Blick auf eine einfache Montage ist es vorteilhaft, wenn die Einrichtung zum Aussenden eines Messstrahls und/oder die Detektionseinrichtung und/oder die Anregungssendeeinrichtung unmittelbar mit dem optischen Medium mechanisch fest verbunden und/oder mittels eines oder mehrerer Lichtwellenleiter an dieses angekoppelt ist.

Auch sind Ausgestaltungen denkbar, bei denen das optische Medium unmittelbar eine Abbildungsoptik trägt und/oder in das optische Medium eine Abbildungs-Optik integriert ist.

Darüber hinaus sind Ausgestaltungen denkbar, bei denen die Oberfläche des optischen Mediums mehrere gegeneinander geneigte Teilflächen aufweist, an denen der Messstrahl, insbesondere der Mess-Lichtstrahl, mehrfach reflektiert wird.

Auch können Ausgestaltungen vorgesehen sein, bei denen in oder an dem optischen Medium ein oder mehrere Spiegelflächen zur Reflektion des Messstrahls, insbesondere Mess-Lichtstrahls, vorgesehen sind.

Mit Blick auf einen kompakten Aufbau ist es denkbar, dass die Anregungssendeeinrichtung und/oder die Einrichtung zum Aussenden des Messstrahls und/oder die Detektionseinrichtung unmittelbar aneinander oder an einem gemeinsamen Träger befestigt sind. Die verschiedenen Einrichtungen können an dem Träger in einer Ausführungsform durch Schweißen oder Kleben oder durch Verschrauben oder durch eine Rastverbindung befestigt sein, wobei eine Justagemöglichkeit entweder beim Zusammenbau oder auch später durch eine Justierschraube oder eine andere mechanische Justiereinrichtung gegeben sein kann. Insbesondere die Einrichtung zum Aussenden des Messstrahls und/oder die Detektionseinrichtung sollten gut zueinander justiert oder justierbar sein. Darum kann es sinnvoll sein, diese beiden Einrichtungen unmittelbar an dem optischen Medium zu befestigen. Die Einrichtung zum Aussenden des Messstrahls und/oder die Detektionseinrichtung können bei geeigneter Lenkung des Messstrahls auch auf derselben Seite des optischen Mediums nebeneinander angeordnet und auf einem gemeinsamen Träger, in einer Ausführungsform einer gemeinsamen Leiterplatte oder einem gemeinsamen Halbleiter befestigt oder als gemeinsame integrierte Halbleitereinrichtung, in einer Ausführungsform als gemeinsamer integrierter Halbleiterbaustein ausgebildet sein. Dieser Träger kann dann als Ganzes gegenüber dem optischen Medium justiert werden, in einer besonderen Ausführungsform auch ohne die Relativposition zwischen der Einrichtung zum Aussenden des Messstrahls und/oder der Detektionseinrichtung noch zu verändern.

Der Träger ist bevorzugt durch eine Leiterplatte, eine Metallplatte oder Kunststoffplatte oder ein Gehäuse oder Gehäuseteil der Vorrichtung gebildet.

Es kann außerdem vorgesehen sein, dass die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist. Die genannten Elemente können gemeinsam aus einem Halbleiterrohling hergestellt, in einer Ausführungsform geätzt sein oder zumindest in einem gemeinsamen Gehäuse untergebracht sein.

Es kann zudem vorgesehen sein, dass das Modulationselement wenigstens ein gegenüber dem übrigen Halbleiterbaustein bewegliches und bezüglich einer Position steuerbares Element, insbesondere einen Spiegel aufweist. Dieser kann durch eine MEMS- Einrichtung ansteuerbar sein.

Es kann auch vorgesehen sein, dass das Modulationselement eine in ihrer Strahlungsdurchlässigkeit steuerbare Schicht aufweist.

Es kann auch vorgesehen sein, dass das Modulationselement eine elektronische Ansteuerschaltung für die Modulation der einen oder mehreren Laserelemente aufweist. Das Modulationselement kann in einer Ausführungsform derart beschaffen sein, dass es durch Interferenz, Phasenversatz/Gangversatz oder eine Polfiltereinrichtung oder andere bekannte Modulationsmechanismen den Anregungsstrahl zeitabhängig verändert.

Das oder die mikrooptischen Bauelemente können als in das Halbleiterbauelement integrierte oder aus diesem herausgearbeitete, insbesondere durch Ätzen herausgearbeitete Spiegel oder Linsen ausgeführt sein.

Die beschriebene Vorrichtung zum Analysieren eines Stoffes kann einen Messwert für eine Stoffkonzentration, in einer Ausführungsform eine Glucosekonzentration ermitteln. Die Vorrichtung kann eine Schnittstelle zu einer Anzeigeeinrichtung von Messwerten und ihrer Bewertung, beispielsweise durch einen Farbcode, für einen Benutzer der Vorrichtung und/oder zu einer Dosiereinrichtung für eine Substanz aufweisen, die in den Stoff, insbesondere das Gewebe oder allgemein den Körper eines Lebewesens abgegeben werden kann. Die Vorrichtung kann eine solche Dosiervorrichtung auch unmittelbar mit umfassen. Dabei kann die Vorrichtung zudem eine Einrichtung zur Erfassung oder Analyse der Stoffoberfläche, in einer Ausführungsform Hautoberfläche oder in einer Ausführungsform auch Augenfläche oder Iris eines Lebewesens aufweisen, die eine Identifizierung einer Person oder eines Lebewesens aufgrund eines Vergleiches mit Referenzdaten ermöglicht und somit dazu dienen kann, sicherzustellen, dass geeignete Referenzwerte und /oder Kalibrierungswerte für die Analyse des Stoffes und die Steuerung der Dosiervorrichtung bereitgestellt werden. Ermittelte Kennwerte der Stoffoberfläche, in einer Ausführungsform ein Fingerabdruck oder die Struktur einer Iris eines Auges, können außer zur Identifizierung und Authentifizierung einer Person, auch gegenüber einer Datenbank, ebenso zur Verschlüsselung der Kommunikation von Zustandswerten und der Steuerung der Dosiereinrichtung, die, verschlüsselt oder unverschlüsselt, grundsätzlich von der Datenbank aus geschehen kann, dienen. Die Dosiereinrichtung kann in einer Ausführungsform mit einem Sensor zur Ermittlung eines Füllstandes einer abzugebenden Substanz, wie in einer Ausführungsform Insulin und/oder Glucagon, Heparin oder ein Anestheticum versehen sein und eine Einrichtung aufweisen, um den Füllstand zu der Vorrichtung zur Stoffanalyse und/oder direkt an die Datenbank zu übermitteln.

Zudem kann die Vorrichtung eine Schnittstelle, in einer Ausführungsform eine Funkschnittstelle zu der Datenbank aufweisen, zu der die Messwerte geschickt werden können und die die Daten weiterverarbeiten kann. Die Datenbank kann derart beschaffen sein, dass sie die Daten mehrerer Patienten , das heißt in einer Ausführungsform auch die Daten von mehreren gleichartigen Vorrichtungen zum Analysieren eines Stoffes verarbeitet und speichert und in einer Ausführungsform auch individuelle Dosiereinrichtungen zur Abgabe von Substanzen steuert. Die Datenbank kann auch die ermittelten Daten über den analysierten Stoff weiter verarbeiten und abgeleitete Analyseergebnisse wie einen Trend der Werte, zeitliche erste und zweite Ableitungen, Minima, Maxima, Standardabweichungen von Stoffmengen oder - Konzentrationen, Blutzuckerwerten oder anderen physiologischen Werten von Patienten ermitteln, vergleichen und daraus Signale, in einer Ausführungsform auch Alarmsignale ableiten. Auch der Füllstand der Dosiereinrichtung kann von der Datenbank erfasst und verarbeitet werde, um in einer Ausführungsform eine zeitliche Reichweite der Füllung oder die Notwendigkeit einer Nachfüllung zu ermitteln und direkt an die Vorrichtung des Patienten oder eine Serviceeinrichtung zu signalisieren. Hierzu kann die Datenbank mit einer Kommunikationseinrichtung in einer Serviceeinrichtung, in einer Ausführungsform einem Krankenhaus oder einer Arztpraxis verbunden sein. Zum Zweck der Übermittlung von Daten von und/oder zu einer Datenbank kann die Vorrichtung in einer Ausführungsform mittels einer Funkverbindung, in einer Ausführungsform Bluetooth oder WLan oder Wifi oder anderer Übertragungsmethoden mit einem Mobilfunkgerät oder einem Pager verbunden sein. Die Vorrichtung kann auch unmittelbar mit einer WLAN- Schnittstelle und einem Internet-Client versehen sein.

Der Gegenstand bezieht sich außerdem auf ein Verfahren zum Analysieren eines Stoffes, wobei bei dem Verfahren mit einer Anregungssendeeinrichtung mindestens ein elektromagnetischer Anregungsstrahl, mit zumindest einer Anregungswellenlänge durch den wenigstens teilweise gleichzeitigen oder aufeinander folgenden Betrieb mehrerer Laseremitter einer Laserlichtquelle erzeugt wird, mit einer Detektionseinrichtung ein Reaktionssignal detektiert wird und anhand des detektierten Reaktionssignals der Stoff analysiert wird. Dabei kann die thermische Diffusivität in dem Stoff und der zeitliche Verlauf des Reaktionssignals zur Charakterisierung der Natur des Stoffes oder einer örtlichen Verteilung einer Substanz in dem Stoff dienen oder zur Charakterisierung der Tiefe, in der der Anregungsstrahl absorbiert wird.

Es kann in einer Ausführungsform vorgesehen sein, dass nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird.

Es kann auch vorgesehen sein, dass Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird. Bei der Verwendung von mehreren Modulationsfrequenzen des Pumpstrahls zur gleichen Zeit ist es beispielsweise möglich das detektierte Signal mittels eines entsprechenden Analyseverfahren, beispielsweise einer Integraltransformation, beispielsweise einer Fourier-Transformation, ihren Frequenzen entsprechend aufzulösen; die FT würde nur das Signal herausfiltern das der gewünschten Frequenz entspricht.

Es kann auch vorgesehen sein, dass ein optisches Medium mit dem Stoff, insbesondere einem ersten Bereich der Oberfläche des Stoffs, in direkten Kontakt gebracht wird, mit der Anregungssendeeinrichtung der ausgesendete Anregungsstrahl erzeugt und insbesondere derart ausgestrahlt wird, dass dieser in das optische Medium eindringt und dieses an einem vorbestimmten Punkt an der Oberfläche des optischen Mediums wieder verlässt, mit einer Einrichtung zum Aussenden eines Messstrahls ein Messstrahl, insbesondere Mess-Lichtstrahl, derart erzeugt wird, dass dieser in das optische Medium eindringt und dass insbesondere der Messstrahl und der Anregungsstrahl sich im Betrieb auf einer Grenzfläche des optischen Mediums und der Oberfläche des Stoffs, an der der Messstrahl reflektiert wird, überlappen, und mit der Detektionseinrichtung ein das Reaktionssignal bildender reflektierter Messstrahl gemessen und/oder direkt oder indirekt die Ablenkung des reflektierten Messstrahls detektiert wird.

Ein Aspekt des Verfahrens ist die Fokussierung der Messung des Reaktionssignals auf selektierte Tiefenbereiche unterhalb der (Abstandsintervalle von der) Stoffoberfläche. Die thermische Wellenlänge d hat dabei den größten Einfluss auf den mit dem Verfahren gemessenen Tiefenbereich. Sie ist definiert als
d = √(D/(π*f)). Wobei D die thermische Diffusivität der Probe (hier bspw. Haut) ist und f die Modulationsfrequenz des Anregungstrahls. Literatur zur thermischen Diffusivität von Haut:
- U. Werner, K. Giese, B. Sennhenn, K. Plamann, and K. Kölmel, "Measurement of the thermal diffusivity of human epidermis by studying thermal wave propagation," Phys. Med. Biol. 37(1), 21-35 (1992).
- A. M. Stoll, Heat Transfer in Biotechnology, Vol 4 of Advances in Heat Transfer, J. P. Hartnett and T. Irvin, eds. (New York, Academic, 1967), p 117.

Zur Eliminierung von Reaktionssignalen aus den obersten Schichten des Stoffes können in einer Ausführungsform Änderungen der Messwerte im Vergleich zu vorangegangenen Messungen herangezogen werden, falls die Messwerte in den obersten Schichten sich im Vergleich zu anderen, tieferliegenden Schichten weniger oder langsamer verändern. Dies kann in einer Ausführungsform bei Messungen an der menschlichen Haut der Fall sein, wo die obersten Hautschichten praktisch keinem Austausch mit den unteren Schichten unterliegen und deshalb physiologische Parameter wenig veränderlich sind. Es kann auch die zeitliche Ableitung von Messwerten zu Reaktionssignalen zum Ausschluss der Signale aus den obersten Hautschichten herangezogen werden. So kann die Messung oder zumindest die Auswertung auf die interstitielle Flüssigkeit in der Haut begrenzt oder fokussiert werden.

Außerdem kann vorgesehen sein, dass in Abhängigkeit von einer in dem Stoff ermittelten Stoffkonzentration eine Dosiereinrichtung zur Abgabe einer Substanz, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird. Dabei können außer einem aktuell ermittelten Messwert auch eine zeitliche Messwertentwicklung, eine Ableitung des Messwertes, Durchschnittwerte von Messwerten, Maxima, Minima, eine Standardabweichung sowie vorgegebene Schwellen für Messwerte berücksichtigt und mit dem aktuellen Messwert verknüpft werden. Die Verarbeitungseinrichtung kann in einer Ausführungsform eine Datenbank sein oder mit einer Datenbank verbunden sein, die Daten mehrerer Patienten sammelt und verarbeitet. Die Datenbank kann direkt mit einer Steuerungseinrichtung der Vorrichtung verbunden oder entfernt von dieser und über eine Kommunikationsschnittstelle angebunden sein.

Um eine erhöhte Sicherheit beim Betrieb einer Dosiereinrichtung, insbesondere für Insulin, zu erreichen, kann vorgesehen sein, dass diese mittels eines voreingestellten Standardverfahrens mit vorgewählten Mengenabgaben zu bestimmten oder bestimmbaren Zeiten lokal oder von einer Datenbank aus gesteuert betrieben wird und dass mittels der oben beschriebenen Vorrichtung sinnvolle Abweichungen von voreingestellten Abgabewerten ermittelt werden, die zu einer Korrektur und Verbesserung der Ansteuerung der Dosiereinrichtung verwendet werden. Auf diese Weise ist auch bei einem Ausfall der Vorrichtung zumindest ein Normal- oder Notbetrieb der Dosiereinrichtung gewährleistet.

Es kann bei dem erfindungsgemäßen Verfahren auch vorgesehen sein, kann dass die Messung, die die Erfassung des Antwortsignals bei einer oder mehreren Modulationsfrequenzen oder zeitaufgelöst nach einem Sendeimpuls und bei einer oder mehreren Wellenzahlen umfasst, nacheinander bei verschiedenen Eintrittswinkeln/Einfallwinkeln des Anregungsstrahls wiederholt wird und dass die Ergebnisse miteinander verknüpft, insbesondere voneinander subtrahiert werden, um die Einflüsse der oberen Hautschichten zu eliminieren oder zu reduzieren. Dabei kann wenigstens ein Einfallwinkel bei oder in der Nähe von 90 Grad, das heißt senkrechtem Eintritt des Anregungsstrahls in die Haut/den zu analysierenden Stoff liegen.

Dieses Konzept geht von der Überlegung aus, dass das Anregungssignal bei einem flacheren, kleineren Einfallwinkel eine längere Strecke in oberen und obersten Hautschichten zurücklegt als bei einem steileren, größeren Einfallwinkel. Damit wird bei einem kleineren Einfallwinkel auch ein größerer Anteil der Anregungsstrahlung in den oberen Hautschichten absorbiert als bei einem größeren Einfallwinkel, so dass sich der Einfluss der oberen Hautschichten wenigstens teilweise isolieren und durch Verknüpfung der Messergebnisse bei verschiedenen Einfallwinkeln eliminieren lasst.

Vorteilhaft dafür ist, dass sich der Bereich, in dem die Anregungsstrahlung absorbiert wird, durch die Änderung des Einfallwinkels nicht merklich parallel zu der Stoffoberfläche, insbesondere der Hautoberfläche verschiebt. Es können dabei Winkeländerungen zwischen den verschiedenen Eintrittswinkeln/Einfallwinkeln von wenigstens 5 Grad, insbesondere wenigstens 10 Grad, weiter insbesondere wenigstens 20 oder wenigstens 30 Grad vorgenommen werden. Die Winkeländerungen können dabei durch Brechung oder Reflexion des Anregungsstrahls außerhalb oder innerhalb des optischen Mediums, innerhalb des Mediums beispielsweise durch gezielte Änderung des Brechungsindex, beispielsweise mittels Anlegen einer elektrischen Spannung an den Kristall infolge eines elektrooptischen Effektes erreicht werden.

Es kann in dem oben erläuterten Rahmen auch ein Verfahren vorgesehen sein, bei dem die Messung das Erfassen des Antwortsignals bei wenigstens einer Modulationsfrequenz umfasst, welche so gewählt ist, dass die resultierende thermische Diffusionslänge, welche eine Funktion der Modulationsfrequenz ist, ein hinreichend tiefes Auslesen der Probe ermöglicht. Zudem können im Sinne der Tiefenprofilierung weitere Modulationsfrequenzen genutzt werden, die zum Charakterisieren der Oberfläche oder oberflächennaher Schichten des zu untersuchenden Stoffes dienen, bspw. Der oberen Hautschichten für die Haut und damit auch zur Eliminierung nicht relevanter Einflüsse dieser (bspw. Kontamination, Verunreinigungen, Absorption nicht relevanter Stoffe).

Zudem können bei der Messung des Antwortsignals bei mindestens einer oder mehreren Modulationsfrequenzen auch die Phasenlage des Antwortsignals mit in die Auswertung einfließen. So können Amplituden und Phasenlage von einer oder mehreren Frequenzen verknüpft werden, beispielsweise durch Subtraktion der Amplituden und/oder Berechnung phasenabhängiger Signalantworten.

Es kann zudem bei den erläuterten Verfahren vorgesehen sein, dass der Abtaststrahl während der Messung wenigstens zeitweise im Wellenzahlenbereich zwischen15000/cm und 16000/cm, insbesondere zeitweise zwischen 15500/cm und 16000/cm, weiter insbesondere zeitweise zwischen 15700/cm und 16000/cm betrieben wird.

Es kann außerdem vorgesehen sein, dass bei einer Messung, bei der der Anregungsstrahl nacheinander auf verschiedene Wellenzahlen und/oder Wellenzalbereiche eingestellt wird, wenigstens 5%, insbesondere wenigstens 10%, weiter insbesondere wenigstens 30%, weiter insbesondere wenigstens 50% des abgedeckten Wellenzahl- oder Wellenlängenbereiches in Bereichen des Spektrums liegt, die bezüglich des nachzuweisenden Stoffes, insbesondere Glukose, insensitiv sind.

Ein Bereich oder ein Punkt des Spektrums wird bzgl. eines in einer Probe enthaltenen, nachzuweisenden Stoffes im Rahmen der vorliegenden Anmeldung als insensitiv bezeichnet, wenn eine Absorptionsintensität der Probe im dem Bereich oder an dem Punkt des Spektrums unabhängig von der Menge und/oder Konzentration des Stoffes in der Probe ist. Dies bedeutet üblicherweise, dass der Stoff in diesem Bereich des Spektrums keine abgrenzbaren Absorptionsbanden aufweist.

Es kann außerdem für eine möglichst störungsfreie und genaue Messung vorgesehen sein, dass zur Berücksichtigung des von der Glukosekonzentration unabhängigen Absorptionsverhaltens der Probe oder des Gewebes wenigstens ein oder zwei Wellenzahlbereiche oder Wellenlängenbereiche ohne eine signifikante Absorption durch Glukose vorgesehen sind, in denen die Absorption vermessen wird.

Es kann hierzu vorgesehen sein, dass wenigstens einer der Wellenzahlbereiche zwischen 1610 und 1695 cm⁻¹ (Amid 1) liegt.

Es kann hierzu auch vorgesehen sein, dass wenigstens einer der Wellenzahlbereiche zwischen 1480 und 1575 cm⁻¹ (Amid 2) liegt.

Zudem kann vorgesehen sein, dass für eine Messung zunächst ein zu untersuchender Tiefenbereich in der Probe selektiert wird und dass danach der Anregungsstrahl derart gesteuert wird, dass zwischen Zeitbereichen (Anregungspulsen), in denen der Anregungsstrahl ausgesendet wird, jeweils mindestens ein Zeitraum liegt, der der Diffusionszeit einer thermischen Welle entspricht, die sie zum Durchlaufen der Strecke zwischen dem zu untersuchenden Tiefenbereich in der Probe und der Probenoberfläche benötigt.

Damit können bestimmte vorgegebene Stoffe gezielt in bestimmten Tiefen oder Tiefenbereichen unter der Oberfläche der Probe nachgewiesen werden, ebenso wie das Konzentrationsprofil in Abhängigkeit von der Tiefe. Im Bereich der Kosmetik oder der Pharmazie kann so beispielsweise die Eindringtiefe oder die Eindringgeschwindigkeit eines Stoffes in die Haut bestimmt werden, der durch die Hautoberfläche einzieht.

Es kann auch vorgesehen sein, dass der zeitliche Intensitätsverlauf der Absorption jeweils nach dem Ende eines Zeitbereichs gemessen wird, in dem der Anregungsstrahl ausgesendet wird. Alternativ oder zusätzlich zur Absorptiosintensität kann somit die Phasenlage des Antwortsignals bei der Auswertung berücksichtigt werden.

Zudem kann vorgesehen sein, dass zur Ansteuerung eines Lasers zur Erzeugung des Anregungsstrahls eine Reihe von gespeicherten äquidistanten oder nicht äquidistanten Einstellungswerten nacheinander eingestellt werden, die jeweils eine Wellenzahl/Wellenlänge des Lasers bestimmen und unter denen insbesondere wenigstens 3, weiter insbesondere wenigstens 5 Wellenzahlen/Wellenlängen von Absorptionsmaxima eines nachzuweisenden Stoffes sind. (lookup table)

Es kann auch vorgesehen sein, dass vor und/oder während und/oder nach einer Messung der mechanische Druck und/oder der Kraft pro Flächeneinheit erfasst wird, mit dem das optische Medium, in dem der Abtaststrahl reflektiert wird, gegen die Probe gedrückt ist. So können Einflüsse des Drucks auf die Probe oder das optische Medium aus den Messergebnissen herausgerechnet oder bei der Auswertung berücksichtigt werden.

Auch kann vorgesehen sein, dass vor und/oder während und/oder nach einer Messung die Feuchtigkeit der Umgebungsluft und/oder ein Feuchtigkeitsgehalt der Probe oder die Feuchtigkeit der oberen Schichten oder der Oberfläche der Probe ermittelt wird.

Damit können Einflüsse der Feuchtigkeit in der Probe oder in der Messeinrichtung, aus den Messergebnissen herausgerechnet oder bei der Auswertung berücksichtigt werden.

Die Erfindung bezieht sich zudem auf ein Verfahren zum Analysieren eines Stoffes, wobei bei dem Verfahren mit einer Anregungssendeeinrichtung mindestens ein elektromagnetischer Anregungsstrahl (SA) mit zumindest einer Anregungswellenlänge durch den aufeinander folgenden Betrieb einer oder mehrerer oder durch den wenigstens teilweise gleichzeitigen Betrieb mehrerer Laseremitter einer Laserlichtquelle erzeugt wird,
mit einer Detektionseinrichtung ein Reaktionssignal (SR) detektiert wird und anhand des detektierten Reaktionssignals (SR) der Stoff analysiert wird.

Durch den aufeinander folgenden Betrieb einer oder mehrerer oder durch den wenigstens teilweise gleichzeitigen Betrieb mehrerer Laseremitter können die verschiedenen Wellenlängen erzeugt werden, die bei charakteristischen Wellenlängen einer in dem Stoff nachzweisenden Substanz, beispielsweise bei oder in der Nähe von Absorptionsmaxima liegen können.

Außerdem bezieht sich die Erfindung auf ein Verfahren bei dem die Temperatur des /der Laseremitter der Anregungssendeeinrichtung und/oder die Temperatur der Detektionseinrichtung insbesondere eines optischen Mediums und/oder einer Detektionsstrahlungsquelle und/oder eines optischen Sensors während der Analyse konstant gehalten, insbesondere auf einer vorgegebenen Temperatur konstant gehalten, weiter insbesondere auf einer vorgegebenen Temperatur oberhalb der Körpertemperatur eines Menschen, weiter vorzugsweise oberhalb von 37 Grad Celsius, weiter vorzugsweise oberhalb von 38 oder 40 Grad Celsius gehalten wird.

Durch diese Maßnahme kann der Temperatureinfluss auf die Messung gering gehalten werden. Auch eine Aufheizung durch den Körperkontakt kann vermieden werden, wenn die Messeinrichtung bei einer Temperatur bei oder über der Körpertemperatur gehalten wird, zumindest während der Messung. Dies kann durch eine Temperaturregeleinrichtung mit einem Heizelement und einem oder mehreren Sensoren geschehen.

Die Erfindung bezieht sich zudem auf ein Verfahren, bei dem während der Analyse die Temperatur des /der Laseremitter der Anregungssendeeinrichtung und /oder die Temperatur der Detektionseinrichtung, insbesondere eines optischen Mediums und/oder einer Detektionsstrahlungsquelle und /oder eines optischen Sensors erfasst und bei der Auswertung der Analyse durch Verknüpfung der Messergebnisse mit Temperaturkorrekturwerten berücksichtigt wird.

Bei dieser Methode werden die Temperatureinflüsse rechnerisch berücksichtigt.

Eine weitere Ausgestaltung der Erfindung kann vorsehen, dass der Stoff, insbesondere ein Körperteil, insbesondere ein Finger, gegen ein optisches Medium gedrückt wird, das Teil der Detektionseinrichtung ist, dass der auf das Medium durch das angedrückte Körperteil ausgeübte Druck erfasst wird und dass in Abhängigkeit von dem erfassten Druck auf das Medium die Anregungssendeeinrichtung eingeschaltet und/oder diese in Abhängigkeit von einer Verminderung des Drucks, insbesondere unter eine bestimmte Schwelle, ausgeschaltet wird. Es wird auf diese Weise durch einen Drucksensor ermittelt, ob die Messeinrichtung gerade benutzt wird und/oder ob das optische Medium gerade durch einen Gegenstand abgedeckt ist, an dem gemessen wird. Nur für diesen Fall wird die Anregungssendeeinrichtung freigeschaltet. Auf diese Weise wird verhindert, dass ein größerer Anteil des Anregungsstrahls über das optische Medium oder auch auf sonstige Weise nach außen in die Umwelt gelangt, wenn keine Messung/Analyse stattfindet.

Es kann auch vorgesehen sein, dass der Stoff, insbesondere ein Körperteil, insbesondere ein Finger, gegen ein optisches Medium gedrückt wird, das Teil der Detektionseinrichtung ist, dass eine Verdunkelung des Mediums im Bereich des angedrückten Körperteils erfasst wird und dass durch Verdunkelung des Mediums die Anregungssendeeinrichtung eingeschaltet und/oder diese durch eine Vergrößerung der Helligkeit im Medium insbesondere über eine bestimmte Schwelle, ausgeschaltet wird.

Es wird somit durch einen Helligkeitssensor/Fotodetektor ermittelt, ob die Messeinrichtung gerade benutzt wird und/oder ob das optische Medium gerade durch einen Gegenstand abgedeckt ist, an dem gemessen wird. Nur für diesen Fall wird die Anregungssendeeinrichtung freigeschaltet.

Außerdem kann auch vorgesehen sein, dass der Stoff, insbesondere ein Körperteil, insbesondere ein Finger, gegen ein optisches Medium gedrückt wird, das Teil der Detektionseinrichtung ist, dass ein Feuchtigkeitsgrad des Mediums im Bereich des angedrückten Körperteils erfasst wird und dass durch Erreichen eines vorgegebenen Feuchtigkeitsgrads an dem Medium die Anregungssendeeinrichtung eingeschaltet und/oder diese durch eine Verminderung des Feuchtigkeitsgrades, insbesondere unter eine bestimmte Schwelle, ausgeschaltet wird.

Es wird also in dieser Konstellation durch einen Feuchtigkeitssensor ermittelt, ob die Messeinrichtung gerade benutzt wird und/oder ob das optische Medium gerade durch einen Gegenstand abgedeckt ist, an dem gemessen wird. Nur für diesen Fall wird die Anregungssendeeinrichtung freigeschaltet.

Eine weitere Ausgestaltung der Erfindung kann vorsehen, dass während der Durchführung des Verfahrens ein an das optische Medium gedrücktes Körperteil an dem Medium fixiert, insbesondere durch Einklemmen des Körperteils an dem Medium, Verkleben des Körperteils mit dem Medium, Adhäsion des Körperteils an dem Medium oder durch Vakuum- Ansaugen des Köperteils an das Medium fixiert wird.

Somit werden stabilere Messbedingungen geschaffen, so dass eine bestimmte Mindestzeit für die Messung zur Verfügung steht. Die Messgenauigkeit und - zuverlässigkeit wird so erhöht.

Zudem kann vorgesehen sein, dass der Anregungsstrahl(SA) mit mindestens zwei oder mehr als zwei Anregungswellenlängen oder Gruppen von Anregungswellenlängen ausgesendet wird, wobei eine erste Anregungswellenlänge oder Gruppe von Anregungswellenlängen den Nachweis einer in dem Stoff nachzuweisenden Substanz ermöglicht, während wenigstens eine weitere Anregungswellenlänge oder Gruppe von Anregungswellenlängen den Nachweis einer von einer nachzuweisenden Substanz verschiedenen Referenzsubstanz ermöglicht und dass für die wenigstens zwei verschiedenen Substanzen Profile bezüglich ihrer Dichteverteilung in der Tiefe des Stoffes ermittelt und miteinander verknüpft werden, und wobei insbesondere ein Tiefenprofil oder wenigstens ein oder mehrere Parameter eines Tiefenprofils der Referenzsubstanz in dem Stoff bekannt ist.

Diese Methode nutzt den Umstand aus, dass das Tiefenprofil mehrerer Substanzen unabhängig voneinander durch geeignete Wahl der Anregungswellenlängen ermittelbar ist. Ist dabei ein Tiefenprofil einer Substanz bekannt, die in die Haut eingebracht ist oder dort ohnehin gemäß physiologischen Gesetzmäßigkeiten profiliert vorhanden ist, so kann die Tiefendimension einer anderen vermessenen Substanz daran geeicht werden.

Es kann auch vorgesehen sein, dass der Anregungsstrahl (SA) moduliert, vorzugsweise nacheinander mit verschiedenen Modulationsfrequenzen moduliert wird, wobei Reaktionssignale für verschiedene Modulationsfrequenzen detektiert werden oder ein Zeitverhalten des Reaktionssignals bei einer Änderung der Intensität des Anregungsstrahls, insbesondere bei dessen periodischem an- und Abschalten, analysiert wird.

Diese Messmethode lässt die Ermittlung der Tiefendimension, aus der die Reaktionssignale auf den Anregungsstrahl kommen, ohne die Verwendung verschiedener Modulationsfrequenzen oder mit nur wenigen Modulationsfrequenzen zu. Die aus verschiedenen Tiefen stammenden Reaktionssignale lassen sich einerseits an der Abhängigkeit ihrer Intensität von der Modulationsfrequenz bestimmen, jedoch auch an dem Zeitverhalten der Reaktionssignale bei einer Intensitätsänderung des Anregungsstrahls (z.B. bei seiner Abschaltung). Diese Verhalten der Reaktionssignale wir bei periodischer Intensitätsänderung des Anregungsstrahls (zB . Pulsieren, An- und Abschalten) phasensensitiv gemessen.

Signale aus geringeren Tiefen folgen dabei den Intensitätsänderungen des Anregungsstrahls schneller als Signale aus größeren Tiefen.

Dabei kann vorgesehen sein, dass die Modulation des Anregungsstrahls (SA) durch die Ansteuerung einer den Anregungslichtstrahl erzeugenden Lasereinrichtung (100) erfolgt.

Eine weitere Ausgestaltung der Erfindung kann vorsehen, dass der Anregungsstrahl mit zwischen 0 und 10 Modulationsfrequenzen, vorzugsweise mit zwischen 0 und 5 Modulationsfrequenzen, weiter vorzugweise mit zwischen 1 und 3 Modulationsfrequenzen, weiter vorzugsweise nur mit einer Modulationsfrequenz moduliert wird und das Zeitverhalten des Reaktionssignals, insbesondere ein phasensensitives Verhalten des Reaktionssignals analysiert wird.

Es kann auch vorgesehen sein, dass das Tastverhältnis des Anregungsstrahls (SA) zwischen 3% und 60%, vorzugsweise zwischen 3% und 50%, weiter vorzugsweise zwischen 3% und 7% liegt.

Eine weitere Ausgestaltung der Erfindung kann vorsehen, dass die Leistungsdichte des Anregungsstrahls (SA) an der Oberfläche des zu analysierenden Stoffes kleiner ist als 5 mW/mm2, insbesondere kleiner als 2 mW/mm2, weiter insbesondere kleiner als 1mW/mm2.

Damit wird eine Aufheizung des zu analysierenden Stoffs vermieden und die Laserlichtquelle wird geschont.

Es kann auch vorgesehen sein, dass das optische Medium der Detektionseinrichtung mit einem Körperteil, insbesondere einem Finger und/oder eine Dialysat führenden Gefäß und/oder einem Blut führenden Gefäß eines Dialysegerätes in Kontakt gebracht wird und dass insbesondere eine oder mehrere Anregungswellenlängen gewählt werden, die den Nachweis von Harnstoff, Cholesterol, Albumin, Protein, Kreatinin, Lactaten oder Phosphaten ermöglichen.

Damit kann sowohl der Dialyseprozess überwacht werden, als auch ein Monitoring von Dialysepatienten dauerhaft mit der erfindungsgemäßen Messeinrichtung stattfinden.

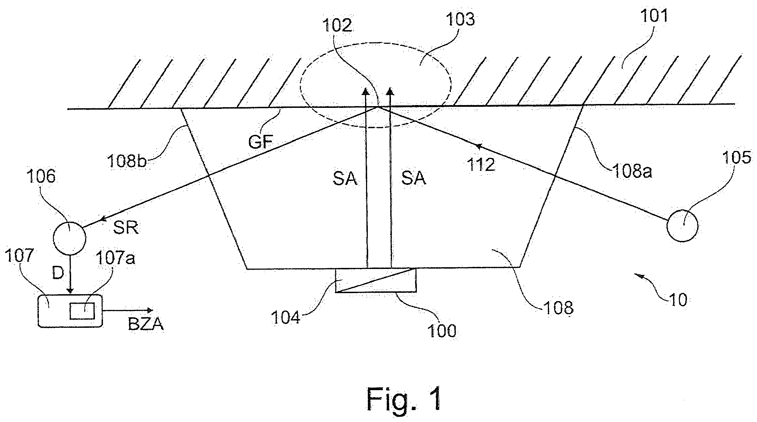

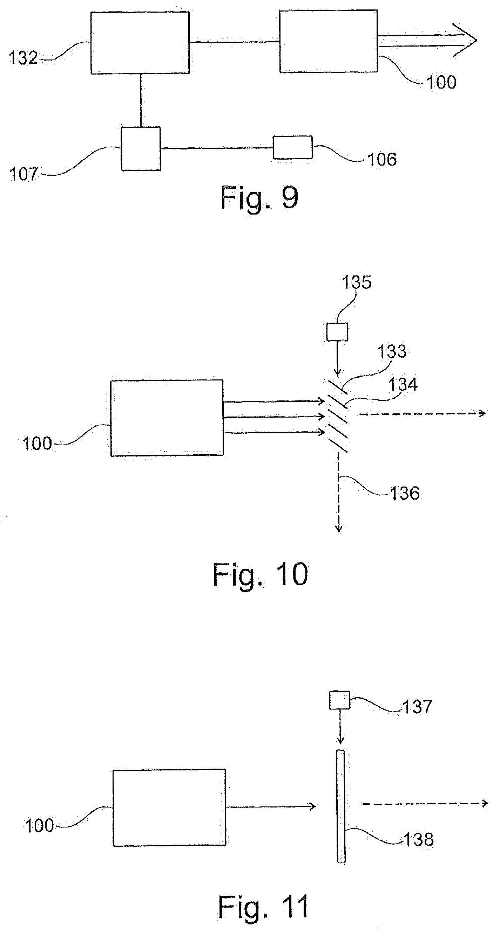

Die Figuren 1 bis 24 zeigen schematisch verschiedene Elemente der Vorrichtung und ihrer Elemente teilweise in unterschiedlichen Ausführungsformen sowie Konzepte der Erfindung und ihrer Anwendung.

Die Figur 1 zeigt ein Ausführungsbeispiel für eine Vorrichtung 10 zum Analysieren eines Stoffes 101. Der Stoff 101 liegt vorzugsweise unmittelbar auf einem optischen Medium 108 auf, das als optisch transparenter Kristall oder Glaskörper oder Kunststoffkörper oder Kunststoffkristall ausgebildet sein kann. Die Vorrichtung zum Analysieren des Stoffes 101 dient zum Beispiel zum Messen des Glukose- bzw. Blutzuckergehalts in einer Flüssigkeit, wie in einer Ausführungsform Blut, und zur Erzeugung einer Glukose- bzw. Blutzuckerspiegelangabe BZA.

Die Vorrichtung umfasst eine Anregungssendeeinrichtung 100 zum Aussenden eines oder mehrerer elektromagnetischer Anregungsstrahlen SA, vorzugsweise in Form von Anregungs-Lichtstrahlen mit einer oder mehreren Anregungswellenlängen, in ein Volumen 103, das in dem Stoff 101 unterhalb eines ersten Bereiches 102 der Oberfläche des Stoffes liegt. Die Anregungssendeeinrichtung 100 wird nachfolgend auch kurz als Anregungs-Lichtquelle 100 bezeichnet. Bei der Anregungs-Lichtquelle 100 kann es sich um einen bezüglich der Wellenlänge durchstimmbaren Laser, insbesondere durchstimmbaren Quantenkaskadenlaser, handeln; bevorzugt wird, wie weiter unten noch erläutert, eine Lichtquellenleiste oder ein Lichtquellenarray mit zumindest zwei Einzelemittern, insbesondere Halbleiterlasern, eingesetzt, mit denen jeweils eine vorgegebene individuelle Wellenlänge emittiert wird.

Außerdem ist eine Einrichtung 104 zur Intensitätsmodulierung des oder der Anregungslichtstrahlen SA vorgesehen, die vorzugsweise durch eine Modulationseinrichtung für die Anregungs-Lichtquelle, insbesondere ihrer Ansteuerung, und/oder wenigstens einen im Strahlengang angeordneten gesteuerten Spiegel und/oder eine bzgl. ihrer Transparenz steuerbare und im Strahlengang angeordnete Schicht gebildet ist.

Zudem weist die Vorrichtung eine Einrichtung 105 zum Aussenden eines elektromagnetischen Messstrahls 112, insbesondere Mess-Lichtstrahls, auf, der an der Grenzfläche GF zwischen dem Stoff 101 und dem optischen Medium 108 reflektiert, vorzugsweise totalreflektiert, wird.

Eine Detektionseinrichtung 106 dient zur Detektion des reflektierten Messstrahls 112, der ein zeitabhängiges Reaktionssignal SR bildet; die Amplitude des Reaktionssignals SR wird von der Wellenlänge des Anregungslichts SA und der Intensitäts-Modulation des Anregungslichts SA beeinflusst, wie weiter unten anhand von Beispielen noch näher erläutert wird.

Die Amplitude des Messsignals hängt hierbei von der Wellenlänge des Anregestrahls, den Absorptionseigenschaften der Probe, sowie den thermischen Eigenschaften, insbesondere der thermischen Diffusivität und thermischen Leitfähigkeit der Probe als auch des optischen Elementes ab. Darüber hinaus spielt auch die Kopplung des thermischen Signals von der Probe in das optische Element hinein eine Rolle.

Eine Einrichtung 107 zum Analysieren des Stoffes wertet die detektierten Reaktionssignale SR aus und erzeugt in einer Ausführungsform eine Glukose- bzw. Blutzuckerspiegelangabe BZA.

Nachfolgend soll der Betrieb der Vorrichtung 10 gemäß Figur 1 und in diesem Zusammenhang ein Verfahren zum Analysieren eines Stoffs 101 beispielhaft näher für den Fall beschrieben werden, dass es sich bei dem zu analysierenden Stoff 101 um menschliches oder tierisches Gewebe handelt und im Rahmen der Analyse des Stoffs eine Glukose- bzw. Blutzuckerspiegelangabe BZA ermittelt werden soll.

Mit der Einrichtung 105 wird ein elektromagnetischer Messstrahl 112, bei dem es sich vorzugsweise um einen Lichtstrahl im sichtbaren Wellenlängenbereich oder um einen Infrarotlichtstrahl handelt, in das optische Medium 108 eingestrahlt; dieser Messstrahl 112 trifft unterhalb des ersten Bereichs 102 der Oberfläche des Gewebes auf die Grenzfläche GF. An der Grenzfläche GF wird der Messstrahl 112 reflektiert und gelangt zur Detektionseinrichtung 106, die den reflektierten Messstrahl 112 misst.

Gleichzeitig werden mit der Anregungs-Lichtquelle 100 eine oder mehrere Anregungsstrahlen SA, bei denen es sich vorzugsweise um Infrarotstrahlen handelt, erzeugt. Die Wellenlänge des oder der Infrarotstrahlen liegt vorzugsweise in einem Bereich zwischen 3 µm und 20 µm, besonders bevorzugt in einem Bereich zwischen 8 µm und 11 µm.

Die Anregungsstrahlen SA werden mit der Einrichtung 104 zur Intensitätsmodulierung intensitäts- bzw. amplitudenmoduliert. in einer Ausführungsform werden mit der Einrichtung 104 zur Intensitätsmodulierung kurze Lichtpulse erzeugt, vorzugsweise mit einer Pulsfrequenz zwischen 1 kHz und 10 MHz, weiter vorzugsweise zwischen 1 kHz und 3 MHz oder Pulspakete (Doppel- oder Mehrfachmodulation), vorzugsweise mit einer Frequenz der Einhüllenden von 1Hz-10 kHz.

Die modulierten Anregungsstrahlen SA werden in das optische Medium 108 eingekoppelt und gelangen nach Passieren der Grenzfläche GF in das Volumen 103 innerhalb des Gewebes.

Die Wellenlänge der Anregungsstrahlen SA ist - mit Blick auf die hier beispielhaft erläuterte Blutzuckermessung - vorzugsweise derart gewählt, dass die Anregungsstrahlen SA von Glukose bzw. Blutzucker signifikant absorbiert werden. Zur Messung von Glukose bzw. Blutzucker sind folgende Infrarotwellenlängen besonders gut geeignet (Vakuumwellenlängen): 8,1 µm, 8,3 µm, 8,5 µm, 8,8 µm, 9,2 µm, 9,4 µm und 9,7 µm. Zudem können glucosetolerante Wellenlängen verwendet werden, die von Glucose nicht absorbiert werden, um andere vorhanden Stoffe zu ermitteln und deren Einfluss auf die Messung auszuschließen.

Durch die Absorption der Anregungsstrahlen SA im Gewebe wird im Bereich des Volumens 103 eine lokale Temperaturerhöhung hervorgerufen, die einen Wärmetransport und Wärmewellen und damit einhergehend auch Druckwellen in Richtung auf die Grenzfläche GF auslöst; durch die dadurch an der Grenzfläche GF auftretenden Temperatur- und Druckschwankungen werden die Brechzahl bzw. die Deformation, Mikrostruktur und das Reflexionsverhalten im Bereich 102 bzw. im Reflexionsbereich der Grenzfläche GF moduliert und der Strahlengang der Messstrahlen 112 beeinflusst.

Geht man beispielsweise davon aus, dass ohne Anregungsstrahlen SA die Ausrichtung zwischen der Einrichtung 105 und der Detektionseinrichtung 106 optimal ist und eine maximale Empfangsleistung von der Detektionseinrichtung 106 detektiert wird, so kann aufgrund der Absorption der Anregungsstrahlen SA im Bereich des Volumens 103 und aufgrund des Wärmetransports und der Druckwellen eine (zumindest temporäre) Veränderung der Amplitude oder, bei einer periodischen Modulation, der Phase des reflektierten Messstrahls 112 hervorgerufen werden bzw. eine Intensitätsmodulation des reflektierten Messstrahls 112 auftreten. Der Umfang der Intensitätsmodulation hängt von der Wellenlänge der Anregungsstrahlen SA (wegen der nötigen Absorption im Gewebe) und von der Pulsfrequenz der Anregungsstrahlen SA (wegen des Temperaturtransports und der Druckwellen vom Gewebeinneren in Richtung Grenzfläche GF) und den thermischen Eigenschaften der Probe und des Mediums ab.

Die Änderung der Reflexion des Messstrahls 112 bzw. die zeitabhängige Änderung des Reaktionssignals SR wird von der Detektionseinrichtung 106 quantitativ erfasst, und das Detektionsergebnis D gelangt zur Einrichtung 107.

Die Einrichtung 107 kann anhand von vorab durchgeführten Kalibrations- bzw. Vergleichsmessungen, die in einer Ausführungsform in Form von Vergleichstabellen oder Vergleichskurven in einem Speicher 107a der Einrichtung 107 abgespeichert sind, auf die jeweils aktuelle Konzentration von Glukose bzw. Blutzucker innerhalb des Gewebes bzw. innerhalb des Volumens 103 schließen und eine entsprechende Glukose- bzw. Blutzuckerspiegelangabe BZA erzeugen. Die Vergleichstabellen oder Vergleichskurven können beispielsweise auf der Basis von Glukose- oder Blutzuckerwerten erstellt worden sein, die anhand von Blutproben ermittelt worden sind.

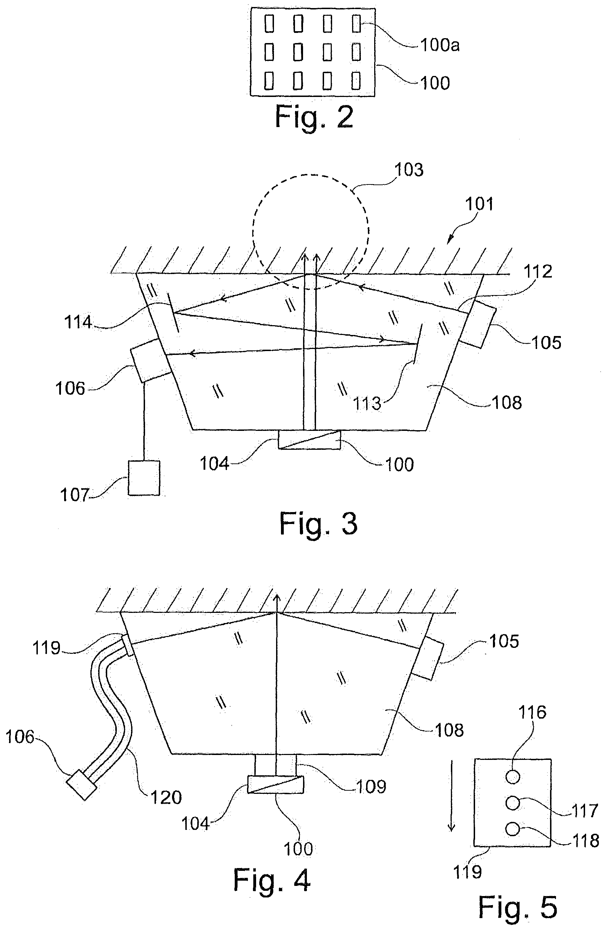

Besonders bevorzugte Ausgestaltungen und Varianten von Vorrichtungen 10 zum Analysieren eines Stoffes 101 werden nachfolgend unter Bezugnahme auf die Figuren 2 bis 10 erläutert.

Die Anregungssendeeinrichtung 100 zum Aussenden des oder der Anregungs-Lichtstrahlen kann als Array ausgebildet sein, wie in der Figur 2 dargestellt ist. Das Array weist wenigstens 5, vorteilhaft wenigstens 10, weiter vorteilhaft wenigstens 15 oder wenigstens 50 oder 100 einzeln ansteuerbare Emitter 100a für jeweils monochromatisches Licht im Absorptionsspektrum eines zu analysierenden Stoffes auf.

Das Array erzeugt vorzugsweise Strahlen mit monochromatischem Licht mit einer oder mehreren, besonders bevorzugt allen der folgenden Wellenlängen (Vakuumwellenlängen): 8,1 µm, 8,3 µm, 8,5 µm, 8,8 µm, 9,2 µm, 9,4 µm und 9,7 µm sowie, falls gewünscht zusätzlich glucosetolerante Wellenlängen.

Die Einrichtung 105 zum Aussenden des Mess-Lichtstrahls 112 und die Detektionseinrichtung 106 können von dem optischen Medium 108 separat angeordnet sein, wie in Figur 1 gezeigt. Mit Blick auf einen minimalen Platzbedarf und minimalen Montageaufwand wird es als vorteilhaft angesehen, wenn die Einrichtung 105 zum Aussenden des Mess-Lichtstrahls 112 und die Detektionseinrichtung 106 unmittelbar an dem optischen Medium 108 angebracht sind, vorzugsweise an gegenüberliegenden Flächenabschnitten 108a und 108b des optischen Mediums 108, wie dies die Figur 3 zeigt.

Es kann vorgesehen sein, dass die Anregungssendeeinrichtung/Anregungs-Lichtquelle 100 unmittelbar oder mittels einer Justagevorrichtung 109 mit dem optischen Medium 108 mechanisch fest verbunden ist. Die Justagevorrichtung 109 ermöglicht vorzugsweise eine Justage des Abstands der Anregungs-Lichtquelle 100 von dem optischen Medium 108 bzw. eine Justage in Strahllängsrichtung und/oder eine Justage in der Ebene senkrecht dazu (vgl. Figur 4).

Wie in den Figuren 3, 4, 6, 7 und 8 gezeigt, kann die Einrichtung 105 zum Aussenden des Mess-Lichtstrahls 112 in den Bereich des optischen Mediums 108 vorgesehen sein, der mit dem ersten Bereich 102 der Stoffoberfläche in Kontakt steht. Eine solche Anordnung ermöglicht das Einstrahlen des Mess-Lichtstrahls 112 in einem flachen Winkel und das Hervorrufen einer Totalreflexion an der Grenzfläche des optischen Mediums 108 zum Stoff 101.

Durch die Einstrahlung in einem flachen (kleinen) Winkel (zur Probeoberfläche) kann die Mirage Deflektion analog zu der bekannten photothermischen 'Bouncing Method' effektiver werden und gleichzeitig die deformationsbedingte Ablenkung des Messstrahls reduziert werden. Der Winkel zwischen der Probeoberfläche und dem Messstrahl kann in einer Ausführungsform kleiner als 20 Grad, kleiner als 10 Grad, insbesondere kleiner als 5 Grad, weiter insbesondere kleiner als 2 Grad oder 1 Grad gewählt werden, um diesen Effekt auszunutzen.

Umgekehrt kann durch die Einstrahlung in steileren (größeren) Winkeln (zur Stoffoberfläche) die Deflektion in Analogie zur der bekannten photothermischen ,Bouncing Method' effektiver werden und gleichzeitig die Mirage-Effekt bedingte Ablenkung des Messstrahls reduziert werden. Der Winkel zwischen der Stoffoberfläche und dem Messstrahl kann in einer Ausführungsform größer als 20 Grad, größer als 30 Grad, insbesondere größer als 45 Grad, weiter insbesondere größer als 60 Grad oder 70 Grad gewählt werden, um diesen Effekt auszunutzen.

Siehe Literatur hierzu:
- M. Bertolotti, G.L. Liakhou, R. Li Voti, S. Paolino, and C. Sibilia. Analysis of the photothermal deflection technique win the surface refection theme: Theory and Experiment. Journal of Applied Physics 83, 966 (1998)

Die Einrichtung 105 zum Aussenden des Mess-Lichtstrahls 112 und/oder die Detektionseinrichtung 106 zur Detektion des Mess-Lichtstrahls 112 bzw. des Reaktionssignals SR können tragend mit dem optischen Medium 108 mechanisch unmittelbar oder mittels einer Justageeinrichtung fest verbunden sein und/oder mittels eines oder mehrerer Lichtwellenleiter 120 an dieses angekoppelt sein.

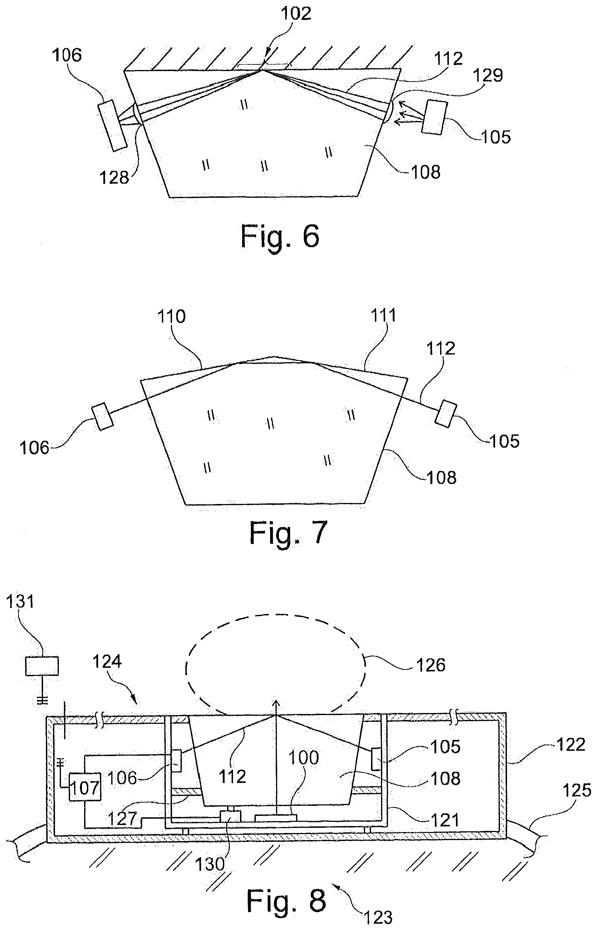

Es kann auch, wie in Figur 6 gezeigt, vorgesehen sein, dass das optische Medium 108 unmittelbar eine Abbildungsoptik 128 und/oder eine Abbildungsoptik 129 (jeweils) in Form einer Linse oder eines anderen Reflektions- oder Beugungsmittels trägt und/oder dass in das optische Medium 108 eine Abbildungs-Optik integriert ist. Die Abbildungsoptik kann jedoch auch in Form einer Linse oder eines anderen Reflexions- oder Beugungselementes in die Anregungssendeeinrichtung oder die Einrichtung zur Erzeugung des Messstrahls integriert sein, beispielsweise, wenn diese als integrierte Bauelemente und/oder als Halbleiterbauelemente ausgebildet sind. Die Abbildungsoptik kann in einer Ausführungsform aus demselben Halbleiterelement durch Ätzen herausgearbeitet sein wie der jeweilige integrierte Schaltkreis, der eine Strahlungsquelle für den Anregungs- oder Messstrahl aufweist.

Es kann auch, wie in Figur 7 gezeigt, vorgesehen sein, dass die Oberfläche des optischen Mediums 108 mehrere gegeneinander geneigte Teilflächen 110, 111 aufweist, an denen der Mess-Lichtstrahl 112 mehrfach reflektiert oder gebrochen wird.

Es kann zudem, wie in Figur 3 dargestellt, vorgesehen sein, dass in oder an dem optischen Medium 108 ein oder mehrere Spiegelflächen 113, 114 zur Reflektion des Mess-Lichtstrahls 112 (und damit des Reaktionssignals SR) vorgesehen sind. Diese Spiegelflächen können durch Inhomogenitäten innerhalb des optischen Mediums 108 oder durch dessen Außenflächen oder durch integrierte/eingepasste/eingegossene oder an dem optischen Medium befestigte, beispielsweise metallische oder metallische beschichtete, Spiegelelemente gebildet sein. Hierdurch wird der optische Weg des Mess-Lichtstrahls 112 in dem optischen Medium 108 bis zum Eintritt in die Detektionseinrichtung 106 verlängert, so dass eine reaktionssignalabhängige Ablenkung des Mess-Lichtstrahls 112 bei der Reflexion an dem Bereich der Oberfläche des Mediums 108, der mit dem ersten Bereich 102 der Stoffoberfläche im Kontakt steht, innerhalb des optischen Mediums 108 vergrößert wird. Die Ablenkung ist dann in der Detektionseinrichtung 106 als absolute Ablenkung nachweisbar.

Die Detektionseinrichtung 106 kann mehrere optisch sensitive Flächen, wie optisch sensitive Halbleiterdioden aufweisen oder auch in einem Anschlusskörper 119 mehrere versetzte Öffnungen 116, 117, 118 (Figur 5), an denen einzelne Lichtwellenleiter 120 (Figur 4) enden, in die das Licht des Messlichtstrahls 112 in Abhängigkeit von seiner Ablenkung eingekoppelt wird. Die Lichtwellenleiter 120 sind dann an einem Anschlusskörper 119, der an dem optischen Medium 108 befestigt sein kann, angeschlossen und leiten das Licht zu dem am Ende der Lichtwellenleiter 120 angeordneten Teil der Detektionseinrichtung 106 (Figur 4). Der Anschlusskörper 119 ist dann, ebenso wie die Lichtwellenleiter 120, auch ein Teil der Detektionseinrichtung 106 zur Detektion des Mess-Lichtstrahls.

Der Vollständigkeit halber soll festgestellt werden, dass auch die Anregungssendeeinrichtung die Anregung ganz oder abschnittsweise mittels eines oder mehrerer Lichtwellenleiter zu der Stoffoberfläche senden kann. in einer Ausführungsform kann die Anregungssendeeinrichtung unmittelbar mit einem oder mehreren Lichtwellenleitern gekoppelt sein die an das optische Medium angekoppelt sind.

Es kann auch, wie in Figur 8 dargestellt, vorgesehen sein, dass die Anregungssendeeinrichtung 100, die Einrichtung 105 zum Aussenden des Mess-Lichtstrahls 112 und die Detektionseinrichtung 106 unmittelbar aneinander oder an einem gemeinsamen Träger 121 befestigt sind. Der Träger kann durch ein Kunststoffteil, eine Leiterplatte oder ein Metallblech gebildet sein, das in einem Gehäuse 122 montiert ist. Der Träger, der in der Figur 8 im Querschnitt U-förmig ausgebildet ist, kann dann das optische Medium 108 in einer Ausführungsform wenigstens teilweise umgeben. Auch das optische Medium kann an dem Träger befestigt sein und gegenüber diesem justiert werden.

Der Träger kann auch durch das Gehäuse 122 selbst oder ein Gehäuseteil gebildet sein.

Es kann auch vorgesehen sein, dass die Vorrichtung mit dem Gehäuse 122 am Körper 123 einer Person befestigbar ist, wobei die Anregungssendeeinrichtung 100 zum Aussenden eines oder mehrerer Anregungs-Lichtstrahlen SA, die Einrichtung 105 zum Aussenden des Mess-Lichtstrahls 112 und die Detektionseinrichtung 106 zur Detektion des zeitabhängigen Reaktionssignals SR derart angeordnet und eingerichtet sind, dass die zur Messung geeignete Seite (mit einem für die Anregungsstrahlung transparenten Messfenster) der Vorrichtung auf der dem Körper abgewandten Seite der Vorrichtung liegt, so dass der zu analysierende Stoff auf der dem Körper 123 abgewandten Seite 124 des Gehäuses 122 vermessbar ist. Hierzu ist in der Figur 8 dargestellt, dass das Gehäuse 122 mittels eines zum Gehäuse gehörenden Riemens 125 am Körper 123 einer Person, in einer Ausführungsform in Form eines Armbandes an einem Handgelenk befestigt ist. Auf der dem Handgelenk abgewandten Seite 124 weist dann das Gehäuse ein für den Anregungs-Lichtstrahl SA durchlässiges Fenster auf, oder das optische Medium 108 ist unmittelbar in die nach außen weisende Seite 124 des Gehäuses eingepasst und bildet selbst abschnittsweise die Oberfläche des Gehäuses.

Wie in Figur 8 dargestellt, kann dann eine gestrichelt schematisiert dargestellte Fingerbeere 126 auf das optische Medium 108 aufgelegt und vermessen werden.

Das optische Medium 108 kann innerhalb des Gehäuses 122, ebenso wie der Träger 121, oder unmittelbar an dem Gehäuse 122 befestigt sein. Das optische Medium 108 kann auch unmittelbar mit dem Träger 121 verbunden sein, wobei eine Justageeinrichtung 127 zur relativen Positionierung des Trägers 121 zum optischen Medium 108 vorgesehen sein sollte.

Es ist auch denkbar, die Anregungs-Lichtquelle 100, die Einrichtung 105 und die Detektionseinrichtung 106 oder auch nur eines oder zwei der genannten Elemente unmittelbar an dem optischen Medium 108 und das oder die jeweils anderen Elemente an dem Träger 121 zu befestigen.

Durch das optische Fenster in dem Gehäuse 122 und/oder durch das optische Medium 108 hindurch können auch andere Parameter der Stoffoberfläche bzw. der aufgelegten Fingerbeere 126 vermessbar sein, wie in einer Ausführungsform ein Fingerabdruck. Hierzu kann in dem Gehäuse ein optischer Detektor 130 in Form einer Kamera beispielsweise auch an dem Träger 121 befestigt sein, die durch das optische Medium 108 hindurch ein Bild der Stoffoberfläche digital aufnimmt. Dieses Bild wird innerhalb einer Verarbeitungseinrichtung 107, die unmittelbar mit der Detektionseinrichtung und auch mit der Anregungssendeeinrichtung verbunden sein kann ebenso verarbeitet wie die Messinformationen von der Detektionseinrichtung 106. Die Verarbeitungseinrichtung kann auch Steuerungsaufgaben der Messung übernehmen. Sie kann auch wenigstens teilweise von den übrigen Teilen der Vorrichtung getrennt und entfernt sein und mittels einer Funkverbindung mit diesen kommunizieren.

Die Bilddaten von der Kamera 130 können somit innerhalb des Gehäuses oder über eine Funkverbindung auch außerhalb des Gehäuses weiterverarbeitet und mit einer Personenidentitäts-Datenbank verglichen werden, um Kalibrationsdaten der identifizierten Person abzurufen.

Derartige Kalibrationsdaten können auch entfernt (remote) in einer Datenbank, in einer Ausführungsform einer Cloud, abrufbar gespeichert sein. Auch die Messdaten der Detektionseinrichtung 106 können sowohl innerhalb als auch außerhalb des Gehäuses weiterverarbeitet werden.

Werden Daten außerhalb des Gehäuses verarbeitet, so sollten die Ergebnisdaten vorzugsweise zu der Vorrichtung innerhalb des Gehäuses zurückgefunkt werden, um dort angezeigt werden zu können.

In jedem Fall kann eine Anzeige an dem Gehäuse 122 vorgesehen sein, die vorteilhaft durch das optische Fenster, in einer Ausführungsform auch teilweise durch das optische Medium ablesbar ist. Die Anzeige kann auch durch das optische Fenster eine Leuchtanzeige auf eine Anzeigefläche projizieren und zu diesem Zweck eine Projektionsvorrichtung aufweisen. Durch die Anzeige kann in einer Ausführungsform ein Mess- oder Analyseergebnis, insbesondere eine Glucosekonzentration angezeigt werden. Die Ausgabe kann in einer Ausführungsform über einen Zeichen- oder Farbcode erfolgen. Es kann durch die Anzeige oder eine zu dieser parallele Signaleinrichtung in einer Ausführungsform ein Vorschlag für eine Insulindosis abhängig von weiteren Patientenparametern (z.B. Insulinkorrekturfaktor) oder eine automatische Signalübertragung an eine Dosiereinrichtung in Form einer Insulinpumpe erfolgen.

Die Verbindung der Vorrichtung von und zu einer externen Datenverarbeitungseinrichtung 131 kann durch alle gängigen Standards, wie Lichtleiter, Kabel, Funk (z. B. Bluetooth, WiFi), oder auch Ultraschall oder Infrarotsignale realisiert sein.

Die Figur 9 zeigt eine Modulationseinrichtung mit einer die Anregungssendeeinrichtung 100 moduliert ansteuernden Steuerung 132. Sowohl die Steuerung 132 als auch die Detektionseinrichtung 106 für den Mess-Lichtstrahl sind mit der Auswertungseinrichtung 107 verbunden.

Figur 10 zeigt eine Anregungs-Lichtquelle 100, vor der eine Spiegeleinrichtung, insbesondere eine durch ein MEMS (Mikro-elektromechanisches System) 135 angetriebene Spiegeleinrichtung mit einem oder mehreren Mikrospiegeln 133, 134, wie sie z. B. aus der optischen Bild-Projektortechnik bekannt sind, zur zeitweiligen Umlenkung des Anregungslichtstrahls in eine Umlenkrichtung 136 angeordnet ist.

Die Figur 11 zeigt eine Anregungs-Lichtquelle 100, vor der eine optische, bezüglich der Transmission mittels einer Steuereinrichtung 137 steuerbare Schicht 138 im Anregungslichtstrahl, in einer Ausführungsform mit LCD-Zellen angeordnet ist.

Zusammenfassend ist festzustellen, dass die vorliegend beschriebene Vorrichtung sowie das Messverfahren insbesondere bei der Anwendung zur Glucosemessung an Patienten diesen die schmerzhafte und störende invasive Messung erspart und damit auch eine regelmäßige und öftere Messung ermöglicht. Auch die Verarbeitbarkeit der Messergebnisse ist einfach und die laufenden Kosten werden minimiert. Die Messung kann ohne den Verbrauch von Analysesubstanzen durchgeführt werden.

Die Empfindlichkeit des Messverfahrens erreicht leicht 30 bis 300 mg pro dl. Abhängigkeiten der Messergebnisse von anderen Stoffen als Glucose, wie z.B. Alkohol oder Medikamenten im Blut sind minimal oder nicht vorhanden. Die Messeinrichtung ist ohne Lern- oder Trainingsaufwand bedienbar und es sind über längere Zeiten Messungen ohne Nachkalibrierung durchführbar.

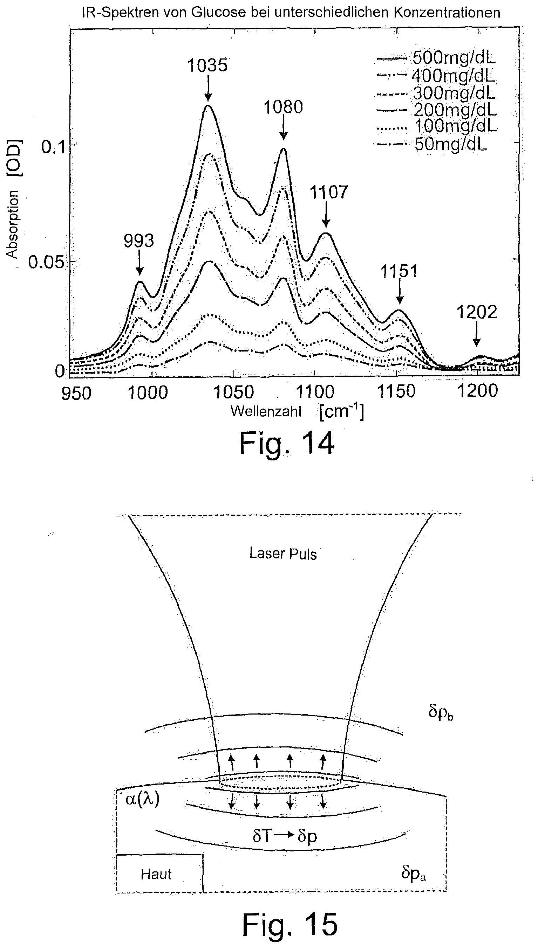

Folgende Faktoren können jeweils einzeln oder kombiniert bei der vorliegend beschriebenen Vorrichtung sowie dem Messverfahren für die Implementierung bei der Glukosemessung eine Rolle spielen:
Es wird ein Spektroskopieverfahren im mittleren Infrarot verwendet, in dem Glukose ein charakteristisches Absorptionsspektrum aufweist (vgl. Figur 14). Dabei kann beispielsweise der gesamte spektrale "Fingerprint" von Glukose abgedeckt werden. Es wird ein gepulster Quantenkaskadenlaser verwendet oder eine alternative Laserlichtquelle, die eine Anzahl von charakteristischen Wellenlängen abdecken kann.

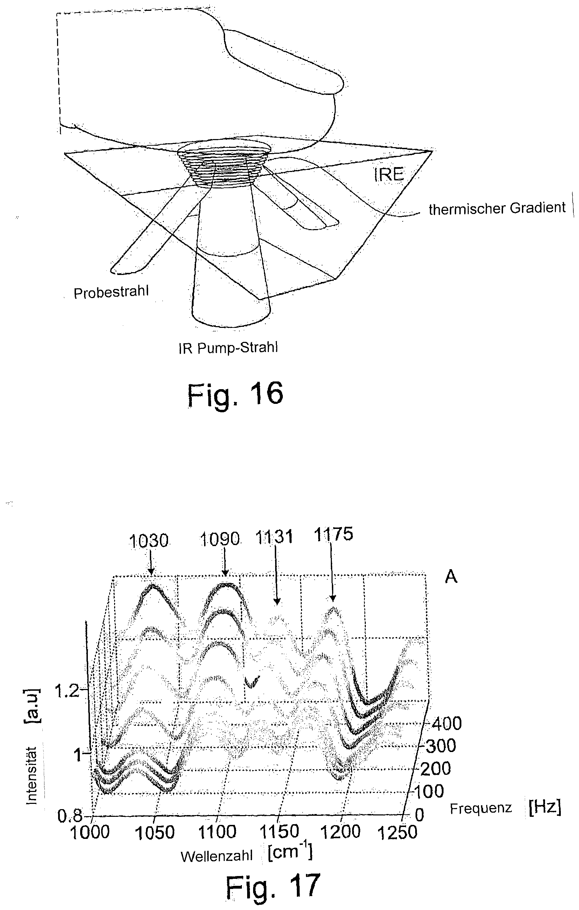

Es wird die beschriebene photothermische Nachweismethode verwendet (Figur 15, Figur 16).

Die Anregung und Detektion sind so eingestellt, dass die Absorption in der interstitiellen Flüssigkeit der Haut gemessen wird. Der Laserstrahl (Anregungsstrahl) dringt bis zu 100 Mikrometer in die Haut ein und erreicht Glukosemoleküle in der interstitiellen Flüssigkeit der Haut. Als Ergebnis der Lichtabsorption und des damit verbundenen Energieübertrags wird eine Wärmewelle erzeugt, die unter anderem zur Hautoberfläche wandert und dort mit dem oben erläuterten photothermischen Detektionselement nachgewiesen werden kann. Dieses bedient sich eines Detektions- oder Abfragelaserstrahls, dessen Ablenkung im optischen Medium von der Wärmewirkung der Wärmewelle im optischen Medium abhängt. Die Ablenkung wird als Indikator für die Absorption des Anregungsstrahls durch Glukose erfasst.

Der Anregungslaserstrahl durchdringt bei Anwendung auf der Haut das Stratum Corneum, das heißt die toten Zellen der Haut an der Oberfläche, die keinen aktuellen Glukosespiegel enthalten. Der Anregungsstrahl erreicht das Stratum Granulosum und Stratum Spinosum mit relevanten Glukoseanteilen. Der Glukosespiegel in diesen Schichten folgt unmittelbar dem Blutgluosespiegel; der Blutglukosespiegel der interstitiellen Flüssigkeit entspricht etwa 85 -90% des Blutglukosespiegels. Dies gilt vor allem für gut durchblutete Teile des Körpers, wie zB. Fingerbeere, Daumen, Ohrläppchen und Lippen. Dabei ist die Stoffzusammensetzung in der interstitiellen Flüssigkeit insgesamt einfacher als im Blut, so dass bei der Messung in der interstitiellen Flüssigkeit die Störfaktoren geringer sind.

Gewisse Störungen der Messungen durch Variationen in den äußersten Hautschichten können von Proband zu Proband auftreten und auch zeitabhängig sein.

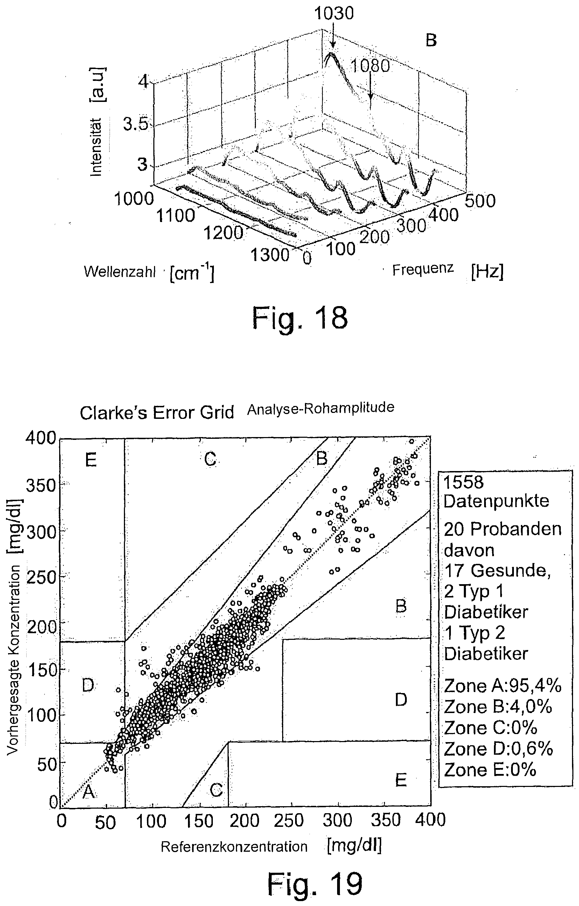

Um derartige Störungen auszublenden oder zu minimieren, werden Messwerte aus verschiedenen Hauttiefen (Entfernungsbereichen zur Hautoberfläche) erfasst. Hierzu werden die Infrarotspektren jeweils für mehrere Modulationsfrequenzen des Anregungsstrahls (Figur 17, Figur 18) ermittelt und dazu benutzt, durch eine Verknüpfung der Spektren Einflüsse oberer Hautschichten zu eliminieren.

Im Ergebnis kann die beschriebene Messmethode für die Messung des Glukosespiegels mit den bisher üblichen invasiven Methoden bezüglich der Genauigkeit und Zuverlässigkeit konkurrieren (vgl. Fig. 19).

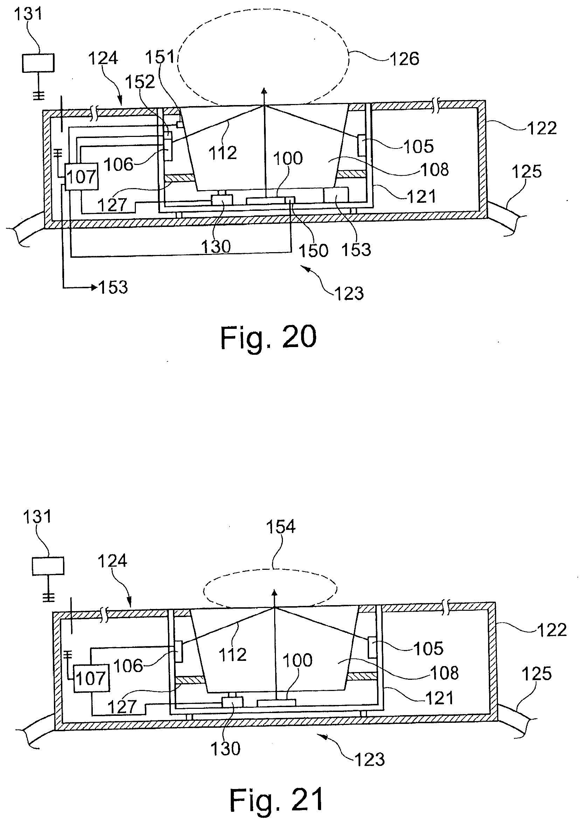

Die Figur 20 zeigt eine Messeinrichtung ähnlich der in Figur 8 dargestellten, wobei gleiche Bezugszeichen gleiche Funktionselemente bezeichnen. Dies gilt auch für die Figuren 21, 22 und 23.

In der Figur 20 sind verschiedene Temperatursensoren 150, 151 und 152 dargestellt, die einzeln oder in Gruppen oder alle in einer Messeinrichtung verwirklicht sein können, wobei der Sensor 150 die Temperatur an der Anregungssendeeinrichtung 100, also beispielsweise an einem Laser oder einem Laserarray, der Temperatursensor 151 die Temperatur an einem optischen Medium 108 und der Temperatursensor 152 die Temperatur an einer Detektionseinrichtung, beispielsweise an einem Fotosensor misst. Ein oder mehrere gemessene Temperaturwerte können entweder bei der Analyse des Stoffes berücksichtigt werden, indem Korrekturkoeffizienten für die Strahlungsintensität der Anregungssendeeinrichtung oder für die Nachweisempfindlichkeit des Fotosensors oder anderer Teile der Detektionseinrichtung bei der Auswertung berücksichtigt werden. Die Korrekturkoeffizienten können in Form einer Berechnungsformel oder in einer Tabelle elektronisch hinterlegt sein Es kann jedoch auch vorgesehen sein, mittels eines Heizelementes 153, das an der Anregungssendeeinrichtung 100, am Fotosensor oder beispielsweise am optischen Medium angeordnet sein kann, die Messeinrichtung ganz oder teilweise durch eine Temperaturregelung auf einer Temperatur zu halten, die höher als die Umgebungstemperatur und/oder die Körpertemperatur eines Patienten ist, so dass eine kurzfristige Temperaturänderung durch Aufheizen bei äußerem Einfluss nicht zu befürchten ist.

Figur 21 zeigt die Verwendung einer Messeinrichtung zur Messung einer Harnstoffkonzentration, wobei die Anregungslichtwellenlängen so gewählt sind, dass sie den Nachweis von Harnstoff ermöglichen, also z.B. die Absorptionswellenlängen des Harnstoffs. Die Messung kann an einem Körperteil, jedoch auch an einer Dialysatleitung 15 oder einer Blutleitung einer Dialyseeinrichtung durchgeführt werden. Die jeweilige Leitung sollte dazu an dem optischen Medium festgeklemmt werden.

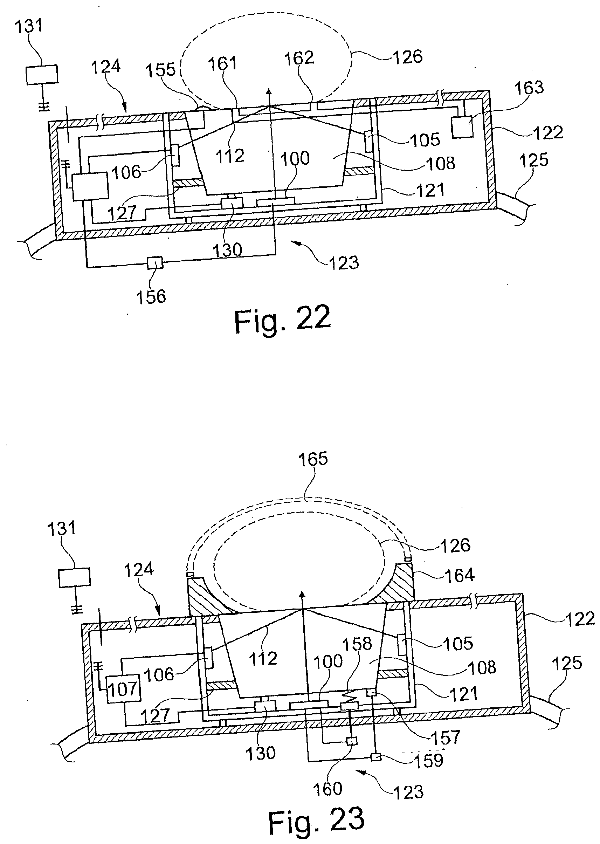

In Figur 22 ist eine Messeinrichtung mit einem Feuchtigkeitssensor 155 dargestellt, der dazu dient, zu erfassen, ob ein Gegenstand auf das optische medium 108 aufgelegt ist. Nur bei erhöhter Feuchtigkeit oder wenn der Feuchtigkeitswert in einem vorgegebenen Wertefenster nachgewiesen wird, wird die Anregungssendeeinrichtung zum Betrieb freigegeben bzw. eingeschaltet. Dadurch soll vermieden werden, dass das Anregungsstrahlung mehr als notwendig in die Umgebung gelangt, auch wenn diese grundsätzlich unbedenklich ist. Der durch den Sensor 155 betätigte Schalter ist mit 156 bezeichnet.

Gleichzeitig ist in der Figur 22 noch eine Einrichtung zum ansaugen von Luft 161, 162, 163 dargestellt, wobei mit 161 un d162 Öffnungen bezeichnet sind, die an der Frontseite des optischen Mediums enden und die mit einem Ansaugkanal verbunden sind. Dieser Ansaugkanal kann z. B kreisförmig um den bereich des optischen Mediums herum verlaufen, an den ein Körperteil zur Messung angepresst wird. Auch die Ansaugöffnungen können kreisförmig verteilt sein. Mittels des Ansaugkanals sind die Ansaugöffnungen mit einer Vakuum- Saugeinrichtung 163, zB. in Form einer Saugpumpe verbunden, bei deren Betrieb ein Gegenstand, der an das optische Medium gedrückt wird, dort festgehalten/fixiert wird.

Aus der Figur 23 geht eine Fixiereinrichtung hervor, die eine Mulde im Bereich des optischen Mediums schafft, in die zB. ein Finger eingelegt werden kann. Alternativ oder zusätzlich kann auch eine Klemmspange 165 vorgesehen sein, die den zu untersuchende Gegenstand einklemmt.

Die Mulde ist gemäß der Figur 23 durch einen umlaufenden erhöhten Rand 164 geschaffen, der aus einem elastischen Werkstoff oder einem Kissen, insbesondere einem aufpumpbaren Kissen bestehen kann. Es kann auch ein aufpumpbares Kissen vorgesehen sein, das nach dem Auflegen eines Gegenstandes, insbesondere eines Fingers, aufpumpbar ist und im aufgepumpten Zustand den Gegenstand/Finger fest einklemmt. Die Messeinrichtung kann dann mit einer Einrichtung zur Steuerung des Drucks in dem Kissen verbunden sein.

In der Figur 23 ist auch ein Drucksensor 158 gezeigt, der beispielsweise an der Rückseite des Optischen Mediums angeordnet sein kann, welche der Frontseite, an die ein Gegenstand zur Messung angedrückt wird, gegenüberliegt. Der Sensor 158 kann z.B. eine Feder mit einem Näherungsschalter/Wegsensor und/oder einen Piezosensor aufweisen, der in Abhängigkeit vom wirkenden Druck ein Signal erzeugt und/oder einen Schalter 160 zur Ein- und /oder Ausschaltung der Anregungssendeeinrichtung betätigt.

Parallel dazu ist in der Figur 23 auch ein Helligkeitssensor 157 dargestellt, der also Fotosensor ausgebildet sein kann und der detektiert, ob die Frontseite des optischen Mediums von einem Gegenstand abgedeckt ist. In diesem Fall wird die Anregungssendeeinrichtung mittels des Schalters 159 eingeschaltet oder freigeschaltet, ansonsten kann sie blockiert werden.

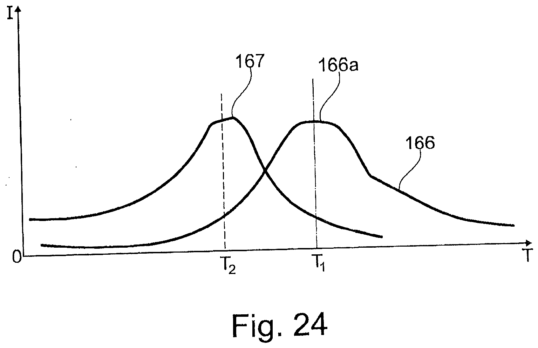

Die Figur 24 zeigt zwei mit dem erfindungsgemäßen Verfahren sowie einer erfindungsgemäßen Vorrichtung aufgenommene oder ermittelte Tiefenprofile der Dichte von zwei Substanzen, die sich in dem zu untersuchenden Stoff/Körperteil befinden. Die beiden Substanzen werden durch Anregungsstrahlen mit unterschiedlichen Wellenlängen/Gruppen von Wellenlängen nachgewiesen, wobei als Wellenlängen beispielsweise ein oder mehrere Wellenlängen gewählt sein können, bei denen oder in deren Nähe sich Absorptionsmaxima der nachzuweisenden Substanzen befinden. Mit der erfindungsgemäßen Messmethode lassen sich Dichteverteilungen 166, 167 I(T)der beiden Stoffe in Abhängigkeit von der Tiefe T, senkrecht zur Stoffoberfläche gemessen, ermitteln. Die Substanzen werden typischerweise gleiche oder unterschiedliche Dichteverteilungen haben. Ist die Dichteverteilung 166 einer Referenzsubstanz bekannt, oder auch nur, in welcher Tiefe T1 sich das Maximum 166a oder ein anderer charakteristischer Punkt der Verteilung 166 unter der Oberfläche befindet, so kann die Dichteverteilung 167 der anderen Substanz oder die Lage T2 des Dichtemaximums ihrer Verteilung an dieser Referenzgröße geeicht werden.

Die vorliegende Schutzrechtsanmeldung bezieht sich (wie einleitend bereits erwähnt) neben den oben beschriebenen Anspruchsgegenständen und Ausführungsbeispielen auch auf die folgenden Aspekte. Diese Aspekte können einzeln oder in Gruppen jeweils mit Merkmalen der Ansprüche kombiniert werden. Die Aspekte stellen darüber hinaus auch jeweils für sich genommen oder miteinander oder mit Anspruchsgegenständen kombiniert eigenständige Erfindungen dar. Die Anmelderin behält sich vor, diese Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen:
1) Verfahren zum Analysieren eine Stoffes in einem Körper, umfassend:
   - Aussenden eines Anregungs-Lichtstrahls mit einer oder mehreren spezifischen Anregungswellenlängen durch einen ersten Bereich der Oberfläche des Körpers,
   - Intensitätsmodulation des Anregungs-Lichtstrahls mit einer oder mehreren Frequenzen, insbesondere nacheinander, durch ein von einem mechanischen Chopper verschiedenes Bauteil, insbesondere durch eine elektronische Ansteuerung der Anregungslichtquelle, eine Verstelleinrichtung für einen Resonator eines als Anregungslichtquelle dienenden Anregungslasers oder eine bewegliche Spiegeleinrichtung, eine steuerbare Beugungseinrichtung, eine Verschluss- oder Spiegeleinrichtung, die an einem Motor, wie einem Steppermotor, oder mit einem MEMS gekoppelt ist oder eine bezüglich der Transmission steuerbare Schicht im Strahlengang,
   - mittels eines außerhalb des Körpers angeordneten Detektors die zeitaufgelöste Detektion eines Reaktionssignals, das auf die Wirkung der wellenlängenabhängigen Absorption des Anregungs-Lichtstrahls im Körper zurückgeht.
   Die Modulation kann in einer Ausführungsform durch Interferenz oder Beeinflussung der Phase oder Polarisation der Strahlung der Anregungssendeeinrichtung erfolgen, insbesondere, wenn diese eine Laserlichteinrichtung umfasst.
2) Verfahren nach Aspekt 1, dadurch gekennzeichnet, dass der Anregungslichtstrahl durch mehrere Emitter oder Multiemitter, insbesondere in Form eines Laser-Arrays, erzeugt wird, die Licht mit unterschiedlichen Wellenlängen gleichzeitig oder nacheinander oder in beliebigen Pulsmustern aussenden.
3) Verfahren nach Aspekt 1 oder 2, dadurch gekennzeichnet, dass an dem ersten Bereich der Oberfläche des Körpers ein akustisches Reaktionssignal durch einen akustischen Sensor erfasst wird.
4) Verfahren nach einem der Aspekte 1 bis 3, dadurch gekennzeichnet, dass an dem ersten Bereich der Oberfläche des Körpers ein Reaktionssignal durch einen Infrarotstrahlungssensor, insbesondere ein Thermoelement, ein Bolometer oder ein Halbleiterdetektor, zum Beispiel ein Quantum-Cascade-Detektor, erfasst wird.
5) Verfahren nach einem der Aspekte 1 bis 4, umfassend die Schritte:
   - Herstellen des Kontakts eines optischen Mediums mit einer Stoffoberfläche, so dass zumindest ein Bereich der Oberfläche des optischen Mediums in Kontakt mit dem ersten Bereich der Oberfläche des Körpers steht;
   - Aussenden eines Anregungs-Lichtstrahls mit einer Anregungswellenlänge in ein in dem Stoff unterhalb des ersten Bereiches der Oberfläche liegendes Volumen insbesondere durch den Bereich der Oberfläche des optischen Mediums hindurch, der in Kontakt mit dem ersten Bereich der Stoffoberfläche steht,
   - Messen der Temperatur in dem ersten Bereich der Oberfläche des optischen Mediums durch ein optisches pyrometrisches Verfahren,
   - Analysieren des Stoffes anhand der detektierten Temperaturerhöhung in Abhängigkeit von der Wellenlänge des Anregungs-Lichtstrahls.
6) Verfahren nach Aspekt 5, gekennzeichnet durch
   das Aussenden eines Mess-Lichtstrahls durch das optische Medium auf den Bereich der Oberfläche, des optischen Mediums, der in direktem Kontakt mit der Stoffoberfläche steht, derart, dass der Mess-Lichtstrahl und der Anregungslichtstrahl auf der Grenzfläche des optischen Mediums und der Stoffoberfläche, an der der Mess-Lichtstrahl reflektiert wird, einander unmittelbar benachbart sind oder überlappen;
   Direktes oder indirektes Detektieren einer Ablenkung des reflektierten Mess-Lichtstrahls in Abhängigkeit von der Wellenlänge des Anregungs-Lichtstrahls und
   Analysieren des Stoffes anhand der detektierten Ablenkung des Mess-Lichtstrahls in Abhängigkeit von der Wellenlänge des Anregungs-Lichtstrahls.
7) Verfahren nach einem der Aspekte 5 oder 6, dadurch gekennzeichnet, dass der Mess-Lichtstrahl durch dieselbe Lichtquelle erzeugt wird, die den Anregungs-Lichtstrahl erzeugt.
8) Verfahren nach einem der Aspekte 5, 6 oder 7, dadurch gekennzeichnet, dass der Mess-Lichtstrahl nach der Ablenkung und vor der Detektion innerhalb des optischen Mediums, außerhalb des optischen Mediums oder teilweise innerhalb und teilweise außerhalb des optischen Mediums ein- oder mehrmals reflektiert wird.
9) Verfahren nach Aspekt 1 oder einem der anderen vorangehenden bzw. folgenden, dadurch gekennzeichnet, dass der Mess-Lichtstrahl ein intensitätsmodulierter, insbesondere gepulster Anregungs-Lichtstrahl insbesondere im Infrarot-Spektralbereich ist, wobei insbesondere die Modulationsrate zwischen 1 Hz und 10 kHz, vorzugsweise zwischen 10Hz und 3000Hz liegt.
10) Verfahren nach Aspekt 1 oder einem der anderen vorangehenden bzw. folgenden, dadurch gekennzeichnet, dass das Licht des/der Anregungs- Lichtstrahlen durch eine integrierte Anordnung mit mehreren Einzellasern, insbesondere einem Laserarray, gleichzeitig oder nacheinander oder teilweise gleichzeitig und teilweise nacheinander erzeugt wird.
11) Verfahren nach Aspekt 1 oder einem der anderen vorangehenden bzw. folgenden, dadurch gekennzeichnet, dass aus den bei verschiedenen Modulationsfrequenzen des Anregungs-Lichtstrahls gewonnenen Reaktionssignalen eine Intensitätsverteilung der Reaktionssignale in Abhängigkeit von der Tiefe unter der Oberfläche ermittelt wird, in der die Reaktionssignale entstehen.
12) Verfahren nach Aspekt 1 oder einem der anderen vorangehenden bzw. folgenden, dadurch gekennzeichnet, dass aus der Phasenlage der Reaktionssignale im Verhältnis zu einem modulierten Anregungs- Lichtstrahl bei einer oder verschiedenen Modulationsfrequenzen des Anregungs-Lichtstrahls eine Intensitätsverteilung der Reaktionssignale in Abhängigkeit von der Tiefe unter der Oberfläche ermittelt wird, in der die Reaktionssignale entstehen.
13) Verfahren nach Aspekt 11 oder 12, dadurch gekennzeichnet, dass zur Ermittlung der Intensitätsverteilung der Reaktionssignale in Abhängigkeit von der Tiefe unter der Oberfläche die Messergebnisse zu verschiedenen Modulationsfrequenzen gewichtet und miteinander verknüpft werden.
14) Verfahren nach Aspekt 11, 12 oder 13, dadurch gekennzeichnet, dass aus der gewonnenen Intensitätsverteilung über die Tiefe unter der Oberfläche des Körpers eine Stoffdichte eines den Anregungs- Lichtstrahl in spezifischen Wellenlängenbereichen absorbierenden Stoffes in einer bestimmten Tiefe oder einem Tiefenbereich ermittelt wird.
15) Verfahren nach Aspekt 1 oder einem der anderen vorangehenden bzw. folgenden, dadurch gekennzeichnet, dass im unmittelbar vor oder nach oder während der Detektion des Reaktionssignals/der Reaktionssignale wenigstens eine biometrische Messung an dem Körper in dem ersten Bereich der Oberfläche oder unmittelbar diesem benachbart, insbesondere eine Vermessung eines Fingerabdrucks durchgeführt und der Körper, insbesondere eine Person identifiziert wird und dass insbesondere der Detektion der Reaktionssignale Referenzwerte (Kalibrierungswerte) zugeordnet werden.
16) Vorrichtung zum Analysieren eines Stoffes,
   mit einer Einrichtung zum Aussenden eines oder mehrerer Anregungs-Lichtstrahlen mit jeweils einer Anregungswellenlänge in ein in dem Stoff unterhalb eines ersten Bereiches seiner Oberfläche liegendes Volumen, mit einer Einrichtung zum Modulieren eines Anregungslichtstrahls, die durch eine Modulationseinrichtung der Strahlungsquelle, insbesondere ihrer Ansteuerung, eine Interferenzeinrichtung, eine Phasen- oder Polarisationsmoduliereinrichtung und/oder wenigstens einen, im Strahlengang angeordneten gesteuerten Spiegel, und/oder eine bzgl. ihrer Transparenz steuerbare, im Strahlengang angeordnete Schicht gebildet ist, sowie mit einer Detektionseinrichtung zur
   Detektion eines zeitabhängigen Reaktionssignals in Abhängigkeit von der Wellenlänge des Anregungslichts und der Intensitäts- Modulation des Anregungslichts und mit einer Einrichtung zum Analysieren des Stoffes anhand der detektierten Reaktionssignale.
17) Vorrichtung nach Aspekt 16 mit einer Einrichtung zur Ermittlung von Reaktionssignalen gesondert nach verschiedenen Intensitäts-Modulationsfrequenzen und/oder mit einer Einrichtung zur Ermittlung von Reaktionssignalen in Abhängigkeit von der Phasenlage des jeweiligen Reaktionssignals relativ zur Phase der Modulation des Anregungs- Lichtstrahls, insbesondere in Abhängigkeit von der Modulationsfrequenz des Anregungs- Lichtstrahls.
18) Vorrichtung zum Analysieren eine Stoffes nach Aspekt 16 oder 17, mit einem optischen Medium zum Herstellen des Kontakts der Oberfläche des optischen Mediums mit einem ersten Bereich der Stoffoberfläche, sowie mit
   einer Einrichtung zum Aussenden eines Anregungs-Lichtstrahls mit einer oder mehreren Anregungswellenlänge in ein in dem Stoff unterhalb der des ersten Bereiches der Oberfläche liegendes Volumen insbesondere durch den Bereich der Oberfläche des optischen Mediums hindurch, der in Kontakt mit der Stoffoberfläche steht, sowie mit einer Einrichtung zum
   Messen der Temperatur in dem Bereich der Oberfläche des optischen Mediums, der mit dem ersten Bereich der Stoffoberfläche im Kontakt steht durch ein optisches Verfahren, und mit einer Einrichtung zum Analysieren des Stoffes anhand der detektierten Temperaturänderungen in Abhängigkeit von der Wellenlänge des Anregungs-Lichtstrahls und der Intensitätsmodulation des Anregungs- Lichtstrahls.
19) Vorrichtung nach Aspekt 18, dadurch gekennzeichnet, dass die Anregungs-Lichtquelle unmittelbar mit dem optischen Medium mechanisch fest verbunden ist.
20) Vorrichtung nach Aspekt 18, dadurch gekennzeichnet, dass eine Einrichtung zum Aussenden eines Mess-Lichtstrahls in den Bereich des optischen Mediums vorgesehen ist, der mit dem ersten Bereich der Stoffoberfläche im Kontakt steht und dass diese Einrichtung/oder die Detektionseinrichtung zur Detektion des Mess- Lichtstrahls unmittelbar mit dem optischen Medium mechanisch fest verbunden und/oder mittels eines Lichtwellenleiters an dieses angekoppelt ist.
21) Vorrichtung nach Aspekt 18, 19 oder 20, dadurch gekennzeichnet, dass das optische Medium unmittelbar eine Abbildungsoptik trägt und/oder dass in das optische Medium eine Abbildungs-Optik integriert ist.
22) Vorrichtung nach Aspekt 18 oder einem der anderen vorangehenden bzw. folgenden, dadurch gekennzeichnet, dass die Oberfläche des optischen Mediums mehrere gegeneinander geneigte Teilflächen aufweist, an denen der Mess-Lichtstrahl mehrfach reflektiert wird.
23) Verfahren nach Aspekt 18 oder einem der anderen vorangehenden bzw. folgenden, dadurch gekennzeichnet, dass in oder an dem optischen Medium ein oder mehrere Spiegelflächen zur Reflektion des Mess- Lichtstrahls vorgesehen sind.
24) Vorrichtung nach Aspekt 16 oder 17, dadurch gekennzeichnet, dass die Detektionseinrichtung zur Detektion eines zeitabhängigen Reaktionssignals einen akustischen Detektor zur Erfassung akustischer Wellen an der Stoffoberfläche, insbesondere mit einem Resonator, weiter insbesondere mit einem Helmholtzresonator. Als Detektor der akustischen Quelle dient eine Quartzgabel mit vorzugsweise der gleichen Resonanzfrequenz wie der Resonator. Der Resonator kann offen oder geschlossen sein. Die Quartzgabel ist vorzugsweise im oder am Hals des Resonantors (off-beam) oder inner-/außerhalb des Resonators (in-beam) angeordnet.
25) Vorrichtung nach Aspekt 16, 17 oder 18, dadurch gekennzeichnet, dass die Detektionseinrichtung zur Detektion eines zeitabhängigen Reaktionssignals einen Wärmestrahlungs- Detektor zur Erfassung der Wärmestrahlung an der Stoffoberfläche, insbesondere einen Infrarotdetektor, weiter insbesondere ein Thermoelement, ein Bolometer, oder einen Halbleiterdetektor aufweist.
26) Vorrichtung nach einem der Aspekte 16 bis 25, dadurch gekennzeichnet, dass die Anregungs-Lichtquelle und die Detektionseinrichtung unmittelbar aneinander oder an einem gemeinsamen Träger befestigt sind, der insbesondere durch ein Gehäuse oder Gehäuseteil der Vorrichtung gebildet ist.
27) Vorrichtung nach einem der Aspekte 16 bis 26, dadurch gekennzeichnet, dass die Vorrichtung ein tragbares Gehäuse aufweist, das am Körper einer Person befestigbar ist, wobei die Einrichtung zum Aussenden eines oder mehrerer Anregungs-Lichtstrahlen und die Detektionseinrichtung zur Detektion eines zeitabhängigen Reaktionssignals derart angeordnet und eingerichtet ist, dass der zu analysierende Stoff auf der dem Körper abgewandten Seite des Gehäuses vermessen wird.
28) Vorrichtung nach einem der Aspekte 16 bis 26, dadurch gekennzeichnet, dass die Vorrichtung ein tragbares Gehäuse aufweist, das am Körper einer Person befestigbar ist und dass das Gehäuse der Vorrichtung ein für den Anregungs- Lichtstrahl durchlässiges Fenster auf seiner bei der vorgesehenen Trageposition vom Körper abgewandten Seite aufweist.
29. Vorrichtung zum Analysieren eines Stoffes mit einer Anregungssendeeinrichtung zum Erzeugen mindestens eines elektromagnetischen Anregungsstrahls, insbesondere Anregungs-Lichtstrahls, mit zumindest einer Anregungswellenlänge, einer Detektionseinrichtung zur Detektion eines Reaktionssignals und einer Einrichtung zum Analysieren des Stoffes anhand des detektierten Reaktionssignals.
30. Vorrichtung nach einem der vorhergehenden Aspekte 16 bis 29, dadurch gekennzeichnet, dass die Detektionseinrichtung zur Messung der Deformation eines Kristalls eingerichtet ist.
   Die Deformation kann analog zur photothermischen Bouncing Method' durch die Wahl von steileren (größeren) Einfallswinkeln des Messstrahls zur Probeoberfläche effektiver gemessen werden und der Einfluss der Mirage-Effekt bedingten Ablenkung des Messstrahls minimiert werden.
   Literatur:
   M. Bertolotti, G.L. Liakhou, R. Li Voti, S. Paolino, and C. Sibilia. Analysis of the photothermal deflection technique win the surface refection theme: Theory and Experiment. Journal of Applied Physics 83, 966 (1998)
31. Vorrichtung nach einem der vorhergehenden Aspekte 16 bis 30, dadurch gekennzeichnet, dass die Anregungssendeeinrichtung einen Abfragelaser oder eine LED, beispielsweise eine NIR(near-infrared)-LED, enthält.
32. Vorrichtung nach einem der vorhergehenden Aspekte 16 bis 31, dadurch gekennzeichnet, dass die Anregungssendeeinrichtung einen Probe-Laser aufweist, der einen kleineren oder größeren Durchmesser als ein zusätzlicher Pump-Laser hat.
33. Vorrichtung nach einem der vorhergehenden Aspekte 16 bis 32, dadurch gekennzeichnet, dass zur Erzielung eines günstigeren Signal-/Rausch-Verhältnis eine spezielle Beschichtung, insbesondere des optischen Elementes, z. B. IRE, vorgesehen ist, so dass Wärme besser abgeführt wird (z. B. "Wärmeleitpaste").
   Das optische Element kann an der Kontaktfläche derartig beschichtet sein, dass eine verbesserte Ableitung des thermischen Signals in das optische Medium erfolgen kann. Zudem kann die Beschichtung des weiteren auch als Kratzschutz dienen, und auch durch geschickte Materialwahl eine reflektierende Oberfläche für den Messstrahl darstellen. Hierbei muss die Transparenz für das Anregungslicht zwingend gegeben bleiben.
34. Vorrichtung nach einem der vorhergehenden Aspekte 16 bis 33, dadurch gekennzeichnet, dass die Vorrichtung eine Einrichtung für
   i. Pulstrains / Doppelmodulation
   ii. Schwingspiegel
   iii. MEMS Interferometer
   aufweist.
35. Vorrichtung nach einem der vorhergehenden Aspekte 16 bis 34, dadurch gekennzeichnet, dass die Vorrichtung für eine Person am Körper in einer Ausführungsform durch eine mit dem Gehäuse verbundene Halteeinrichtung wie einen Riemen, ein Band oder eine Kette oder eine Spange, dauerhaft tragbar ausgeführt ist und/ oder die Detektionseinrichtung eine Detektionsfläche aufweist, die auch als Anzeigefläche für Informationen wie Messwerte, Uhrzeiten und/ oder Textinformationen nutzbar ist.
36. Vorrichtung nach dem vorhergehenden Aspekt 35, dadurch gekennzeichnet, dass die Vorrichtung eine Abziehfolie im Bereich der Detektionsfläche, vorzugsweise neben der Detektionsfläche, zur Vorbehandlung der Stoffoberfläche und Sicherstellung einer sauberen Oberfläche und/ oder in einer Ausführungsform im Fall der Glucosemessung konkret zur Hautreinigung aufweist.
37. Vorrichtung nach einem der vorhergehenden Aspekte 16 bis 36, dadurch gekennzeichnet, dass die Detektionseinrichtung eingerichtet ist zum Lesen und Erkennen von Fingerabdrücken zum Abruf bestimmter Werte/ Kalibrierungen einer Person und/ oder zur Erkennung der Lage eines Fingers, vorzugsweise zum Erkennen und Bestimmen einer ungewollten Bewegung während der Messung, aufweist.
38. Vorrichtung nach einem der vorhergehenden Aspekte 16 bis 37, dadurch gekennzeichnet, dass die Detektionseinrichtung eine Ergebnisanzeige aufweist, die vorzugsweise mit Farbkodierung, als analoge Anzeige, in einer Ausführungsform inkl. Fehleranzeige (beispielhaft: "100mg/dl plus/minus 5mg/dl"), akustisch und/ oder mit Ergebnisanzeige von Messwerten in größeren Schritten als die Messgenauigkeit der Vorrichtung es erlaubt ausgeführt ist. Hierdurch werden z.B. kleine Schwankungen, die einen Benutzer verunsichern könnten, nicht mitgeteilt.
39. Vorrichtung nach einem der vorhergehenden Aspekte 16 bis 38, dadurch gekennzeichnet, dass die Vorrichtung
   zum Austausch gemessener Daten sowie zum Abruf von Kalibrier- oder anderen Daten von anderen Geräten oder Cloud-Systemen Datenschnittstellen aufweist,
   wobei die Vorrichtung vorzugsweise so eingerichtet ist, dass die Datenübertragung verschlüsselt, insbesondere durch Fingerabdruck oder andere biometrische Daten des Bedieners verschlüsselt, erfolgen kann.
40. Vorrichtung nach einem der vorhergehenden Aspekte 16 bis 39, dadurch gekennzeichnet, dass die Vorrichtung so eingerichtet ist, dass ein Vorschlag für eine der Person zu gebende Insulindosis durch die Vorrichtung im Zusammenspiel mit anderen Daten ermittelbar ist (z.B. Insulinkorrekturfaktor) und/oder Gewicht, Körperfett mitmessbar und/oder manuell angebbaren oder von anderen Geräten an die Vorrichtung übermittelbar ist.
41. Vorrichtung nach einem der vorhergehenden Aspekte 16 bis 40 dadurch gekennzeichnet, dass die Vorrichtung zur Erhöhung der Messgenauigkeit eingerichtet ist zur Ermittlung weiterer Parameter, in einer Ausführungsform mittels Sensoren zum Ermitteln der Hauttemperatur, Diffusivität, Leitfähigkeit/ Feuchtigkeit der Haut, zur Messung der Polarisation des Lichtes (Ausschluss von Wasser/Schweiß auf der Fingeroberfläche) oder dergleichen.
   Wasser und Schweiß auf der Hautoberfläche eines Menschen, die die Glucosemessung beeinflussen können, können durch eine Test- Anregung mit einer Anregungsstrahlung durch die Anregungssendeinrichtung mit den wasserspezifischen Banden bei 1640 cm-1 (6.1 µm) und 690 cm⁻¹ (15 µm) detektiert werden. Sollte die Absorption einen bestimmten Wert überschreiten ist der Messort/die Stoffoberfläche/Hautoberfläche zu nass für eine zuverlässige Messung. Alternativ kann die Leitfähigkeit des Stoffes in der Nähe oder direkt an der Messstelle gemessen werden, um die Feuchtigkeit zu bestimmen. Es kann dann eine Fehlermeldung sowie eine Anweisung zum Trocknen ausgegeben werden.
42. Vorrichtung nach einem der vorhergehenden Aspekte 16 bis 41 dadurch gekennzeichnet, dass die Vorrichtung über eine Abdeckung im Strahlengang des Pump- und/oder Messstrahl-Lasers verfügt. Hierdurch kann für die obligatorische Augensicherheit von Lebewesen gesorgt werden.
43. Vorrichtung nach einem der vorhergehenden Aspekte 16 bis 42 dadurch gekennzeichnet, dass die Vorrichtung über eine wechselbare Detektionsfläche verfügt.
44. Vorrichtung nach einem der vorhergehenden Aspekte 16 bis 43 dadurch gekennzeichnet, dass die Vorrichtung als optisches Medium über einen stellenweise geriffelten oder aufgerauhten Kristall verfügt, der der Probe (z.B. dem Finger) einen bessere Justierung ermöglicht. Die Messstelle, auf die die zu analysierende Stoffoberfläche aufgelegt wird, ist vorzugsweise ohne Riffelung und glatt ausgebildet.
45. Vorrichtung nach einem der vorhergehenden Aspekte 16 bis 44 dadurch gekennzeichnet, dass für den Messstrahl eine zylindrische TEMpl TEM00 Mode oder anstelle der zylindrischen TEMpl TEM00 Mode andere Moden TEM01 (Dougnut) TEM02 oder TEM03 verwendet werden. Besonders die letzteren haben den Vorteil das ihre Intensität auf das Sensitivitäts-Profil der Quadrantendiode abgestimmt werden können, die den Detektor für den abgelenkten Messstrahl darstellt (siehe Figuren). Desweiteren können rechteckige Moden TEMmn benutzt werden wie TEM30 oder TEM03 oder höher. Damit können Abtast/Messstrahlen genutzt werden die in horizontaler oder vertikaler Richtung Störungsunempfindlicher sind.
46. Vorrichtung nach einem der vorhergehenden Aspekte 16 bis 45 dadurch gekennzeichnet, dass die Vorrichtung nicht nur an einem Punkt sondern in einem Raster misst. Dies kann entweder durch eine Verschiebung des Pump- oder des Probe-Lasers oder der Detektionseinheit geschehen. Anstatt einer Verschiebung ist auch ein oder mehrere Arrays von Pump- oder Probe-Lasern denkbar.
   Zusätzlich sollen noch folgende Aspekte der Erfindung genannt werden, die auch für sich eine oder mehrere Erfindungen darstellen können:
47. Vorrichtung, die eingerichtet ist zum Analysieren eines Stoffes 101, mit einer Anregungssendeeinrichtung 100, mit der mindestens ein elektromagnetischer Anregungsstrahl (SA) mit zumindest einer Anregungswellenlänge durch den aufeinander folgenden Betrieb einer oder mehrerer oder durch den wenigstens teilweise gleichzeitigen Betrieb mehrerer Laseremitter einer Laserlichtquelle erzeugt wird, und
   mit einer Detektionseinrichtung 106, mit der ein Reaktionssignal (SR) detektiert wird und einer Analyseeinrichtung, mit der anhand des detektierten Reaktionssignals (SR) der Stoff analysiert wird.
48. Vorrichtung nach Aspekt 47, die derart eingerichtet ist, dass mittels einer Temperaturregeleinrichtung die Temperatur des /der Laseremitter der Anregungssendeeinrichtung 100 und/oder die Temperatur der Detektionseinrichtung 106, insbesondere eines optischen Mediums (108) und/oder einer Detektionsstrahlungsquelle (105) und/oder eines optischen Sensors während der Analyse konstant gehalten, insbesondere auf einer vorgegebenen Temperatur konstant gehalten, weiter insbesondere auf einer vorgegebenen Temperatur oberhalb der Körpertemperatur eines Menschen, weiter vorzugsweise oberhalb von 37 Grad Celsius, weiter vorzugsweise oberhalb von 38 oder 40 Grad Celsius gehalten wird.
49. Vorrichtung nach Aspekt 47 oder 48, die derart eingerichtet ist, dass während der Analyse die Temperatur des /der Laseremitter der Anregungssendeeinrichtung (100) und /oder die Temperatur der Detektionseinrichtung (106), insbesondere eines optischen Mediums (108) und/oder einer Detektionsstrahlungsquelle (105) und /oder eines optischen Sensors mittels eines Temperatursensors erfasst und bei der Auswertung der Analyse durch Verknüpfung der Messergebnisse mit Temperaturkorrekturwerten berücksichtigt wird.
50. Vorrichtung nach Aspekt 47, 48 oder 49 die derart eingerichtet ist, dass der Stoff, insbesondere ein Körperteil, insbesondere ein Finger, gegen ein optisches Medium (108) pressbar ist, das Teil der Detektionseinrichtung ist, dass der auf das Medium durch das angedrückte Körperteil ausgeübte Druck mittels eines Drucksensors erfassbar ist und dass in Abhängigkeit von dem erfassten Druck auf das Medium (108) die Anregungssendeeinrichtung (100) mittels eines Schalters eingeschaltet und/oder diese in Abhängigkeit von einer Verminderung des Drucks, insbesondere unter eine bestimmte Schwelle, ausgeschaltet wird.
51. Vorrichtung nach Aspekt 47, 48, 49 oder 50, die derart eingerichtet ist, dass der Stoff, insbesondere ein Körperteil, insbesondere ein Finger, gegen ein optisches Medium (108) pressbar ist, das Teil der Detektionseinrichtung ist, dass eine Verdunkelung des Mediums im Bereich des angedrückten Körperteils mittels eines Helligkeitssensors erfasst wird und dass durch Verdunkelung des Mediums die Anregungssendeeinrichtung (100) mittels eines Schalters eingeschaltet und/oder diese durch eine Vergrößerung der Helligkeit im optischen Medium insbesondere über eine bestimmte Schwelle, ausgeschaltet wird.
52. Vorrichtung nach Aspekt 47, 48. 49, 50 oder 51, die derart eingerichtet ist, dass der Stoff, insbesondere ein Körperteil, insbesondere ein Finger, gegen ein optisches Medium (108) pressbar ist, das Teil der Detektionseinrichtung ist, dass ein Feuchtigkeitsgrad des Mediums (108) im Bereich des angedrückten Körperteils mittels eines Feuchtigkeitssensors erfasst wird und dass durch Erreichen eines vorgegebenen Feuchtigkeitsgrads an dem Medium die Anregungssendeeinrichtung (100) mittels eines Schalters eingeschaltet und/oder diese durch eine Verminderung des Feuchtigkeitsgrades, insbesondere unter eine bestimmte Schwelle, oder auch durch das Ansteigen des Feuchtigkeitswertes über eine bestimmte Schwelle ausgeschaltet wird.
53. Vorrichtung nach Aspekt 47 oder einem der folgenden, die derart eingerichtet ist, dass während der Durchführung des Verfahrens ein an das optische Medium (108) pressbares Körperteil an dem Medium (108) fixierbar, insbesondere durch Einklemmen des Körperteils mit einer Klemmeinrichtung an dem Medium, Verkleben des Körperteils mit dem Medium mittels einer Klebeschicht, Adhäsion des Körperteils an dem Medium oder durch Vakuum-Ansaugen des Köperteils an das Medium mittels einer Ansaugeinrichtung fixiert wird.
54. Vorrichtung nach Aspekt 47, die derart eingerichtet ist, dass der Anregungsstrahl(SA) mit mindestens zwei Anregungswellenlängen oder Gruppen von Anregungswellenlängen ausgesendet wird, wobei eine erste Anregungswellenlänge oder Gruppe von Anregungswellenlängen den Nachweis einer in dem Stoff nachzuweisenden Substanz ermöglicht, während wenigstens eine weitere Anregungswellenlänge oder Gruppe von Anregungswellenlängen den Nachweis einer von einer nachzuweisenden Substanz verschiedenen Referenzsubstanz ermöglicht und dass für die wenigstens zwei verschiedenen Substanzen Profile bezüglich ihrer Dichteverteilung in der Tiefe des Stoffes ermittelt und miteinander verknüpft werden, und wobei insbesondere ein Tiefenprofil oder wenigstens ein oder mehrere Parameter eines Tiefenprofils der Referenzsubstanz in dem Stoff bekannt ist.
55. Vorrichtung nach Aspekt 47 oder einem der folgenden, die derart eingerichtet ist, dass der Anregungsstrahl (SA) moduliert, vorzugsweise nacheinander mit verschiedenen Modulationsfrequenzen durch eine Modulationseinrichtung moduliert wird, wobei Reaktionssignale für verschiedene Modulationsfrequenzen mittels einer Detektionseinrichtung detektiert werden oder ein Zeitverhalten des Reaktionssignals bei einer Änderung der Intensität des Anregungsstrahls, insbesondere bei dessen periodischem Anschalten und/oder Abschalten, analysiert wird.
56. Vorrichtung nach Aspekt 55, die derart eingerichtet ist, dass die Modulation des Anregungsstrahls (SA) durch eine Ansteuereinrichtung einer den Anregungslichtstrahl erzeugenden Lasereinrichtung (100) erfolgt.
57. Vorrichtung nach Aspekt 47 oder einem der folgenden, die derart eingerichtet ist, dass der Anregungsstrahl mit zwischen 0 und 10 Modulationsfrequenzen, vorzugsweise mit zwischen 0 und 5 Modulationsfrequenzen, weiter vorzugweise mit zwischen 1 und 3 Modulationsfrequenzen, weiter vorzugsweise nur mit einer Modulationsfrequenz moduliert wird und das Zeitverhalten des Reaktionssignals, insbesondere ein phasensensitives Verhalten des Reaktionssignals analysiert wird.
58. Vorrichtung nach Aspekt 47 oder einem der folgenden, die derart eingerichtet ist, dass das Tastverhältnis des Anregungsstrahls (SA) zwischen 3 und 10%, vorzugsweise zwischen 3 und 7% liegt.
59. Vorrichtung nach Aspekt 47 oder einem der folgenden, die derart eingerichtet ist, dass die Leistungsdichte des Anregungsstrahls (SA) an der Oberfläche des zu analysierenden Stoffes kleiner ist als 5 mW/mm2, insbesondere kleiner als 2 mW/mm2, weiter insbesondere kleiner als 1mW/mm2.
60. Vorrichtung nach Aspekt 47 oder einem der folgenden, die derart eingerichtet ist, dass das optische Medium (108) der Detektionseinrichtung mit einem Körperteil, insbesondere einem Finger und/oder einem Dialysat führenden Gefäß und/oder einem Blut führenden Gefäß eines Dialysegerätes in Kontakt bringbar ist und dass insbesondere eine oder mehrere Anregungswellenlängen für den Anregungsstrahl ausgewählt sind, die den Nachweis von Harnstoff ermöglichen.

Weitere Detektionsmethoden zum Nachweis eines Reaktionssignals nach Aussendung eines Anregungsstrahls können umfassen:
- Photoakustische Detektion - photoakustische Detektion mittels einer Stimmgabel oder eines anderen Schwingungselementes oder: eine leicht abgewandelte Form der Photoakustik mit offener QePAS-Zelle (Quartz enhanced PhotoAcustic Spectroskopy)). Durch diese Methoden können Druckschwankungen/schwingungen an der Stoffoberfläche nachgewiesen und ebenso ausgewertet werden, wie oben für die gemessene Strahlablenkung beschrieben.

Grundsätzlich können zur Tiefenprofilierung ermittelte Werte einer Phasenverschiebung des Reaktionssignals gegenüber einer periodischen Modulation des Anregungsstrahls genutzt werden. (Erwärmungs-/Abkühlphasen der Stoffoberfläche sollten dazu genauer bzgl. ihres Verlaufes ausgewertet werden).

Mit der beschriebenen Vorrichtung kann ein Vorrat von Klebestreifen zum Abtragen toter Hautschichten verbunden sein, um eine möglichst störungsfreie Messung an einem menschlichen Körper zu erlauben, sowie Pflaster mit Wärmeleitpaste, die auf das optische Medium regelmäßig aufgebracht werden können. Das optische Medium kann bei geeigneter Befestigung und Justierung der übrigen Teile austauschbar sein.

Die Vorrichtung kann zur Messung nicht nur an einem Finger einer Person, sondern auch an einer Lippe oder einem Ohrläppchen vorgesehen und eingerichtet sein.

Die Messung kann in einigen Ausführungen auch ohne direkte Berührung und Auflage des Fingers oder eines anderen Körperteils (auf Entfernung) funktionieren, wodurch ein kontaktloses Messen ermöglicht wird.

Die Messung kann durch Kombination mehrerer der geschilderten und erläuterten Messsysteme mit ähnlicher Fehleranfälligkeit bezüglich der Genauigkeit und Zuverlässigkeit verbessert werden.

DAQ und LockIn-Verstärker in der Auswertung können in einem Gerät zusammengefasst werden und die Auswertung kann insgesamt digitalisiert werden.

Die Messung kann mit der Vorrichtung auch an einer bewegten Stoffoberfläche durchgeführt werden, so dass im Zuge einer Rastermessung: Anregungslichtquelle und- und/oder Messlichtquelle während der Messung in einem Raster über die Haut fahren, sich ggf. Hautunregelmäßigkeiten kompensieren oder wegmitteln lassen.

Die Sensitivität der Detektionseinrichtung/Deflektionseinheit kann durch Einstellung /Variation der Wellenlänge des Probe-Beams/der Messlichtquelle optimiert werden. Hierzu kann die Messlichtquelle bzgl. der Wellenlänge veränderbar sein oder mehrere Laserlichtquellen verschiedener Wellenlänge zur Auswahl oder Kombination enthalten.

Für die Ablenkung des Pump-/Probe-Lasers kann eine optimale transversale Mode (TEM) gewählt werden.

Anregungssendeeinrichtung,, Messlichtquelle und Detektor können als ein gemeinsamer Array aufgebaut werden und die strahlen können im optischen Medium geeignet umgelenkt werden, um Aussendung und Empfang aller Strahlen auf eine Stelle zu konzentrieren.

Eine Linse auf oder im Kristall des optischen Mediums kann dazu beitragen, den Messlichtstrahl Reaktionssignalabhängig stärker abzulenken.

Zudem ist die Verwendung einer gapfree Photodiode zur Detektion denkbar, dann könnte eine Linse den Messlichtstrahl nach Austritt bündeln und so eine genauere Messung ermöglichen.

Eine zusätzliche Ausgestaltung der Erfindung gemäß den Patentansprüchen wird in dem folgenden Konzept dargestellt. Dieses Konzept stellt darüber hinaus auch für sich genommen, kombiniert mit den obigen Aspekten oder mit Anspruchsgegenständen zumindest eine eigenständige Erfindung dar. Die Anmelderin behält sich vor, diese Erfindung oder Erfindungen zu einem späteren Zeitpunkt zum Gegenstand von Ansprüchen zu machen. Dies kann im Rahmen dieser Anmeldung oder auch im Rahmen von späteren Teilanmeldungen oder Nachanmeldungen unter Inanspruchnahme der Priorität dieser Anmeldung geschehen.

Konzept für nichtinvasive Blutzuckermessung durch eine Bestimmung der Glucose in der Haut mittels Anregung durch Quantenkaskadenlaser und Messung der Wärmewelle durch Strahlungswärme.

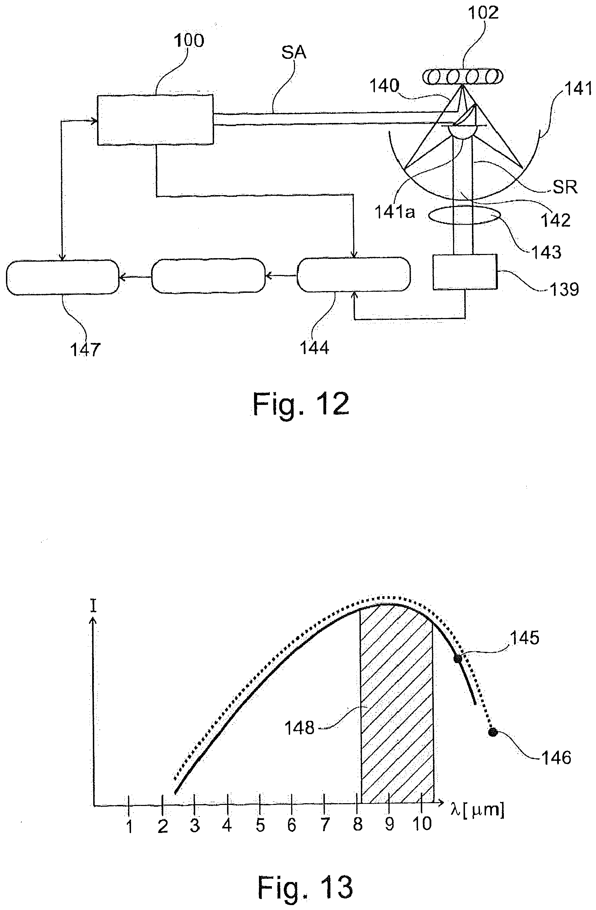

Es wird anhand der Figuren 12 und 13 ein Verfahren beschrieben, mit dem die Konzentration der Glucose oder auch einem anderen Stoff in der interstitiellen Flüssigkeit (ISF) in der Haut bestimmt werden kann. Glucose in der ISF ist repräsentativ für Blutglucose und folgt ihr schnell bei Änderungen. Das Verfahren besteht aus zumindest einzelnen oder Gruppen der folgenden Schritte oder aus der Gesamtabfolge:
1. Die Hautstelle 102 (in diesem Fall der erste Bereich der Stoffoberfläche) wird mit einem fokussierten und gegebenenfalls an einem Spiegel oder Hohlspiegel 140 reflektierten Strahl eines Quantenkaskadenlasers bestrahlt, der stufenweise oder kontinuierlich über einen bestimmten Infrarotbereich durchgestimmt wird, in dem Glucose spezifisch absorbiert. Anstelle des Quantenkaskadenlasers 100 kann auch ein Laserarray mit mehreren mit einzelnen Wellenlängen strahlenden Lasern verwendet werden. Der Spektralbereich kann (oder die einzelnen Wellenlängen, typisch 5 oder mehr Wellenlängen können) insbesondere zwischen ca. 900 und ca. 1300 cm⁻¹ liegen, in dem Glucose einen Absorptionsfingerprint, das heißt typische und repräsentative Absorptionslinien aufweist.
2. Der mit SA bezeichnete Anregungsstrahl wird kontinuierlich (CW-Laser) oder gepulst mit hoher Pulswiederholrate oder moduliert verwendet. Zusätzlich wird der Anregungsstrahl niederfrequent moduliert, insbesondere im Frequenzbereich zwischen 10 und 1000 Hz. Die niederfrequente Modulation kann mit verschiedenen periodischen Funktionen, in verschiedenen Ausführungsformen Sinus, Rechteck oder Sägezahn oder ähnlich erfolgen.
3. Durch die Bestrahlung der Haut dringt die IR-Strahlung bis etwa 50-100 µm tief in die Haut ein und regt - je nach Wellenlänge - bestimmte Schwingungen im Molekül Glucose an. Diese Anregungen vom Schwingungsniveau v0 nach v1 gehen innerhalb von sehr kurzer Zeit in den Grundzustand zurück; bei diesem Schritt wird Wärme frei.
4. Als Folge der Wärmeentwicklung nach (3) bildet sich eine Wärmewelle aus, die isotrop vom Ort der Absorption ausgeht. Abhängig von der thermischen Diffusionslänge, die durch die unter (2) beschriebene niederfrequenten Modulation bestimmt wird, erreicht die Wärmewelle periodisch mit der Modulationsfrequenz die Oberfläche der Haut.
5. Das periodische Auftauchen der Wärmewelle an der Oberfläche entspricht einer periodischen Modulation der Wärmestrahlungseigenschaft der Haut (Stoffoberfläche der Probe). Die Haut kann hier näherungsweise als Schwarzer Strahler beschrieben werden, dessen Gesamtemission durch das Stefan-Boltzmann Gesetz proportional zur vierten Potenz der Oberflächentemperatur ist.
6. Mit einem Detektor 139 für Wärmestrahlung, d.h. einem Infrarotdetektor, d.h. einem Thermoelement, Bolometer, Halbleiterdetektor oder ähnlichem, der auf die Hautstelle der Einstrahlung gerichtet ist, wird die unter (5) beschriebene periodische Temperaturerhöhung registriert. Sie ist abhängig von der unter (1) und (2) beschriebenen Einstrahlung von Infrarotlicht und von der unter (3) beschriebenen Absorption und somit abhängig von der Konzentration der Glucose. Die Wärmestrahlung SR (in diesem Fall das Reaktionssignal) wird mittels eines optischen Elementes, in einer Ausführungsform einer Infrarotlinse oder eines Spiegels, insbesondere eines konkaven Parabolspiegels 141, gesammelt und, in einer Ausführungsform über einen konvexen Spiegel 141a auf den Detektor 139 geleitet. Hierzu kann ein verwendeter Sammelspiegel in einer Ausführungsform eine Öffnung 142 aufweisen, durch die der gesammelte Strahl geleitet wird. Zudem kann ein Filter 143 im Strahlengang vorgesehen sein, das nur Infrarotstrahlung eines bestimmten Wellenlängenbereichs durchlässt.
7. Es kann bei der Bearbeitung der Reaktionssignale speziell die Modulationsfrequenz berücksichtigt werden, wozu das Reaktionssignal in einem Lock- in- Verstärker 144 verarbeitet werden kann. Durch Analyse der Phasenlage zwischen Anregungssignal und Wärmestrahlungssignal (Reaktionssignal) mittels einer Steuerungs- und Verarbeitungseinrichtung 147 kann die Tiefeninformation über die Tiefe unter der Stoffoberfläche gewonnen werden, aus der die Reaktionssignale vorwiegend erhalten werden.
8. Durch die Auswahl und Analyse verschiedener niederfrequenter Modulationsfrequenzen wie unter (2) beschrieben für den Anregungsstrahl und die Verknüpfung der Ergebnisse für verschiedene Modulationsfrequenzen (wobei die Ergebnisse für verschiedene Modulationsfrequenzen auch unterschiedlich gewichtet werden können) kann ebenfalls die Tiefeninformation erhalten werden. Dabei können ggf. Differenzverfahren oder andere Bestimmungsverfahren verwendet werden, um die Absorption der obersten Hautschichten zu kompensieren.
9. Um die Detektion der Wärmestrahlung nach (6) möglichst empfindlich zu machen, wird sie spektral breitbandig für den gesamten in Frage kommenden Infrarotbereich genutzt. Dabei sollen möglichst viele Bereiche der Planckschen Strahlungskurve genutzt werden. Um die Detektion für die intensive Anregungsstrahlung unempfindlich zu machen, wird die Detektion der Wärmestrahlung mit Sperrfilter (Notch-Filter) 143 für diese Anregungswellenlängen versehen. Der durch das Sperrfilter 143 durchgelassene Wellenlängenbereich 148 ist auch aus dem Diagramm der Figur 13 ersichtlich. Dort ist zudem die Intensität des Reaktionssignals wellenlängenabhängig einmal in einer ersten (durchgezogenen) Kurve 145 ohne einen Anregungsstrahl oder nur mit Anregungsstrahlung in für den nachzuweisenden Stoff unspezifischen Wellenlängen (d.h. ohne die Wellenlängen, bei denen spezifische Absorptionsbanden des Stoffes vorliegen) dargestellt und zudem in einer zweiten (gestrichelten) Kurve 146 eine vergleichbare Kurve, wobei ein Anregungsstrahl eingestrahlt wird, der spezifische Absorptionswellenlängen des nachzuweisenden Stoffes enthält.
10. Aus dem nach (6-9) gemessenen, von der Anregungswellenlänge abhängigen Wärmesignal wird somit in einer Ausführungsform, wenn Glucose nachgewiesen werden soll, zunächst bei nicht (oder unter Ausnahme von) glucoserelevanten Wellenlängen des Anregungsstrahls (Kurve 145) der Hintergrund bestimmt, anschließend bei (oder unter Einschluss von) glucoserelevanten Wellenlängen die Differenz zum Hintergrundsignal. Daraus ergibt sich die Glucosekonzentration in der Hautschicht oder den Hautschichten, die durch die ausgewählte Phasenlage nach (7) oder die verschiedenen Modulationsfrequenzen nach (8) oder deren Verknüpfung festgelegt werden.

Obwohl die Erfindung im Detail durch bevorzugte Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

Weiterhin werden die folgenden Beispiele offenbart.
1. Vorrichtung (10) zum Analysieren eines Stoffes (101) mit
   - einer Anregungssendeeinrichtung (100) zum Erzeugen mindestens eines elektromagnetischen Anregungsstrahls (SA), insbesondere Anregungs-Lichtstrahls, mit zumindest einer Anregungswellenlänge,
   - einer Detektionseinrichtung (106, 139) zur Detektion eines Reaktionssignals (SR) und
   - einer Einrichtung (107, 147) zum Analysieren des Stoffes anhand des detektierten Reaktionssignals (SR).
2. Vorrichtung nach Beispiel 1,
   dadurch gekennzeichnet, dass
   - die Anregungssendeeinrichtung eine Strahlungsquelle, in einer Ausführungsform eine monochromatische, insbesondere eine polarisierte Strahlungsquelle, weiter insbesondere eine Laserlichtquelle ist,
   - die Vorrichtung (10) ein optisches Medium (108) aufweist, das mit dem Stoff (101), insbesondere einem ersten Bereich (102) der Oberfläche des Stoffs, in direktem Kontakt steht,
   - wobei vorzugsweise die Anregungssendeeinrichtung (100) so angeordnet ist, dass der ausgesendete Anregungsstrahl (SA) in das optische Medium (108) eindringt und dieses an einem vorbestimmten Punkt an der Oberfläche des optischen Mediums wieder verlässt, und
   - die Vorrichtung eine Einrichtung (105) zum Aussenden eines Messstrahls (112), insbesondere Mess-Lichtstrahls, umfasst, die so angeordnet ist, dass der ausgesendete Messstrahl (112) in das optische Medium (108) eindringt und wobei vorzugsweise im Betrieb der Messstrahl (112) und der Anregungsstrahl (SA) sich auf einer Grenzfläche (GF) des optischen Mediums (108) und der Oberfläche des Stoffs (101), an der der Messstrahl (112) reflektiert wird, überlappen, und
   - die Detektionseinrichtung (106) eine Einrichtung zum Empfangen des das Reaktionssignal (SR) bildenden reflektierten Messstrahls (112) und/oder zum direkten oder indirekten Detektieren einer Ablenkung des reflektierten Messstrahls (112) ist.
3. Vorrichtung nach einem der voranstehenden Beispiele,
   dadurch gekennzeichnet, dass
   die Vorrichtung (10) ein optisches Medium (108) aufweist, das mit dem Stoff (101), insbesondere einem ersten Bereich (102) der Oberfläche des Stoffs, in direktem Kontakt steht und dass die Detektionseinrichtung (106) zur Detektion eines Reaktionssignals (SR)eine Parameteränderung des optischen Mediums, insbesondere in einem dem ersten Bereich benachbarten Bereich, infolge des Reaktionssignals, insbesondere eine Verformung und/oder Dichteänderung des optischen Mediums, erfasst.
4. Vorrichtung nach einem der voranstehenden Beispiele,
   dadurch gekennzeichnet, dass
   die Detektionseinrichtung ein mit dem optischen Medium verbundenes oder in dieses integriertes Piezoelement als Detektor zur Erfassung der Verformung und/oder Dichteänderung aufweist.
5. Vorrichtung nach einem der voranstehenden Beispiele,
   dadurch gekennzeichnet, dass
   dass die Detektionseinrichtung Temperatursensoren als Detektor zur Erfassung des Reaktionssignals aufweist.
6. Vorrichtung nach einem der voranstehenden Beispiele,
   dadurch gekennzeichnet, dass
   - die Vorrichtung eine Einrichtung (104) zur Intensitätsmodulierung des Anregungslichtstrahls (SA) aufweist und
   - die Detektionseinrichtung (106) zur Detektion eines zeitabhängigen Reaktionssignals (SR) in Abhängigkeit von der Wellenlänge des Anregungslichts und/oder der Intensitäts-Modulation des Anregungslichts geeignet ist.
7. Vorrichtung nach einem der voranstehenden Beispiele,
   dadurch gekennzeichnet, dass
   die Anregungssendeeinrichtung (100) den mindestens einen elektromagnetischen Anregungsstrahl (SA) in ein Stoffvolumen (103) einstrahlt, das unterhalb eines ersten Bereiches (102) der Oberfläche des Stoffs (101) liegt.
8. Vorrichtung nach einem der voranstehenden Beispiele,
   dadurch gekennzeichnet, dass
   die Anregungssendeeinrichtung (100) zwei oder mehr Sendeelemente (100a), insbesondere in Form eines ein-, zwei- oder mehrdimensionalen Sendeelementarrays, umfasst.
9. Vorrichtung nach einem der voranstehenden Beispiele,
   dadurch gekennzeichnet, dass
   die zwei oder mehr Sendeelemente (100a) jeweils einen eigenen elektromagnetischen Anregungsstrahl erzeugen und diesen in das Volumen unterhalb des ersten Bereiches (102) einstrahlen.
10. Vorrichtung nach einem der voranstehenden Beispiele,
   dadurch gekennzeichnet, dass
   sich die Wellenlängen der elektromagnetischen Anregungsstrahlen der zwei oder mehr Sendeelemente (100a) unterscheiden.
11. Vorrichtung nach einem der voranstehenden Beispiele,
   dadurch gekennzeichnet, dass
   die Anregungssendeeinrichtung zwei oder mehr Laser, insbesondere in Form eines ein-, zwei- oder mehrdimensionalen Laserarrays, und/oder zwei oder mehr Leuchtdioden, insbesondere in Form eines ein-, zwei- oder mehrdimensionalen Diodenarrays, umfasst.
12. Vorrichtung nach einem der voranstehenden Beispiele,
   dadurch gekennzeichnet, dass
   die Anregungssendeeinrichtung unmittelbar - oder mittelbar mittels einer Justagevorrichtung (109) - mit einem optischen Medium (108), das mit dem Stoff (101), insbesondere dem ersten Bereich (102) der Oberfläche des Stoffs (101), in direktem Kontakt steht, mechanisch fest verbunden ist.
13. Vorrichtung nach einem der voranstehenden Beispiele,
   dadurch gekennzeichnet, dass
   die Einrichtung (104) zur Intensitätsmodulierung eine elektrische Modulationseinrichtung, die mit der Anregungssendeeinrichtung (100) elektrisch in Verbindung steht und diese elektrisch ansteuert, umfasst oder durch eine solche gebildet ist.
14. Vorrichtung nach einem der voranstehenden Beispiele,
   dadurch gekennzeichnet, dass
   die Einrichtung (104) zur Intensitätsmodulierung wenigstens einen im Strahlengang angeordneten gesteuerten Spiegel (133, 134) umfasst.
15. Vorrichtung nach einem der voranstehenden Beispiele,
   dadurch gekennzeichnet, dass
   die Einrichtung (104) zur Intensitätsmodulierung wenigstens eine bezüglich ihrer Transparenz steuerbare, im Strahlengang angeordnete Schicht (138) umfasst oder durch eine solche gebildet ist.
16. Vorrichtung nach einem der voranstehenden Beispiele,
   dadurch gekennzeichnet, dass
   eine Einrichtung (105) zum Aussenden eines Messstrahls (112), insbesondere Mess-Lichtstrahls, in denjenigen Bereich eines optischen Mediums (108) vorgesehen ist, der mit dem ersten Bereich (102) der Oberfläche des Stoffs in Kontakt steht.
17. Vorrichtung nach einem der voranstehenden Beispiele,
   dadurch gekennzeichnet, dass
   die Einrichtung zum Aussenden eines Messstrahls und die Detektionseinrichtung derart zueinander ausgerichtet sind, dass die Detektionseinrichtung als das zeitabhängige Reaktionssignal den Messstrahl detektiert, nachdem dieser zumindest einmal an derjenigen Grenzfläche (GF) des optischen Mediums (108) reflektiert worden ist, die mit dem Stoff, insbesondere dem ersten Bereich (102) der Oberfläche des Stoffs (101), in Kontakt steht.
18. Vorrichtung nach einem der voranstehenden Beispiele,
   dadurch gekennzeichnet, dass
   die Einrichtung zum Aussenden eines Messstrahls und/oder die Detektionseinrichtung und/oder Anregungssendeeinrichtung unmittelbar mit dem optischen Medium mechanisch fest verbunden und/oder mittels eines Lichtwellenleiters (120) an dieses angekoppelt ist.
19. Vorrichtung nach einem der voranstehenden Beispiele,
   dadurch gekennzeichnet, dass
   das optische Medium unmittelbar eine Abbildungsoptik (128, 129) trägt und/oder in das optische Medium eine Abbildungsoptik (128, 129) integriert ist.
20. Vorrichtung nach einem der voranstehenden Beispiele,
   dadurch gekennzeichnet, dass
   die Oberfläche des optischen Mediums mehrere gegeneinander geneigte Teilflächen (110, 111) aufweist, an denen der Messstrahl (112), insbesondere der Mess-Lichtstrahl, mehrfach reflektiert wird.
21. Vorrichtung nach einem der voranstehenden Beispiele,
   dadurch gekennzeichnet, dass
   in oder an dem optischen Medium (108) ein oder mehrere Spiegelflächen (113, 114) zur Reflektion des Messstrahls (112), insbesondere Mess-Lichtstrahls, vorgesehen sind.
22. Vorrichtung nach einem der voranstehenden Beispiele,
   dadurch gekennzeichnet, dass
   die Anregungssendeeinrichtung (100) und/oder die Einrichtung (105) zum Aussenden des Messstrahls und/oder die Detektionseinrichtung (106) unmittelbar aneinander oder an einem gemeinsamen Träger (121) befestigt sind.
23. Vorrichtung nach einem der voranstehenden Beispiele,
   dadurch gekennzeichnet, dass
   der Träger (121) durch eine Leiterplatte, eine Metallplatte oder Kunststoffplatte oder ein Gehäuse (122) oder Gehäuseteil der Vorrichtung gebildet ist.
24. Vorrichtung nach einem der voranstehenden Beispiele,
   dadurch gekennzeichnet, dass
   die Anregungssendeeinrichtung einen integrierten Halbleiterbaustein umfasst, der einen oder mehrere Laserelemente sowie mindestens ein mikrooptisches Bauelement und vorzugsweise zusätzlich ein Modulationselement aufweist.
25. Vorrichtung nach einem der voranstehenden Beispiele,
   dadurch gekennzeichnet, dass
   das Modulationselement wenigstens ein gegenüber dem übrigen Halbleiterbaustein bewegliches und bezüglich einer Position steuerbares Element, insbesondere einen Spiegel aufweist.
26. Vorrichtung nach einem der voranstehenden Beispiele,
   dadurch gekennzeichnet, dass
   das Modulationselement eine in ihrer Strahlungsdurchlässigkeit steuerbare Schicht aufweist.
27. Vorrichtung nach einem der voranstehenden Beispiele,
   dadurch gekennzeichnet, dass
   das Modulationselement eine elektronische Ansteuerschaltung für die Modulation der einen oder mehreren Laserelemente aufweist.
28. Vorrichtung nach einem der voranstehenden Beispiele,
   dadurch gekennzeichnet, dass die Anregungssendeeinrichtung (100) dazu eingerichtet ist, seine Sendeleistung überwiegend, insbesondere zu mehr als 80% der Leistung im mittleren Infrarot-Wellenzahlbereich zwischen 900/cm und 1650/cm abzustrahlen.
29. Vorrichtung nach einem der voranstehenden Beispiele,
   dadurch gekennzeichnet, dass die Anregungssendeeinrichtung (100) dazu eingerichtet ist, zwischen 20 und 100 Wellenlängen einzeln gleichzeitig oder nacheinander, insbesondere zwischen 20 und 70, weiter insbesondere zwischen 20 und 50, weiter insbesondere zwischen 30 und 50 Wellenlängen abzustrahlen.
30. Vorrichtung nach einem der voranstehenden Beispiele,
   dadurch gekennzeichnet, dass bei einer Messung die Mehrzahl von angeregten Wellenlängen, insbesondere mehr als 50%, weiter insbesondere mehr als 80% der Wellenlängen bei Absorptionsmaxima im Bereich von Grundschwingungen des Glucosemoleküls liegen.
31. Vorrichtung nach einem der voranstehenden Beispiele,
   dadurch gekennzeichnet, dass die Vorrichtung, insbesondere die Anregungssendeeinrichtung dazu eingerichtet ist, die Anregung mit einer Frequenz zwischen 1Hz und 10 kHz, insbesondere zwischen 10 und 1000 Hertz, weiter insbesondere zwischen 20 und 500 Hertz zu modulieren.
32. Vorrichtung nach einem der voranstehenden Beispiele,
   dadurch gekennzeichnet, dass die Vorrichtung dazu eingerichtet ist, vorzugsweise nach einem Anregungspuls von zwischen 10 ms und 1000 ms Dauer, weiter vorzugsweise zwischen 50 und 500 ms, weiter vorzugsweise zwischen 50 und 150 ms Dauer, den zeitlichen Verlauf des Antwortsignals zu erfassen und einer Fourier- Transformation zu unterziehen,
33. Vorrichtung nach einem der voranstehenden Beispiele,
   dadurch gekennzeichnet, dass die Vorrichtung dazu eingerichtet ist, dass die Messung 2-10 Mal wiederholt wird und ein gemittelter Signalverlauf einer Integraltransformation, insbesondere Fouriertransformation oder Wavlet- Transformation mit geeigneter Basisfunktion, unterzogen oder eine Mittelung der einzelnen Integraltransformation, Insbesondere Fouriertrasnformation oder Wavlet- Transformation mit geeigneter Basisfunktion, durchgeführt wird.
34. Vorrichtung nach einem der voranstehenden Beispiele,
   dadurch gekennzeichnet, dass eine Vorrichtung zur Einstellung verschiedener Eintrittswinkel des Anregungsstrahls in den zu untersuchenden Stoff vorgesehen ist.
35. Vorrichtung nach einem der voranstehenden Beispiele,
   dadurch gekennzeichnet, dass die Vorrichtung dazu eingerichtet ist, dass die Messung nacheinander bei verschiedenen Eintrittswinkeln des Anregungsstrahls wiederholt wird und die Ergebnisse miteinander verknüpft, insbesondere voneinander subtrahiert werden, um die Einflüsse der oberen Hautschichten zu eliminieren.
36. Vorrichtung nach einem der vorhergehenden Beispiele, dadurch gekennzeichnet, dass diese auf Körpertemperatur des Lebewesens, insbesondere auf 37 Grad Celsius kalibriert ist, insbesondere dadurch, dass wenigstens Teile der Vorrichtung mittelbar oder unmittelbar ständig oder vorübergehend mit dem Körper eines Patienten im Kontakt sind.
37. Vorrichtung nach einem der vorhergehenden Beispiele, dadurch gekennzeichnet, dass diese eingerichtet ist zur Medikamentenspiegelbestimmung in einer Körperflüssigkeit, insbesondere zur Bestimmung von Paracetamol, Phenytoin, Valproinsäure, Lamotrigin,Phenobarbital, Flecainid,Digitoxin, Digoxin, Tacrolimus,Everolimus,Amiodaron,Aminoglykoside,Theophyllin,Vancomycin,Lithium,Carbam azepin*,*Sirolimus, und/ oder Methotrexat.
38. Vorrichtung nach einem der vorhergehenden Beispiele, dadurch gekennzeichnet, dass diese dazu eingerichtet ist dass bei Vorliegen von drei charakteristischen nebeneinanderliegenden Extremstellen einer Substanz mindestens zehn, vorzugsweise mindestens zwanzig Wellenlängen im Intervall zwischen den beiden äußeren Extremstellen ausgesendet werden.
39. Verfahren zum Analysieren eines Stoffes (101), wobei bei dem Verfahren
   - mit einer Anregungssendeeinrichtung (100) mindestens ein elektromagnetischer Anregungsstrahl (SA) mit zumindest einer Anregungswellenlänge durch den wenigstens teilweise gleichzeitigen oder aufeinander folgenden Betrieb mehrerer Laseremitter einer Laserlichtquelle erzeugt wird,
   - mit einer Detektionseinrichtung (106) ein Reaktionssignal (SR) detektiert wird und
   - anhand des detektierten Reaktionssignals (SR) der Stoff analysiert wird.
40. Verfahren nach Beispiel 39, dadurch gekennzeichnet, dass nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird.
41. Verfahren nach Beispiel 40,
   dadurch gekennzeichnet, dass
   Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird.
42. Verfahren nach Beispiel 41,
   dadurch gekennzeichnet, dass
   bei Verwendung von mehreren Modulationsfrequenzen des Pumpstrahls zur gleichen Zeit das detektierte Signal mittels eines Analyseverfahrens, vorzugsweise einer Fourier-Transformation, seinen Frequenzen entsprechend getrennt wird und
   jeweils nur das Teilsignal herausgefiltert wird, das der gewünschten Frequenz entspricht.
43. Verfahren nach einem der voranstehenden Beispiele 39-42,
   dadurch gekennzeichnet, dass
   - ein optisches Medium (108) mit dem Stoff (101), insbesondere einem ersten Bereich (102) der Oberfläche des Stoffs (101), in direkten Kontakt gebracht wird,
   - mit der Anregungssendeeinrichtung (100) der ausgesendete Anregungsstrahl (SA) erzeugt und insbesondere derart ausgestrahlt wird, dass dieser in das optische Medium (108) eindringt und dieses an einem vorbestimmten Punkt an der Oberfläche des optischen Mediums (108) wieder verlässt,
   - mit einer Einrichtung (105) zum Aussenden eines Messstrahls (112) ein Messstrahl (112), insbesondere Mess-Lichtstrahl, derart erzeugt wird, dass dieser in das optische Medium (108) eindringt und dass insbesondere der Messstrahl (112) und der Anregungsstrahl (SA) sich im Betrieb auf einer Grenzfläche (GF) des optischen Mediums (108) und der Oberfläche des Stoffs (101), an der der Messstrahl (112) reflektiert wird, überlappen, und
   - mit der Detektionseinrichtung (106) ein das Reaktionssignal (SR) bildender reflektierter Messstrahl (112) gemessen
   - und/oder direkt oder indirekt die Ablenkung des reflektierten Messstrahls detektiert wird.
44. Verfahren nach einem der voranstehenden Beispiele 39-43,
   dadurch gekennzeichnet, dass
   der Anregungsstrahl periodisch mit einer ersten Frequenz amplitudenmoduliert wird und eine Frequenzverschiebung des Reaktionssignals relativ zu der ersten Frequenz ermittelt und eine Bewegungsgeschwindigkeit des vermessenen Stoffes aufgrund des Dopplereffektes ermittelt wird.
45. Verfahren nach einem der voranstehenden Beispiele 39-44,
   dadurch gekennzeichnet, dass
   in Abhängigkeit von einer in dem Stoff ermittelten Stoffkonzentration eine Dosiereinrichtung zur Abgabe einer Substanz in den Stoff, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird.
46. Verfahren nach Beispiel 39 oder einem der folgenden, dadurch gekennzeichnet, dass die Anregung überwiegend, insbesondere zu mehr als 80% der Leistung im mittleren Infrarot-Wellenlängenbereich bei Wellenzahlen zwischen 900/cm und 1300/cm stattfindet.
47. Verfahren nach Beispiel 39 oder einem der folgenden, dadurch gekennzeichnet, dass zwischen 20 und 100 Wellenlängen einzeln angeregt werden, insbesondere zwischen 20 und 70, weiter insbesondere zwischen 20 und 50, weiter insbesondere zwischen 30 und 50 Wellenlängen.
48. Verfahren nach Beispiel 39 oder einem der folgenden, dadurch gekennzeichnet, dass die Mehrzahl von angeregten Wellenlängen, insbesondere mehr als 50%, weiter insbesondere mehr als 80% der Wellenlängen bei Absorptionsmaxima von Grundschwingungen des Glucosemoleküls liegen
49. Verfahren nach Beispiel 39 oder einem der folgenden, dadurch gekennzeichnet, dass die Anregung mit einer Frequenz zwischen 1 Hertz und 10 kHz, insbesondere zwischen 10 und 1000 Hertz, weiter insbesondere zwischen 20 und 500 Hertz moduliert wird
50. Verfahren nach Beispiel 39 oder einem der folgenden, dadurch gekennzeichnet, dass der zeitliche Verlauf des Antwortsignals erfasst und einer Integraltransformation unterzogen wird, beispielsweise einer Fouriertransformation oder einer Wavelettransformation mit geeigneter Basisfunktion, vorzugsweise nach einem Anregungspuls von zwischen 10 ms und 1000 ms Dauer, weiter vorzugsweise zwischen 50 und 500 ms, weiter vorzugsweise zwischen 50 und 150 ms Dauer,
51. Verfahren nach Beispiel 50, dadurch gekennzeichnet, dass die Messung 2-10 Mal wiederholt wird und ein gemittelter Signalverlauf einer Integraltransformation unterzogen oder eine Mittlung der einzelnen Integraltransformationen durchgeführt wird.
52. Verfahren nach Beispiel 39 oder einem der folgenden, dadurch gekennzeichnet, dass die Messung, die die Erfassung des Antwortsignals bei wenigstens einer Modulationsfrequenz oder zeitaufgelöst nach einem Sendeimpuls und bei einer oder mehreren Wellenzahlen umfasst, nacheinander bei verschiedenen Eintrittswinkeln des Anregungsstrahls wiederholt wird und dass die Ergebnisse miteinander verknüpft, insbesondere voneinander subtrahiert werden, um die Einflüsse der oberen Hautschichten zu eliminieren.
53. Verfahren nach Beispiel 39 oder einem der folgenden, dadurch gekennzeichnet, dass der Abtaststrahl/Messstrahl der detektionseinrichtung während der Messung wenigstens zeitweise im Wellenzahlenbereich zwischen 15000/cm und 16000/cm, insbesondere zeitweise zwischen 15500/cm und 16000/cm, weiter insbesondere zeitweise zwischen 15700/cm und 16000/cm betrieben wird.
54. Verfahren nach Beispiel 39 oder einem der folgenden dadurch gekennzeichnet, dass bei einer Messung, bei der der Anregungsstrahl nacheinander auf verschiedene Wellenzahlen und/oder Wellenzalbereiche eingestellt wird, wenigstens 5%, insbesondere wenigstens 10%, weiter insbesondere wenigstens 30%, weiter insbesondere wenigstens 50% des abgedeckten Wellenzahl- oder Wellenlängenbereiches in Bereichen des Spektrums liegt, die bezüglich des nachzuweisenden Stoffes, insbesondere Glukose, insensitiv sind.
55. Verfahren nach Beispiel 39 oder einem der folgenden, dadurch gekennzeichnet, dass zur Berücksichtigung des von der Glukosekonzentration unabhängigen Absorptionsverhaltens der Probe oder des Gewebes wenigstens ein oder zwei Wellenzahlbereiche oder Wellenlängenbereiche ohne eine signifikante Absorption durch Glukose vorgesehen sind, in denen die Absorption vermessen wird.
56. Verfahren nach Beispiel 55, dadurch gekennzeichnet, dass wenigstens einer der Wellenzahlbereiche zwischen 1610 und 1695 cm⁻¹ (Amid 1) liegt.
57. Verfahren nach Beispiel 55 oder 56, dadurch gekennzeichnet, dass wenigstens einer der Wellenzahlbereiche zwischen 1480 und 1575 cm⁻¹ (Amid 2) liegt.
58. Verfahren nach Beispiel 39 oder einem der folgenden, dadurch gekennzeichnet, dass für eine Messung zunächst ein zu untersuchender Tiefenbereich in der Probe selektiert wird und dass danach der Anregungsstrahl derart gesteuert wird, dass zwischen Zeitbereichen (Anregungspulsen), in denen der Anregungsstrahl ausgesendet wird, jeweils mindestens ein Zeitraum liegt, der der Diffusionszeit einer thermischen Welle entspricht, die sie zum Durchlaufen der Strecke zwischen dem zu untersuchenden Tiefenbereich in der Probe und der Probenoberfläche benötigt.
59. Verfahren nach Beispiel 39 oder einem der folgenden, dadurch gekennzeichnet, dass der zeitliche Intensitätsverlauf der Absorption jeweils nach dem Ende eines Zeitbereichs gemessen wird, in dem der Anregungsstrahl ausgesendet wird. (Phasenlage des Antwortsignals).
60. Verfahren nach Beispiel 39 oder einem der folgenden, dadurch gekennzeichnet, dass zur Ansteuerung eines Lasers zur Erzeugung des Anregungsstrahls eine Reihe von gespeicherten äquidistanten oder nicht äquidistanten Einstellungswerten nacheinander eingestellt werden, die jeweils eine Wellenzahl/Wellenlänge des Lasers bestimmen und unter denen insbesondere wenigstens 3, weiter insbesondere wenigstens 5 Wellenzahlen/Wellenlängen von Absorptionsmaxima eines nachzuweisenden Stoffes sind. (lookup table)
61. Verfahren nach Beispiel 39 oder einem der folgenden, dadurch gekennzeichnet, dass vor und/oder während und/oder nach einer Messung der mechanische Druck und/oder der Druck pro Flächeneinheit erfasst wird, mit dem das optische Medium, in dem der Abtaststrahl reflektiert wird, gegen die Probe gedrückt ist.
61. Verfahren nach Beispiel 39 oder einem der folgenden, dadurch gekennzeichnet, dass vor und/oder während und/oder nach einer Messung die Feuchtigkeit der Umgebungsluft und/oder ein Feuchtigkeitsgehalt der Probe oder die Feuchtigkeit der oberen Schichten oder der Oberfläche der Probe ermittelt wird.
62. Verfahren zum Analysieren eines Stoffes (101), wobei bei dem Verfahren mit einer Anregungssendeeinrichtung (100) mindestens ein elektromagnetischer Anregungsstrahl (SA) mit zumindest einer Anregungswellenlänge durch den aufeinander folgenden Betrieb einer oder mehrerer oder durch den wenigstens teilweise gleichzeitigen Betrieb mehrerer Laseremitter einer Laserlichtquelle erzeugt wird,
   mit einer Detektionseinrichtung (106) ein Reaktionssignal (SR) detektiert wird und anhand des detektierten Reaktionssignals (SR) der Stoff analysiert wird.
63. Verfahren nach einem der Beispiele 39 bis 62, dadurch gekennzeichnet, dass die Temperatur des /der Laseremitter der Anregungssendeeinrichtung (100) und/oder die Temperatur der Detektionseinrichtung (106), insbesondere eines optischen Mediums (108) und/oder einer Detektionsstrahlungsquelle (105) und/oder eines optischen Sensors während der Analyse konstant gehalten, insbesondere auf einer vorgegebenen Temperatur konstant gehalten, weiter insbesondere auf einer vorgegebenen Temperatur oberhalb der Körpertemperatur eines Menschen, weiter vorzugsweise oberhalb von 37 Grad Celsius, weiter vorzugsweise oberhalb von 38 oder 40 Grad Celsius gehalten wird.
64. Verfahren nach einem der Beispiele 39 bis 63, dadurch gekennzeichnet, dass während der Analyse die Temperatur des /der Laseremitter der Anregungssendeeinrichtung (100) und /oder die Temperatur der Detektionseinrichtung (106), insbesondere eines optischen Mediums (108) und/oder einer Detektionsstrahlungsquelle (105) und /oder eines optischen Sensors erfasst und bei der Auswertung der Analyse durch Verknüpfung der Messergebnisse mit Temperaturkorrekturwerten berücksichtigt wird.
65. Verfahren nach einem der Beispiele 39 bis 64, dadurch gekennzeichnet, dass der Stoff, insbesondere ein Körperteil, insbesondere ein Finger, gegen ein optisches Medium (108) gedrückt wird, das Teil der Detektionseinrichtung ist, dass der auf das Medium durch das angedrückte Körperteil ausgeübte Druck erfasst wird und dass in Abhängigkeit von dem erfassten Druck auf das Medium (108) die Anregungssendeeinrichtung (100) eingeschaltet und/oder diese in Abhängigkeit von einer Verminderung des Drucks, insbesondere unter eine bestimmte Schwelle, ausgeschaltet wird.
66. Verfahren nach einem der Beispiele 39 bis 65, dadurch gekennzeichnet, dass der Stoff, insbesondere ein Körperteil, insbesondere ein Finger, gegen ein optisches Medium (108) gedrückt wird, das Teil der Detektionseinrichtung ist, dass eine Verdunkelung des Mediums im Bereich des angedrückten Körperteils erfasst wird und dass durch Verdunkelung des Mediums die Anregungssendeeinrichtung (100) eingeschaltet und/oder diese durch eine Vergrößerung der Helligkeit im Medium insbesondere über eine bestimmte Schwelle, ausgeschaltet wird.
67. Verfahren nach einem der Beispiele 39 bis 66, dadurch gekennzeichnet, dass der Stoff, insbesondere ein Körperteil, insbesondere ein Finger, gegen ein optisches Medium (108) gedrückt wird, das Teil der Detektionseinrichtung ist, dass ein Feuchtigkeitsgrad des Mediums (108) im Bereich des angedrückten Körperteils erfasst wird und dass durch Erreichen eines vorgegebenen Feuchtigkeitsgrads an dem Medium die Anregungssendeeinrichtung (100) eingeschaltet und/oder diese durch eine Verminderung des Feuchtigkeitsgrades, insbesondere unter eine bestimmte Schwelle, oder durch eine Erhöhung des Feuchtigkeitsgrades, insbesondere über eine bestimmte Schwelle, ausgeschaltet wird.
68. Verfahren nach einem der Beispiele 39 bis 67, dadurch gekennzeichnet, dass während der Durchführung des Verfahrens ein an das optische Medium (108) gedrücktes Körperteil an dem Medium (108) fixiert, insbesondere durch Einklemmen des Körperteils an dem Medium, Verkleben des Körperteils mit dem Medium, Adhäsion des Körperteils an dem Medium oder durch Vakuum- Ansaugen des Köperteils an das Medium fixiert wird.
69. Verfahren nach einem der Beispiele 39 bis 68, dadurch gekennzeichnet, dass der Anregungsstrahl(SA) mit mindestens zwei Anregungswellenlängen oder Gruppen von Anregungswellenlängen ausgesendet wird, wobei eine erste Anregungswellenlänge oder Gruppe von Anregungswellenlängen den Nachweis einer in dem Stoff nachzuweisenden Substanz ermöglicht, während wenigstens eine weitere Anregungswellenlänge oder Gruppe von Anregungswellenlängen den Nachweis einer von einer nachzuweisenden Substanz verschiedenen Referenzsubstanz ermöglicht und dass für die wenigstens zwei verschiedenen Substanzen Profile bezüglich ihrer Dichteverteilung in der Tiefe des Stoffes ermittelt und miteinander verknüpft werden, und wobei insbesondere ein Tiefenprofil oder wenigstens ein oder mehrere Parameter eines Tiefenprofils der Referenzsubstanz in dem Stoff bekannt ist.
70. Verfahren nach Beispiel 69, wobei der Anregungsstrahl (SA) moduliert, vorzugsweise nacheinander mit verschiedenen Modulationsfrequenzen moduliert wird, wobei Reaktionssignale für verschiedene Modulationsfrequenzen detektiert werden oder ein Zeitverhalten des Reaktionssignals bei einer Änderung der Intensität des Anregungsstrahls, insbesondere bei dessen periodischem Anschalten und/oder Abschalten, analysiert wird.
71. Verfahren nach Beispiel 70, dadurch gekennzeichnet, dass die Modulation des Anregungsstrahls (SA) durch die Ansteuerung einer den Anregungslichtstrahl erzeugenden Lasereinrichtung (100) erfolgt.
72. Verfahren nach einem der Beispiele 70 oder 71, dadurch gekennzeichnet, dass der Anregungsstrahl mit zwischen 0 und 10 Modulationsfrequenzen, vorzugsweise mit zwischen 0 und 5 Modulationsfrequenzen, weiter vorzugweise mit zwischen 1 und 3 Modulationsfrequenzen, weiter vorzugsweise nur mit einer Modulationsfrequenz moduliert wird und das Zeitverhalten des Reaktionssignals, insbesondere ein phasensensitives Verhalten des Reaktionssignals analysiert wird.
73. Verfahren nach einem der Beispiele 62 bis 72, dadurch gekennzeichnet, dass das Tastverhältnis des Anregungsstrahls (SA) zwischen 3 und 10%, vorzugsweise zwischen 3 und 7% liegt.
74. Verfahren nach einem der Beispiele 62 bis 73, dadurch gekennzeichnet, dass die Leistungsdichte des Anregungsstrahls (SA) an der Oberfläche des zu analysierenden Stoffes kleiner ist als 5 mW/mm2, insbesondere kleiner als 2 mW/mm2, weiter insbesondere kleiner als 1mW/mm2.
75. Verfahren nach einem der Beispiele 39 bis 74, dadurch gekennzeichnet, dass das optische Medium (108) der Detektionseinrichtung mit einem Körperteil, insbesondere einem Finger und/oder eine Dialysat führenden Gefäß und/oder einem Blut führenden Gefäß eines Dialysegerätes in Kontakt gebracht wird und dass insbesondere eine oder mehrere Anregungswellenlängen gewählt werden, die den Nachweis von Harnstoff ermöglichen.

### Bezugszeichenliste

- 10: Vorrichtung
- 100: Anregungssendeeinrichtung / Anregungs-Lichtquelle
- 100a: Emitter / Sendeelemente
- 101: Stoff
- 102: erster Bereich
- 103: Volumen
- 104: Einrichtung
- 105: Einrichtung
- 106: Detektionseinrichtung
- 107: Verarbeitungseinrichtung /Auswertungseinrichtung
- 107a: Speicher
- 108: optisches Medium
- 108a: Flächenabschnitt
- 108b: Flächenabschnitt
- 109: Justagevorrichtung
- 110: Teilfläche
- 111: Teilfläche
- 112: Messstrahl / Mess-Lichtstrahl
- 113: Spiegelfläche
- 114: Spiegelfläche
- 116: Öffnung
- 117: Öffnung
- 118: Öffnung
- 119: Anschlusskörper
- 120: Lichtwellenleiter
- 121: Träger
- 122: Gehäuse
- 123: Körper
- 124: Seite
- 125: Riemen
- 126: Fingerbeere
- 127: Justageeinrichtung
- 128: Abbildungsoptik
- 129: Abbildungsoptik
- 130: optischer Detektor / Kamera
- 131: Datenverarbeitungseinrichtung
- 132: Steuerung
- 133: Mikrospiegel
- 134: Mikrospiegel
- 135: Mikro-elektromechanisches System
- 136: Umlenkeinrichtung
- 137: Steuereinrichtung
- 138: Schicht
- 139: Infrarot- Detektor
- 140: Spiegel
- 141: Hohlspiegel
- 142: Öffnung in 141
- 143: Wellenlängenfilter
- 144: Lock-in-Verstärker
- 145: Signalkurve des Reaktionssignals (durchgezogen)
- 146: Signalkurve des Reaktionssignals (gestrichelt)
- 147: Steuerungs- und Verarbeitungseinrichtung
- 148: Wellenlängenbereich

- BZA: Blutzuckerspiegelangabe
- D: Detektionsergebnis
- GF: Grenzfläche
- SA: Anregungsstrahl
- SR: Reaktionssignal

## Patentansprüche

1. Vorrichtung (10) zum Analysieren eines Stoffes (101) mit
- einer Anregungssendeeinrichtung (100) zum Erzeugen mindestens eines elektromagnetischen Anregungsstrahls (SA), insbesondere AnregungsLichtstrahls, mit zumindest einer Anregungswellenlänge,
- einer Detektionseinrichtung (106, 139) zur Detektion eines Reaktionssignals (SR) und
- einer Einrichtung (107, 147) zum Analysieren des Stoffes anhand des detektierten Reaktionssignals (SR).

2. Vorrichtung nach Anspruch 1, **dadurch gekennzeichnet, dass**
- die Anregungssendeeinrichtung eine Strahlungsquelle, in einer Ausführungsform eine monochromatische, insbesondere eine polarisierte Strahlungsquelle, weiter insbesondere eine Laserlichtquelle ist,
- die Vorrichtung (10) ein optisches Medium (108) aufweist, das mit dem Stoff (101), insbesondere einem ersten Bereich (102) der Oberfläche des Stoffs, in direktem Kontakt steht,
- wobei vorzugsweise die Anregungssendeeinrichtung (100) so angeordnet ist, dass der ausgesendete Anregungsstrahl (SA) in das optische Medium (108) eindringt und dieses an einem vorbestimmten Punkt an der Oberfläche des optischen Mediums wieder verlässt, und
- die Vorrichtung eine Einrichtung (105) zum Aussenden eines Messstrahls (112), insbesondere Mess-Lichtstrahls, umfasst, die so angeordnet ist, dass der ausgesendete Messstrahl (112) in das optische Medium (108) eindringt und wobei vorzugsweise im Betrieb der Messstrahl (112) und der Anregungsstrahl (SA) sich auf einer Grenzfläche (GF) des optischen Mediums (108) und der Oberfläche des Stoffs (101), an der der Messstrahl (112) reflektiert wird, überlappen, und
- die Detektionseinrichtung (106) eine Einrichtung zum Empfangen des das Reaktionssignal (SR) bildenden reflektierten Messstrahls (112) und/oder zum direkten oder indirekten Detektieren einer Ablenkung des reflektierten Messstrahls (112) ist.

3. Vorrichtung nach einem der voranstehenden Ansprüche,
**dadurch gekennzeichnet, dass**
die Vorrichtung (10) ein optisches Medium (108) aufweist, das mit dem Stoff (101), insbesondere einem ersten Bereich (102) der Oberfläche des Stoffs, in direktem Kontakt steht und dass die Detektionseinrichtung (106) zur Detektion eines Reaktionssignals (SR)eine Parameteränderung des optischen Mediums, insbesondere in einem dem ersten Bereich benachbarten Bereich, infolge des Reaktionssignals, insbesondere eine Verformung und/oder Dichteänderung des optischen Mediums, erfasst, und/oder
bei der die Detektionseinrichtung ein mit dem optischen Medium verbundenes oder in dieses integriertes Piezoelement als Detektor zur Erfassung der Verformung und/oder Dichteänderung aufweist, und/oder bei der die Detektionseinrichtung Temperatursensoren als Detektor zur Erfassung des Reaktionssignals aufweist.

4. Vorrichtung nach einem der voranstehenden Ansprüche,
**dadurch gekennzeichnet, dass**
die Vorrichtung eine Einrichtung (104) zur Intensitätsmodulierung des Anregungslichtstrahls (SA) aufweist unddie Detektionseinrichtung (106) zur Detektion eines zeitabhängigen Reaktionssignals (SR) in Abhängigkeit von der Wellenlänge des Anregungslichts und/oder der Intensitäts-Modulation des Anregungslichts geeignet ist, wobei die Einrichtung (104) zur Intensitätsmodulierung vorzugsweise eine elektrische Modulationseinrichtung, die mit der Anregungssendeeinrichtung (100) elektrisch in Verbindung steht und diese elektrisch ansteuert, umfasst oder durch eine solche gebildet ist, oder die Einrichtung (104) zur Intensitätsmodulierung vorzugsweise wenigstens einen im Strahlengang angeordneten gesteuerten Spiegel (133, 134) umfasst, oder die Einrichtung (104) zur Intensitätsmodulierung vorzugsweise wenigstens eine bezüglich ihrer Transparenz steuerbare, im Strahlengang angeordnete Schicht (138) umfasst oder durch eine solche gebildet ist, und/oder dass die Anregungssendeeinrichtung (100) den mindestens einen elektromagnetischen Anregungsstrahl (SA) in ein Stoffvolumen (103) einstrahlt, das unterhalb eines ersten Bereiches (102) der Oberfläche des Stoffs (101) liegt.

5. Vorrichtung nach einem der voranstehenden Ansprüche,
**dadurch gekennzeichnet, dass** die Anregungssendeeinrichtung (100) zwei oder mehr Sendeelemente (100a), insbesondere in Form eines ein-, zwei- oder mehrdimensionalen Sendeelementarrays, umfasst, wobei die Sendeelemente insbesondere durch Laser oder Leuchtdioden gebildet sind, und/oder **dadurch gekennzeichnet, dass** die Anregungssendeeinrichtung unmittelbar - oder mittelbar mittels einer Justagevorrichtung (109) - mit einem optischen Medium (108), das mit dem Stoff (101), insbesondere dem ersten Bereich (102) der Oberfläche des Stoffs (101), in direktem Kontakt steht, mechanisch fest verbunden ist.

6. Vorrichtung nach einem der voranstehenden Ansprüche,
**dadurch gekennzeichnet, dass**
die Einrichtung zum Aussenden eines Messstrahls und/oder die Detektionseinrichtung und/oder Anregungssendeeinrichtung unmittelbar mit dem optischen Medium mechanisch fest verbunden und/oder mittels eines Lichtwellenleiters (120) an dieses angekoppelt ist, und/oder
dass die Anregungssendeeinrichtung (100) und/oder die Einrichtung (105) zum Aussenden des Messstrahls und/oder die Detektionseinrichtung (106) unmittelbar aneinander oder an einem gemeinsamen Träger (121) befestigt sind, wobei der Träger (121) vorzugsweise durch eine Leiterplatte, eine Metallplatte oder Kunststoffplatte oder ein Gehäuse (122) oder Gehäuseteil der Vorrichtung gebildet ist, und/oder dass das optische Medium unmittelbar eine Abbildungsoptik (128, 129) trägt und/oder in das optische Medium eine Abbildungsoptik (128, 129) integriert ist.

7. Vorrichtung nach einem der voranstehenden Ansprüche,
**dadurch gekennzeichnet, dass** die Anregungssendeeinrichtung (100) dazu eingerichtet ist, seine Sendeleistung überwiegend, insbesondere zu mehr als 80% der Leistung im mittleren Infrarot-Wellenzahlbereich zwischen 900/cm und 1650/cm abzustrahlen, und/oder dass die Anregungssendeeinrichtung (100) dazu eingerichtet ist, zwischen 20 und 100 Wellenlängen einzeln gleichzeitig oder nacheinander, insbesondere zwischen 20 und 70, weiter insbesondere zwischen 20 und 50, weiter insbesondere zwischen 30 und 50 Wellenlängen abzustrahlen, wobei die Vorrichtung insbesondere dazu eingerichtet ist, dass bei Vorliegen von drei charakteristischen nebeneinanderliegenden Extremstellen einer Substanz mindestens zehn, vorzugsweise mindestens zwanzig Wellenlängen im Intervall zwischen den beiden äußeren Extremstellen ausgesendet werden, und/oder dass bei einer Messung die Mehrzahl von angeregten Wellenlängen, insbesondere mehr als 50%, weiter insbesondere mehr als 80% der Wellenlängen bei Absorptionsmaxima im Bereich von Grundschwingungen des Glucosemoleküls liegen, und/oder dass die Vorrichtung, insbesondere die Anregungssendeeinrichtung dazu eingerichtet ist, die Anregung mit einer Frequenz zwischen 1Hz und 10 kHz, insbesondere zwischen 10 und 1000 Hertz, weiter insbesondere zwischen 20 und 500 Hertz zu modulieren.

8. Vorrichtung nach einem der voranstehenden Ansprüche, **dadurch gekennzeichnet, dass** die Vorrichtung dazu eingerichtet ist, vorzugsweise nach einem Anregungspuls von zwischen 10 ms und 1000 ms Dauer, weiter vorzugsweise zwischen 50 und 500 ms, weiter vorzugsweise zwischen 50 und 150 ms Dauer, den zeitlichen Verlauf des Antwortsignals zu erfassen und einer Fourier- Transformation zu unterziehen, und/oder dass die Vorrichtung dazu eingerichtet ist, dass die Messung 2-10 Mal wiederholt wird und ein gemittelter Signalverlauf einer Integraltransformation, insbesondere Fouriertransformation oder Wavelet- Transformation mit geeigneter Basisfunktion, unterzogen oder eine Mittelung der einzelnen Integraltransformation, Insbesondere Fouriertransformation oder Wavelet- Transformation mit geeigneter Basisfunktion, durchgeführt wird.

9. Verfahren zum Analysieren eines Stoffes (101), wobei bei dem Verfahren
- mit einer Anregungssendeeinrichtung (100) mindestens ein elektromagnetischer Anregungsstrahl (SA) mit zumindest einer Anregungswellenlänge durch den wenigstens teilweise gleichzeitigen oder aufeinander folgenden Betrieb mehrerer Laseremitter einer Laserlichtquelle erzeugt wird,
- mit einer Detektionseinrichtung (106) ein Reaktionssignal (SR) detektiert wird und
- anhand des detektierten Reaktionssignals (SR) der Stoff analysiert wird.

10. Verfahren nach Anspruch 9, **dadurch gekennzeichnet, dass** nacheinander unter Verwendung verschiedener Modulationsfrequenzen der Anregungssendeeinrichtung Reaktionssignale, insbesondere zeitliche Reaktionssignalverläufe, ermittelt werden und dass mehrere Reaktionssignalverläufe zu verschiedenen Modulationsfrequenzen miteinander verknüpft werden und dass daraus insbesondere eine für einen Tiefenbereich unter der Stoffoberfläche spezifische Information gewonnen wird, wobei vorzugsweise Reaktionssignalverläufe zu verschiedenen Modulationsfrequenzen jeweils für verschiedene Wellenlängen des Anregungsstrahls ermittelt werden und daraus insbesondere jeweils eine für einen Tiefenbereich unter der Stoffoberfläche spezifische Information gewonnen wird, wobei vorzugsweise bei Verwendung von mehreren Modulationsfrequenzen des Pumpstrahls zur gleichen Zeit das detektierte Signal mittels eines Analyseverfahrens, vorzugsweise einer Fourier-Transformation, seinen Frequenzen entsprechend getrennt wird und
jeweils nur das Teilsignal herausgefiltert wird, das der gewünschten Frequenz entspricht.

11. Verfahren nach Anspruch 9 oder 10, **dadurch gekennzeichnet, dass** ein optisches Medium (108) mit dem Stoff (101), insbesondere einem ersten Bereich (102) der Oberfläche des Stoffs (101), in direkten Kontakt gebracht wird, mit der Anregungssendeeinrichtung (100) der ausgesendete Anregungsstrahl (SA) erzeugt und insbesondere derart ausgestrahlt wird, dass dieser in das optische Medium (108) eindringt und dieses an einem vorbestimmten Punkt an der Oberfläche des optischen Mediums (108) wieder verlässt, mit einer Einrichtung (105) zum Aussenden eines Messstrahls (112) ein Messstrahl (112), insbesondere Mess-Lichtstrahl, derart erzeugt wird, dass dieser in das optische Medium (108) eindringt und dass insbesondere der Messstrahl (112) und der Anregungsstrahl (SA) sich im Betrieb auf einer Grenzfläche (GF) des optischen Mediums (108) und der Oberfläche des Stoffs (101), an der der Messstrahl (112) reflektiert wird, überlappen, und mit der Detektionseinrichtung (106) ein das Reaktionssignal (SR) bildender reflektierter Messstrahl (112) gemessen und/oder direkt oder indirekt die Ablenkung des reflektierten Messstrahls detektiert wird, und/oder dass
in Abhängigkeit von einer in dem Stoff ermittelten Stoffkonzentration eine Dosiereinrichtung zur Abgabe einer Substanz in den Stoff, insbesondere in einen Patientenkörper angesteuert wird und/oder ein akustisches und/oder optisches Signal ausgegeben wird und/oder ein Signal mittels einer Funkverbindung an eine Verarbeitungseinrichtung abgegeben wird.

12. Verfahren nach einem der Ansprüche 9 bis 11, **dadurch gekennzeichnet, dass** die Anregung überwiegend, insbesondere zu mehr als 80% der Leistung im mittleren Infrarot-Wellenlängenbereich bei Wellenzahlen zwischen 900/cm und 1300/cm stattfindet, und/oder dass zwischen 20 und 100 Wellenlängen einzeln angeregt werden, insbesondere zwischen 20 und 70, weiter insbesondere zwischen 20 und 50, weiter insbesondere zwischen 30 und 50 Wellenlängen, und/oder dass die Mehrzahl von angeregten Wellenlängen, insbesondere mehr als 50%, weiter insbesondere mehr als 80% der Wellenlängen bei Absorptionsmaxima von Grundschwingungen des Glucosemoleküls liegen, und/oder dass die Anregung mit einer Frequenz zwischen 1 Hertz und 10 kHz, insbesondere zwischen 10 und 1000 Hertz, weiter insbesondere zwischen 20 und 500 Hertz moduliert wird, und/oder dass der zeitliche Verlauf des Antwortsignals erfasst und einer Integraltransformation unterzogen wird, beispielsweise einer Fouriertransformation oder einer Wavelettransformation mit geeigneter Basisfunktion, vorzugsweise nach einem Anregungspuls von zwischen 10 ms und 1000 ms Dauer, weiter vorzugsweise zwischen 50 und 500 ms, weiter vorzugsweise zwischen 50 und 150 ms Dauer, wobei die Messung vorzugsweise 2-10 Mal wiederholt wird und ein gemittelter Signalverlauf einer Integraltransformation unterzogen oder eine Mittlung der einzelnen Integraltransformationen durchgeführt wird.

13. Verfahren nach einem der Ansprüche 9 bis 11, **dadurch gekennzeichnet, dass** der zeitliche Intensitätsverlauf der Absorption jeweils nach dem Ende eines Zeitbereichs gemessen wird, in dem der Anregungsstrahl ausgesendet wird, und/oder dass vor und/oder während und/oder nach einer Messung der mechanische Druck und/oder der Druck pro Flächeneinheit erfasst wird, mit dem das optische Medium, in dem der Abtaststrahl reflektiert wird, gegen die Probe gedrückt ist, und/oder vor und/oder während und/oder nach einer Messung die Feuchtigkeit der Umgebungsluft und/oder ein Feuchtigkeitsgehalt der Probe oder die Feuchtigkeit der oberen Schichten oder der Oberfläche der Probe ermittelt wird.

14. Verfahren zum Analysieren eines Stoffes (101), wobei bei dem Verfahren mit einer Anregungssendeeinrichtung (100) mindestens ein elektromagnetischer Anregungsstrahl (SA) mit zumindest einer Anregungswellenlänge durch den aufeinander folgenden Betrieb einer oder mehrerer oder durch den wenigstens teilweise gleichzeitigen Betrieb mehrerer Laseremitter einer Laserlichtquelle erzeugt wird, mit einer Detektionseinrichtung (106) ein Reaktionssignal (SR) detektiert wird und anhand des detektierten Reaktionssignals (SR) der Stoff analysiert wird, wobei vorzugsweise die Temperatur des /der Laseremitter der Anregungssendeeinrichtung (100) und/oder die Temperatur der Detektionseinrichtung (106), insbesondere eines optischen Mediums (108) und/oder einer Detektionsstrahlungsquelle (105) und/oder eines optischen Sensors während der Analyse konstant gehalten, insbesondere auf einer vorgegebenen Temperatur konstant gehalten, weiter insbesondere auf einer vorgegebenen Temperatur oberhalb der Körpertemperatur eines Menschen, weiter vorzugsweise oberhalb von 37 Grad Celsius, weiter vorzugsweise oberhalb von 38 oder 40 Grad Celsius gehalten wird, und/oder wobei vorzugsweise während der Analyse die Temperatur des /der Laseremitter der Anregungssendeeinrichtung (100) und /oder die Temperatur der Detektionseinrichtung (106), insbesondere eines optischen Mediums (108) und/oder einer Detektionsstrahlungsquelle (105) und /oder eines optischen Sensors erfasst und bei der Auswertung der Analyse durch Verknüpfung der Messergebnisse mit Temperaturkorrekturwerten berücksichtigt wird.

15. Verfahren nach einem der Ansprüche 9 bis 14, **dadurch gekennzeichnet, dass** der Stoff, insbesondere ein Körperteil, insbesondere ein Finger, gegen ein optisches Medium (108) gedrückt wird, das Teil der Detektionseinrichtung ist, und dass entweder der auf das Medium durch das angedrückte Körperteil ausgeübte Druck erfasst wird und dass in Abhängigkeit von dem erfassten Druck auf das Medium (108) die Anregungssendeeinrichtung (100) eingeschaltet und/oder diese in Abhängigkeit von einer Verminderung des Drucks, insbesondere unter eine bestimmte Schwelle, ausgeschaltet wird, oder eine Verdunkelung des Mediums im Bereich des angedrückten Körperteils erfasst wird und dass durch Verdunkelung des Mediums die Anregungssendeeinrichtung (100) eingeschaltet und/oder diese durch eine Vergrößerung der Helligkeit im Medium insbesondere über eine bestimmte Schwelle, ausgeschaltet wird, und/oder dass der Anregungsstrahl (SA) mit mindestens zwei Anregungswellenlängen oder Gruppen von Anregungswellenlängen ausgesendet wird, wobei eine erste Anregungswellenlänge oder Gruppe von Anregungswellenlängen den Nachweis einer in dem Stoff nachzuweisenden Substanz ermöglicht, während wenigstens eine weitere Anregungswellenlänge oder Gruppe von Anregungswellenlängen den Nachweis einer von einer nachzuweisenden Substanz verschiedenen Referenzsubstanz ermöglicht und dass für die wenigstens zwei verschiedenen Substanzen Profile bezüglich ihrer Dichteverteilung in der Tiefe des Stoffes ermittelt und miteinander verknüpft werden, und wobei insbesondere ein Tiefenprofil oder wenigstens ein oder mehrere Parameter eines Tiefenprofils der Referenzsubstanz in dem Stoff bekannt ist,
wobei der Anregungsstrahl (SA) insbesondere moduliert, vorzugsweise nacheinander mit verschiedenen Modulationsfrequenzen moduliert wird, wobei Reaktionssignale für verschiedene Modulationsfrequenzen detektiert werden oder ein Zeitverhalten des Reaktionssignals bei einer Änderung der Intensität des Anregungsstrahls, insbesondere bei dessen periodischem Anschalten und/oder Abschalten, analysiert wird.
